# EUROPEAN PATENT SPECIFICATION

(11) **EP 2 920 320 B1**
(45) Date of publication and mention of the grant of the patent: **28.12.2016**
(21) Application number: 13791997.3
(22) Date of filing: 14.11.2013
(51) Int. Cl.: C12Q 1/68

(54) **RCA REPORTER PROBES AND THEIR USE IN DETECTING NUCLEIC ACID MOLECULES**
RCA-REPORTERSONDEN UND DEREN VERWENDUNG ZUR DETEKTION VON NUKLEINSÄUREMOLEKÜLEN
SONDES RCA RAPPORTEUR ET LEUR UTILISATION POUR DÉTECTER DES MOLÉCULES D'ACIDE NUCLÉIQUE

(30) Priority: 14.11.2012 GB 201220504; 23.05.2013 GB 201309328
(43) Date of publication of application: 23.09.2015
(73) Proprietor: Olink Bioscience AB, 751 24 Uppsala (SE)
(72) Inventor: LANDEGREN, Ulf, 75645 Uppsala (SE); CHEN, Lei, Yangzhou Jiangsu 225111 (CN); WU, Di, 75108 Uppsala (SE); NONG, Yuan, Nanning Guangxi 530003 (CN)
(74) Representative: Dzieglewska, Hanna Eva
(86) International application number: PCT/EP2013/073888
(87) International publication number: WO 2014/076214

(56) References cited:
- WO-A1-99/49079
- WO-A1-2012/152942
- WO-A2-2006/108422
- US-A1- 2005 069 939
- IRENE WEIBRECHT ET AL: "Visualising individual sequence-specific protein-DNA interactions in situ", NEW BIOTECHNOLOGY, vol. 29, no. 5, 31 August 2011 (2011-08-31), pages 589-598, XP055031292, ISSN: 1871-6784, DOI: 10.1016/j.nbt.2011.08.002
- Rachel Yuan Nong: "Proximity Ligation Assays for Disease Biomarkers Analysis", Acta Universitatis Upsaliensis, 7 October 2011 (2011-10-07), XP055031648, Uppsala Retrieved from the Internet: URL:http://uu.diva-portal.org/smash/get/di va2:441225/FULLTEXT01 [retrieved on 2012-07-03]
- W. M. HOWELL ET AL: "Glycosylases and AP-cleaving enzymes as a general tool for probe-directed cleavage of ssDNA targets", NUCLEIC ACIDS RESEARCH, vol. 38, no. 7, 1 April 2010 (2010-04-01), pages e99-e99, XP055097905, ISSN: 0305-1048, DOI: 10.1093/nar/gkp1238

### SEQUENCE MISTING

<110> Olink AB
<120> RCA Reporters
<130> 27.35.114482/02
<150> GB 1220504.3
   <151> 2012-11-14
<150> GB 1309328.1
   <151> 2013-05-23
<160> 25
<170> PatentIn version 3.5
<210> 1
   <211> 61
   <212> DNA
   <213> Artificial sequence
<220>
   <223> Target
<220>
   <221> modified_base
   <222> (1)..(1)
   <223> 5' Biotin
<400> 1
   aaaaaaaaaa cgcgtccgcc ccgcgaaagc ctcgcctttg ccgaaaccgc gctcgtcgtc 60
   g 61
<210> 2
   <211> 144
   <212> DNA
   <213> Artificial sequence
<220>
   <223> Continuous RCA two part probe part 1
<400> 2
   cgacgacgag cgcggaaaag acaggcaaag cggaggggaa acaaggaaaa auuccuuguu 60
   uccccuccgc uuugccuguc ugaagcggtt tttgactcga gacgaagtct cgagtcaaaa 120
   accgctttgc ctgtctcgtg cttg 144
<210> 3
   <211> 89
   <212> DNA
   <213> Artificial sequence
<220>
   <223> Continuous RCA probe two part probe part 2
<220>
   <221> modified_base
   <222> (89)..(89)
   <223> 3' Biotin
<400> 3
   tgcagtgagg gctcgtttgc ggttctaaat tccttgtttc ccctcactgc acaagcacga 60
   aacgcggggc ggacgcgagt gagctagac 89
<210> 4
   <211> 158
   <212> DNA
   <213> Artificial sequence
<220>
   <223> Cleavage strand RCA probe RCA template strand
<400> 4
   cgacgacgag cgcggaaaag acaggcaaag cggaggggaa acaaggaaga gtcaaaaacc 60
   gctttgcctg tctcgtgctt gtgcagtgag ggctcgtttg cggttctgaa ttccttgttt 120
   cccctcactg cacaagcacg gaacgcgggg cggacgcg 158
<210> 5
   <211> 40
   <212> DNA
   <213> Artificial sequence
<220>
   <223> Cleavage strand
<400> 5
   uuuuugactc uuccuuguuu ccccuccgcu uugccugucu 40
<210> 6
   <211> 35
   <212> DNA
   <213> Artificial sequence
<220>
   <223> Prox probe domain 1
<220>
   <221> modified_base
   <222> (1)..(1)
   <223> 5' thiol
<220>
   <221> modified_base
   <222> (35)..(35)
   <223> 3' RNA Ome
<400> 6
   aaaaaaaaaa gacgctaata gttaagacgc ttuuu 35
<210> 7
   <211> 32
   <212> DNA
   <213> Artificial sequence
<220>
   <223> Prox probe domain 2
<220>
   <221> modified_base
   <222> (1)..(1)
   <223> 5' thiol
<400> 7
   aaaaaaaaaa tatgacagaa ctagacactc tt 32
<210> 8
   <211> 182
   <212> DNA
   <213> Artificial sequence
<220>
   <223> Two part linear RCA template strand
<400> 8
   tctctctctc aagagtgtct agttctgtca tagaaagaca ggcaaagcgg aggggaaaca 60
   aggaagagtc aaaaaccgct ttgcctgtct attgcttgtg cagtgagggc tcgtttgcgg 120
   ttctgaattc cttgtttccc ctcactgcac aagcaatgaa aagcgtctta actattagcg 180
   tc 182
<210> 9
   <211> 103
   <212> DNA
   <213> Artificial sequence
<220>
   <223> Target
<220>
   <221> modified_base
   <222> (1)..(1)
   <223> 5' Biotin
<220>
   <221> modified_base
   <222> (103)..(103)
   <223> 3' RNA Ome
<400> 9
   tctctctctc tgctgcttcg ttgtggaagt ctcggttttc cgcgaagctt tcgttggtgg 60
   cgaactcgtt tgcggttctg aattccttgt ttcccctgaa uuu 103
<210> 10
   <211> 55
   <212> DNA
   <213> Artificial sequence
<220>
   <223> RCA primer part
<220>
   <221> modified_base
   <222> (1)..(1)
   <223> 5' Biotin
<400> 10
   agtgagctag acaggggaaa caaggaagaa cgacgaacgt gatacgagtc aaaaa 55
<210> 11
   <211> 20
   <212> DNA
   <213> Artificial sequence
<220>
   <223> Connection template 1
<400> 11
   ggcaaagcgg uuuuugacuc 20
<210> 12
   <211> 137
   <212> DNA
   <213> Artificial sequence
<220>
   <223> Padlock 1
<400> 12
   ccgctttgcc tgtctgaacg tgcttgtttc cgtgcagtgg ccagtatcac gttcgtcgtg 60
   tgcgactttc tcatgttgtg cgtttggcca ctgcacggaa acaagcacgg aaagaaccgc 120
   aaacgaggag tcaaaaa 137
<210> 13
   <211> 20
   <212> DNA
   <213> Artificial sequence
<220>
   <223> Connection template 2
<400> 13
   caagcggacc uuuuugacuc 20
<210> 14
   <211> 122
   <212> DNA
   <213> Artificial sequence
<220>
   <223> Padlock 2
<400> 14
   ggtccgcttg tcttggaagc ctttgcttcg tctgcagtgg acaaacaccg tccatacgcc 60
   tgcttgtcgt gtccactgca gacgaagcaa aggcgaagcc accaacgaaa gcgagtcaaa 120
   aa 122
<210> 15
   <211> 40
   <212> DNA
   <213> Artificial sequence
<220>
   <223> Cleavage strand 1
<400> 15
   ccugcuuguc gugucccgcu uugccugucu uuuuugacuc 40
<210> 16
   <211> 58
   <212> DNA
   <213> Artificial sequence
<220>
   <223> Ligation 1
<400> 16
   agacaggcaa agcgggacac gacaagcagg gaagcggaaa accgagacga gtcaaaaa 58
<210> 17
   <211> 40
   <212> DNA
   <213> Artificial sequence
<220>
   <223> Cleavage strand 2
<400> 17
   uguugugcgu uuggcggucc gcuugucuug uuuuugacuc 40
<210> 18
   <211> 58
   <212> DNA
   <213> Artificial sequence
<220>
   <223> Ligation 2
<400> 18
   caagacaagc ggaccgccaa acgcacaaca gaacacaacg aagcagcaga gtcaaaaa 58
<210> 19
   <211> 25
   <212> DNA
   <213> Artificial sequence
<220>
   <223> Cleavage strand 3
<400> 19
   uuuuugacuc cgacgaacgu gauac 25
<210> 20
   <211> 20
   <212> DNA
   <213> Artificial sequence
<220>
   <223> Detection oligo 1
<220>
   <221> modified_base
   <222> (1)..(1)
   <223> 5' Cy3
<400> 20
   tgcgtttggc ggtccgcttg 20
<210> 21
   <211> 20
   <212> DNA
   <213> Artificial sequence
<220>
   <223> Detection oligo 2
<220>
   <221> modified_base
   <222> (1)..(1)
   <223> 5' Cy5
<400> 21
   ttgtcgtgtc ccgctttgcc 20
<210> 22
   <211> 48
   <212> DNA
   <213> Artificial sequence
<220>
   <223> Correct target
<220>
   <221> modified_base
   <222> (1)..(1)
   <223> 5' Biotin
<220>
   <221> modified_base
   <222> (48)..(48)
   <223> 3' RNA Ome
<400> 22
   tctctctctc tctctccgcg ctcgtcgtcg cgcgtccgcc ccgcguuu 48
<210> 23
   <211> 48
   <212> DNA
   <213> Artificial sequence
<220>
   <223> Wrong target
<220>
   <221> modified_base
   <222> (1)..(1)
   <223> 5' Biotin
<220>
   <221> modified_base
   <222> (48)..(48)
   <223> 3' RNA Ome
<400> 23
   tctctctctc tctctccgcg ctcgtcgtcg agcgtccgcc ccgcguuu 48
<210> 24
   <211> 158
   <212> DNA
   <213> Artificial sequence
<220>
   <223> Two part RCA template strand
<220>
   <221> modified_base
   <222> (1)..(1)
   <223> 5' phosphorylation
<400> 24
   cgacgacgag cgcggaaaag acaggcaaag cggaggggaa acaaggaaga gtcaaaaacc 60
   gctttgcctg tctcgtgctt gtgcagtgag ggctcgtttg cggttctgaa ttccttgttt 120
   cccctcactg cacaagcacg gaacgcgggg cggacgcg 158
<210> 25
   <211> 30
   <212> DNA
   <213> Artificial sequence
<220>
   <223> Detection oligonucleotide
<220>
   <221> modified_base
   <222> (1)..(1)
   <223> 5' Cy3
<400> 25
   ttccttgttt cccctccgct ttgcctgtct 30

## Description

The present invention relates to the detection of an analyte in a sample and in particular to the detection of a nucleic acid molecule (e.g. a nucleic acid analyte) in a sample, which may be the analyte for detection or may be indicative of the presence of the analyte (i.e. a marker or proxy for the analyte) in the sample. The invention concerns the provision of a new nucleic acid detection probe (a nucleic acid construct) for use in rolling circle amplification (RCA) assays (also known as rolling circle replication (RCR) assays). The present invention also provides a method for using said probe in the detection of an analyte, e.g. a target nucleic acid molecule (i.e. a nucleic acid analyte).

The scope of the present invention is defined by appended claims 1-15. The detection probe of the present invention is designed to provide the nucleic acid components (i.e. substrates) sufficient to initiate (i.e. to directly enable) a RCA (RCR) reaction, i.e. without the need to add further nucleic acid components to the assay in order to generate a RCA product. The RCA components provided by the probe are unavailable for a RCA reaction until the probe has been cleaved and/or unfolded, i.e. at least one of the components of the RCA reaction must be released by unfolding the probe and/or a cleavage of the probe before the RCA reaction can take place. Cleaved and/or unfolded probes that interact with the target nucleic acid molecule will generate RCA products, particularly RCA products that are localized to the target nucleic acid molecule. The cleaved and/or unfolded probe is extended (i.e. a domain of the probe is extended) and the detection of the extension product signals the presence of the target nucleic acid molecule in the sample. Thus, the probe of the present invention the has the effect of reducing the number of distinct initial nucleic acid components added to an assay to achieve a rolling circle amplification reaction, i.e. decreasing the complexity of the initial components present in a detection assay whilst retaining the advantages associated with RCA assays. The observable effect of reducing the complexity of the initial components in the assay is a reduction in "background" signals, i.e. signals from non-specific interactions between components in the assay, thereby increasing both the specificity and sensitivity of the assay.

Rolling circle replication (RCR) reactions are well described in the art and have been demonstrated to be useful in a variety of assays for the detection of target nucleic acid molecules in a sample, i.e. for the replication or amplification of a target nucleic acid molecule, wherein the replicated or amplified nucleic acid molecule is detected. Accordingly, rolling circle replication (RCR) commonly is referred to as a rolling circle amplification (RCA), and these terms are used interchangeably herein., RCA methods have been developed in which the rolling circle product is further amplified by a hyperbranch or DNA cascade reaction, as described in WO 97/19193 and WO 97/20948. However, in the present application, RCA is not intended to be limited to reactions that require hyperbranch or DNA cascade reactions.

RCA relates to the synthesis of nucleic acid molecules using a circular single stranded nucleic acid molecule, e.g. a circle oligonucleotide, as rolling circle template (a RCA template) and a strand-displacing polymerase to extend a primer which is hybridised to the template. The primer may in certain typical assays be provided by a target RNA molecule or a target DNA molecule. The addition of a polymerase and nucleotides starts the synthesis reaction, i.e. polymerization. As the rolling circle template is endless, the resultant product is a long single stranded nucleic acid molecule composed of tandem repeats that are complementary to the rolling circle template.

In practice, RCA reactions often utilise linear nucleic acid molecules, e.g. oligonucleotides such as padlock probes as described in more detail below, which are manipulated to generate circular nucleic acid molecules, typically by ligating the ends of the nucleic acid molecule together, e.g. using a ligase enzyme. For instance, the ends of the nucleic acid molecule may be brought into proximity to each other by their hybridisation to adjacent sequences on a target nucleic acid molecule which acts as a ligation template. The formation of the circular nucleic acid molecule allows it to be copied in a RCA reaction. This reaction may initiated by adding a primer to the closed circle or a primer may be generated from the target nucleic acid molecule, i.e. ligation template. The RCA product therefore forms part of the initial primer. This can be particularly advantageous because it may allow localised detection of the target nucleic acid, i.e. in embodiments where the nucleic acid molecule used to prime RCA is immobilized the RCA product will also be immobilized.

Thus, the RCA product may remain as string of tandemly repeated complementary copies of the nucleic acid circle, which can be particularly useful for *in situ* detection, but may be detected in homogeneous ("in solution") assays. For instance, a RCA reaction may result in a 1000-fold amplification of the circle in just 1 hour (based on a circle consisting of about 100 nucleotides). Thus, the RCA of a circular oligonucleotide may result in a RCA product that forms a bundle or "blob" of DNA that can be about 1µm in diameter. The product, i.e. blob, may be detected by the hybridization of nucleic acid probes (detection probes) conjugated to fluorescent labels which allows the blob to be visualised by fluorescence microscopy (in heterogeneous assays, i.e. *in situ*) or flow cytometry (in homogeneous assays). In other embodiments, the RCA products may be reduced to monomers by digestion with a restriction enzyme or a ribozyme, e.g. WO 98/38300 and Dahl F et al., Proc Natl Acad Sci USA. 101 (13), 4548-53 (2004), which are then detected.

RCA may be used for the detection of any nucleic acid molecule in a sample and typically has been used to detect directly target nucleic acids in cell and tissue samples, e.g. *in situ,* i.e. localised, detection of nucleic acid molecules, which is of significant interest both for research and diagnostic purposes. However, RCA assays are not limited to use in heterogeneous formats and may also find utility in homogeneous assays (see e.g. WO 2009/037659).

RCA has also been utilised in methods for the detection of other analytes, i.e. analytes other than nucleic acid molecules such as cells, viruses, proteins, peptides, small molecules etc. In this respect, a variety of assays have been developed in which a nucleic acid molecule may be used to directly or indirectly tag or label a target analyte in a sample and detection of the nucleic acid molecule serves to indicate the presence of the analyte in the sample. In some methods a new nucleic acid molecule may be generated in a sample (i.e. a nucleic acid molecule that was not present in the original sample and was not one of the components added to the sample) when one or more molecules that interact with, e.g. bind to, the target analyte. The detection of the generated nucleic acid molecule is indicative of the analyte in a sample, i.e. the generated nucleic acid molecule acts as a marker or proxy for the target analyte.

Various methods that detect a nucleic acid molecule as a marker or proxy for the target analyte, e.g. protein, are well described in the art. For instance, immuno-PCR, immuno-RCA and proximity probe assays may rely on the detection of a nucleic acid molecule as a substitute for detecting the target analyte directly.

Immuno-PCR involves labelling an antibody for a specific target analyte with a nucleic acid molecule. Typically, the target analyte is captured on a substrate, e.g. with a first antibody and contacted with the antibody:nucleic acid complex. Excess antibody may be washed away before the sample is subjected to a polymerase chain reaction (PCR), which is used to amplify the nucleic acid molecule conjugated to the antibody. Detection of the PCR product is indicative of the presence of the analyte in the sample and proportional to the amount of analyte.

Immuno-RCA relies on the same principles as immuno-PCR. The difference is that a circular (or circularisable) oligonucleotide (RCA template) is hybridized to the nucleic acid molecule conjugated to the antibody (the RCA template may be pre-hybridized or added after the antibody has been allowed to interact with the target analyte). The nucleic acid molecule conjugated to the antibody is used as the primer for RCA. Thus, the RCA product is tethered to the antibody that is interacting with the target analyte, thereby allowing localised detection of the analyte in the sample, e.g. in a cell or tissue sample.

A proximity assay relies on the principle of "proximity probing", wherein an analyte is detected by the binding of multiple (i.e. two or more, generally two or three) probes, which when brought into proximity by binding to the analyte (hence "proximity probes") allow a signal to be generated. Typically, at least one of the proximity probes comprises a nucleic acid domain linked to the analyte-binding domain of the probe, and generation of the signal involves an interaction between the nucleic acid moieties and/or a further functional moiety which is carried by the other probe(s). Thus signal generation is dependent on an interaction between the probes (more particularly by the nucleic acid or other functional moieties/domains carried by them) and hence only occurs when the probes have bound to the analyte, thereby lending improved specificity to the detection system. The concept of proximity probing has been developed in recent years and many assays based on this principle are now well known in the art. For example, proximity ligation assays (PLAs) rely on proximal binding of proximity probes to an analyte to generate a signal from a ligation reaction involving or mediated by (e.g. between and/or templated by) the nucleic acid domains of the proximity probes.

Proximity probes of the art are generally used in pairs, and individually consist of an analyte-binding domain with specificity to the target analyte, and a functional domain, e.g. a nucleic acid domain coupled thereto. The analyte-binding domain can be for example a nucleic acid "aptamer" (Fredriksson et al (2002) Nat Biotech 20:473-477) or can be proteinaceous, such as a monoclonal or polyclonal antibody (Gullberg et al (2004) Proc Natl Acad Sci USA 101:8420-8424). The respective analyte-binding domains of each proximity probe pair may have specificity for different binding sites on the analyte, which analyte may consist of a single molecule or a complex of interacting molecules, or may have identical specificities, for example in the event that the target analyte exists as a multimer. When a proximity probe pair come into close proximity with each other, which will primarily occur when both are bound to their respective sites on the same analyte molecule, the functional domains (e.g. nucleic acid domains) are able to interact, for example, nucleic acid domains may be joined to form a new nucleic acid sequence generally by means of a ligation reaction, which may be templated by a splint oligonucleotide added to the reaction, said splint oligonucleotide containing regions of complementarity for the ends of the respective nucleic acid domains of the proximity probe pair. The new nucleic acid sequence thereby generated serves to report the presence or amount of analyte in a sample, and can be qualitatively or quantitatively detected, for example by realtime, quantitative PCR (q-PCR).

Alternatively, rather than being ligated to each other, the nucleic acid domains of the proximity probes when in proximity may template the ligation of one or more added oligonucleotides to each other, including an intramolecular ligation, to circularise one or more added linear oligonucleotides, for example based on the so-called padlock probe principle, wherein the ends of the added linear oligonucleotide(s) are brought into juxtaposition for ligation by hybridising to a template, here one or more nucleic acid domains of the proximity probes (in the case of a padlock probe the target nucleic acid for the probe). Various such assay formats are described in WO 01/61037.

WO 97/00446 and US 6,511,809 disclose a heterogeneous format for proximity ligation assays, i.e. the analyte is first immobilised to a solid substrate by means of a specific analyte-binding reagent.

Homogeneous proximity ligation assays (i.e., in solution) are disclosed in WO 01/61037, WO 03/044231, WO 2005/123963, Fredriksson et al (2002) Nat Biotech 20:473-477 and Gullberg et al (2004) Proc Natl Acad Sci USA 101:8420-8424.

Not all proximity assays are based on ligation. For instance, a proximity assay based on extension is described in WO/EP2012/051474. WO 2007/044903 discloses proximity probe-based assays for detecting analytes which rely on the formation and detection of a released nucleic acid cleavage product. Some of the described embodiments involve a probe comprised of an analyte-binding moiety and an attached enzyme, which enzyme acts on a nucleic acid moiety attached to the analyte-binding moiety of a second probe, resulting in the release of a detectable nucleic acid cleavage product.

Analyte detection assays, including in some embodiments proximity probe-like reagents, wherein a polymerase enzyme attached to an analyte-binding moiety of one probe acts on a nucleic acid moiety attached to the analyte-binding moiety of a second probe, are described in WO 2009/012220. In these assays, the action of the "tethered" polymerase which is part of one of the probes of a probe pair results in the generation of a template, free in solution, which is susceptible to amplification by an added polymerase.

Proximity-based assays involving RCA are disclosed for example in Weibrecht et al. (NEW BIOTECHNOLOGY, 2011, 29(5):589-598), Rachel Yuan Nong: "Proximity Ligation Assays for Disease Biomarkers Analysis", Acta Universitatis Upsaliensis, 7 October 2011, and WO 99/49078.

It will be evident that RCA may be of utility in the specific detection of any nucleic acid molecule in a sample, regardless of whether it is the "original" target analyte in a sample or it is a "proxy" or "marker" for the target analyte generated by the interaction of specific detection molecules, e.g. proximity probes, with the target analyte, e.g. protein. Similarly, RCA may be useful in the detection of amplified nucleic acid molecules. For instance, in samples in which the target nucleic acid molecule is present in particularly low amounts, e.g. rare transcripts, rare viral genomes etc, RCA can be used to enhance the detection of the nucleic acid molecule for detection. In some instances the nucleic acid molecule for detection may itself be a RCA product. Thus, a RCA reaction may be useful to amplify a primary RCA product, i.e. to produce secondary RCA products. Secondary RCA products may also be the target for further RCA reactions, i.e. to produce tertiary RCA products, and so on.

Despite the existence of a plurality of methods for the sensitive, rapid and convenient detection or quantification of one or more analytes, e.g. nucleic acid molecules, in a sample, there is a continuous need to develop assays with increased sensitivity, e.g. improved signal to noise ratio. There is also a desire to simplify assays to reduce errors and inefficiencies that arise from a large number of steps, e.g. handling errors, and to reduce costs and reaction times.

It has been inevitable that the efforts made to provide analyte detection assays with increased sensitivity have resulted in more complex assays, i.e. assays with more components and/or substrates. For example, expanding the number of specific interactions that must occur in a reaction to produce a target specific signal is likely to increase the specificity of the assay. However, the addition of a new component to a detection assay will result in an increase in the complexity of the reaction, wherein the increase in complexity may not be commensurate with, i.e. proportionate to, the number of components added. For example, each new component in an assay may introduce a multitude of possible new interactions, i.e. the new component may be cross-reactive with a variety of components in the assay. Accordingly, expanding the number of components in an assay increases the probability that non-specific interactions will occur, therefore risking the possibility of increasing the background signal, which may overwhelm any improvement in the specificity of the assay. The addition of components to the assay may also result in an increase in the number of handling steps and, accordingly, the amount of time and resources needed to complete the assay, i.e. additional components may make automation of the assay more difficult.

Accordingly, there is a need to reduce the number of components in an assay and/or minimise the number of potential interactions between components in the sample, whilst maintaining multiple target specific interactions that contribute to the specificity and selectivity of the assay, i.e. there is a desire to retain multiple processing steps that yield the specificity but without having to add more components or steps to the assay.

The probe of the present invention is directed to addressing this need and provides the nucleic acid components to directly enable a rolling circle amplification (RCA) reaction in the form of a single nucleic acid construct. Typically, the RCA nucleic acid components provided by (i.e. comprised as part of or generated from) the probe are able to initiate a RCA reaction following a specific interaction with the target nucleic acid molecule and cleavage and/or unfolding of the probe. Accordingly, the probe achieves specificity through a number of precise interactions without increasing the number of components to be added to the reaction. Alternatively put, the probe of the present invention may be viewed as providing a nucleic acid construct (i.e. nucleic acid molecule) that is necessary and sufficient for a RCA reaction when used in a method for the detection of a nucleic acid molecule in a sample.

Unlike standard RCA probes for detecting a target nucleic acid molecule (e.g. RCA templates, such as padlock probes), the probe of the present invention does not result in the amplification or replication of all or part of the target nucleic acid molecule. The probe provides both the circular template (RCA template) and primer for RCA (and in some embodiments, a ligation template to template the ligation of a circularisable RCA template). Accordingly, the sequence of the RCA product generated by the probe of the invention may be entirely distinct from the sequence of the target nucleic acid molecule. This may be particularly useful in multiplex embodiments. For instance, where it is desirable to detect two or more target nucleic acid molecules in a sample that comprise very similar sequences (e.g. single nucleotide polymorphisms, SNPs, such as in viral genome sequence variants), the part of the probes that interacts with the targets will be very similar, but the part of the probes that template the RCA may be distinct, thereby allowing signals generated from similar targets to be distinguished easily. This is in contrast to standard RCA probes, e.g. padlock probes, wherein the RCA product will necessarily comprise a sequence derived from the target nucleic acid.

Representative examples of the probes of the invention, and how the probes generate a RCA product, are described below to illustrate the features required by the probes and the variation with which the features may be incorporated into a probe design.

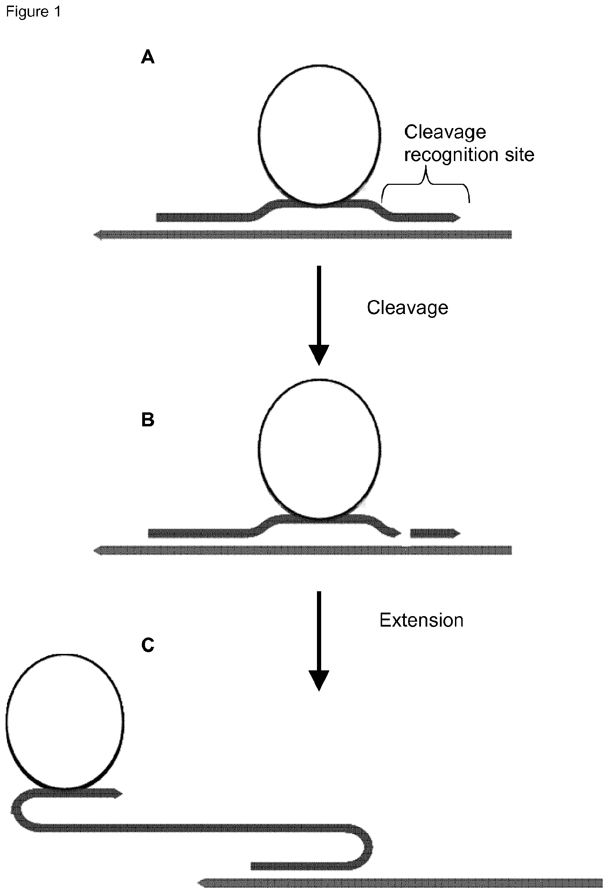

One of the simplest forms the probe of the present invention is depicted in Figure 1. The probe provides a nucleic acid construct comprising two nucleic acid strands and is referred to herein as a type of "circle RCA probe" as defined below.

The first nucleic acid strand comprises two domains that each have a region of complementarity to the target nucleic acid molecule (so-called target complementary domains, target binding domains or target recognition domains). The target complementary domain at the 5' end of the first nucleic acid strand is used to secure (i.e. attach) the RCA product to the target nucleic acid molecule. The target complementary domain at the 3' end of the first nucleic acid strand comprises a sequence that is recognised by a cleavage enzyme when bound to the target nucleic acid molecule, i.e. the domain comprises a cleavage recognition site, i.e. a site or domain that may be cleaved or result in the cleavage of an adjacent site or domain in the probe. The target complementary domains are linked by a domain that comprises a region of complementarity to the RCA template (a circular nucleic acid molecule in this embodiment). Thus, the sequence linking the target complementary domains may be viewed as comprising a RCA template complementary domain or RCA template binding domain. This domain will form at least part of the primer for RCA and therefore may be viewed as a primer domain (i.e. the RCA primer domain). Accordingly, the first nucleic acid strand of the probe may be referred to as the "primer strand", i.e. the RCA primer is provided by, or generated from, the first strand.

The second nucleic acid strand is in the form of a circular nucleic acid molecule (RCA template) that is hybridized to at least part of the primer domain (i.e. hybridized to the RCA template binding domain part of the primer domain). Thus, the second nucleic acid strand may be referred to as the "RCA template strand", "template strand" or "circle strand". As discussed below in more detail, it will be evident that in some embodiments the RCA template may be a circularisable nucleic acid molecule (or circularisable oligonucleotide), e.g. a padlock probe. Accordingly, the primer domain (e.g. the RCA binding domain part of the primer domain) may template the ligation of the circularisable RCA template. Hence, the primer domain and/or the RCA template binding domain may also be viewed as a ligation template domain.

In the presence of the target nucleic acid molecule, the target complementary regions of the probe hybridize to the target sites in the nucleic acid molecule (probe binding sites). Hybridization of the probe and target forms a "functional" cleavage recognition site (Figure 1A). In the presence of an enzyme that recognizes the cleavage recognition site, e.g. an endonuclease such as a nickase, the first strand of the probe is cleaved (in some embodiments, both the first nucleic acid strand of the probe and the target nucleic acid molecule may be cleaved), which results in the release of an extendable 3' end that is not hybridized to the target nucleic acid molecule (i.e. a single stranded domain) (Figure 1 B). This 3' extendable end may be complementary to the RCA template. Alternatively, the single stranded part of the first strand of the probe may be degraded, e.g. by exonuclease digestion. In either case, cleavage of the primer strand (which may involve more than one cleavage event) results in the release of the primer domain, i.e. the RCA primer. The primer domain is extended using the RCA template as a template for polymerisation to generate the RCA product which is secured, or attached, to the target nucleic acid by the 5' target complementary domain of the probe (Figure 1 C).

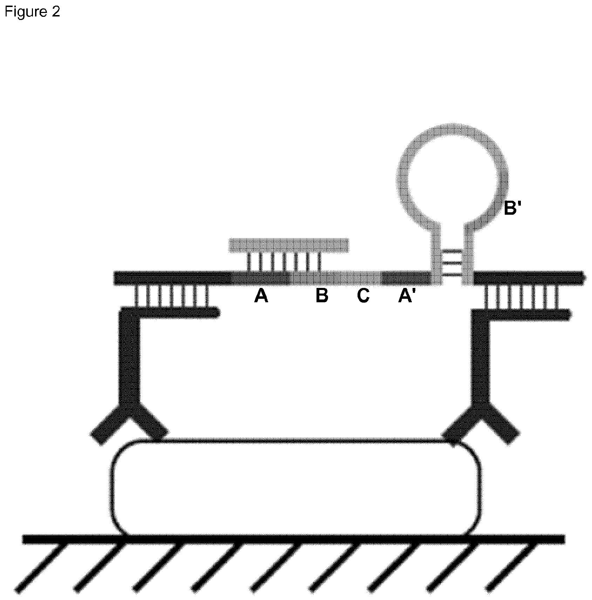

Another exemplary embodiment of the probe of the invention is depicted in Figure 2, which depicts a type of "hairpin RCA probe", as defined below. The probe is in the form of a first nucleic acid strand that has a hairpin structure, which comprises a cleavage recognition site. The first nucleic acid strand is capable of providing the nucleic acid components sufficient to initiate a RCA reaction, i.e. a primer, RCA template and ligation template can be generated from the first nucleic acid strand. In some variant embodiments the ligation template may be provided as part of the second nucleic acid strand. Accordingly, the first nucleic acid strand of the hairpin RCA probes may be viewed as the primer strand or RCA template strand, i.e. the first nucleic acid strand of the hairpin RCA probes always provides the primer and RCA template. The first strand comprises two target complementary domains (target binding domains), one at each end, which hybridize to the target nucleic acid molecule(s). A second nucleic acid strand is hybridized to the first nucleic acid strand and the double stranded portion of the probe acts as a cleavage recognition site, e.g. a site or domain that is recognised by a cleavage enzyme. Accordingly, the second nucleic acid strand may be viewed as a cleavage strand.

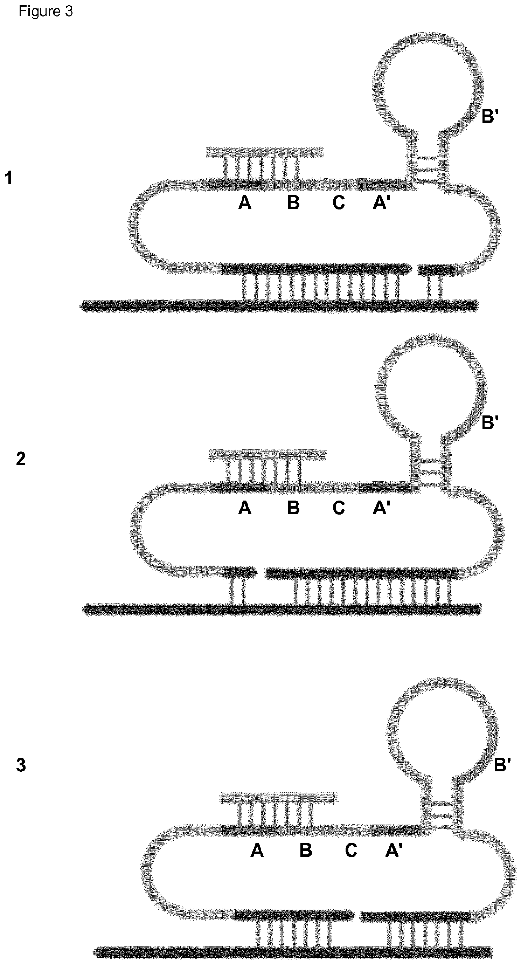

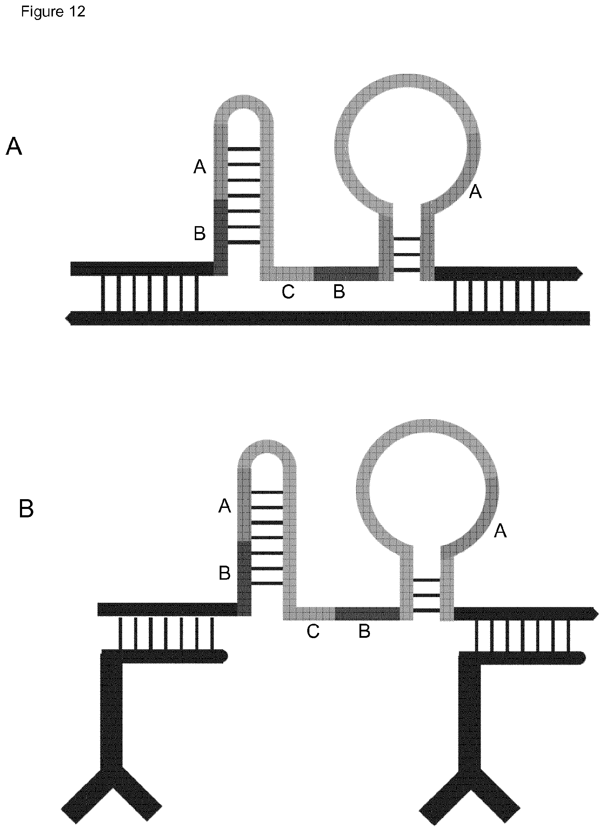

In Figure 2 the target complementary domains at each end of the probe are hybridized to different nucleic acid molecules that are in close proximity; each end hybridizes to the nucleic acid domain of a proximity probe. However, it will be apparent that the target complementary domains may be capable of hybridizing to adjacent regions of a single target nucleic acid molecule (the target nucleic acid molecule may comprise directly or indirectly adjacent probe binding sites, see e.g. Figure 12A). Moreover, in some embodiments the target complementary domains of the probe may hybridize to the target nucleic acid molecule(s) such that the 5' and 3' ends of probe are directly or indirectly ligatable, such that the probe may be ligated to form a circular nucleic acid molecule using the target nucleic acid molecule(s) as a ligation template (see Figure 3).

Following contact with a sample under conditions that allow the probe to hybridize with the target nucleic acid molecule(s), one or more cleavage agents that recognize the cleavage recognition sites are added to the sample which results in the cleavage at least two sites in the probe, i.e. the loop of the hairpin structure and the double stranded portion of the probe or a region adjacent thereto. Upon cleavage of the probe the hairpin structure is able to unfold to provide the RCA template (a circularisable nucleic acid molecule) that is attached to the target nucleic acid molecule via hybridization to a portion of the probe comprising a target complementary domain. In some embodiments, the portion of the probe to which the RCA template is hybridized (i.e. attached) may function as the primer for the RCA reaction (e.g. Figure 3). In other embodiments the primer is provided by the portion of the probe that is attached to the target nucleic acid molecule via the other target complementary domain. In these embodiments, the same portion of the probe may provide both the primer and ligation template. However, it will be evident from the description below that the primer and ligation template may be provided as separate domains of the probe, e.g. the ligation template may be provided by the second nucleic acid strand of the probe.

In the presence of the target nucleic acid molecule(s), the RCA nucleic acid components are directly or indirectly hybridized to the target nucleic acid molecule(s) (via the target complementary domains) thereby maintaining the RCA nucleic acid components in close proximity. The RCA template (circularisable nucleic acid molecule) hybridizes to both the primer and ligation template, which may be the same part of the probe nucleic acid construct. The ligation template part of the probe templates the circularisation of the RCA template in the presence of a ligase enzyme. The primer part of the probe can be extended using the circularised RCA template as a polymerisation template to generate the RCA product. It will be evident that in the absence of a target nucleic acid molecule the cleaved parts of the probe that form the RCA nucleic acid components are not maintained in proximity, thereby preventing the production of a RCA product in the absence of target nucleic acid.

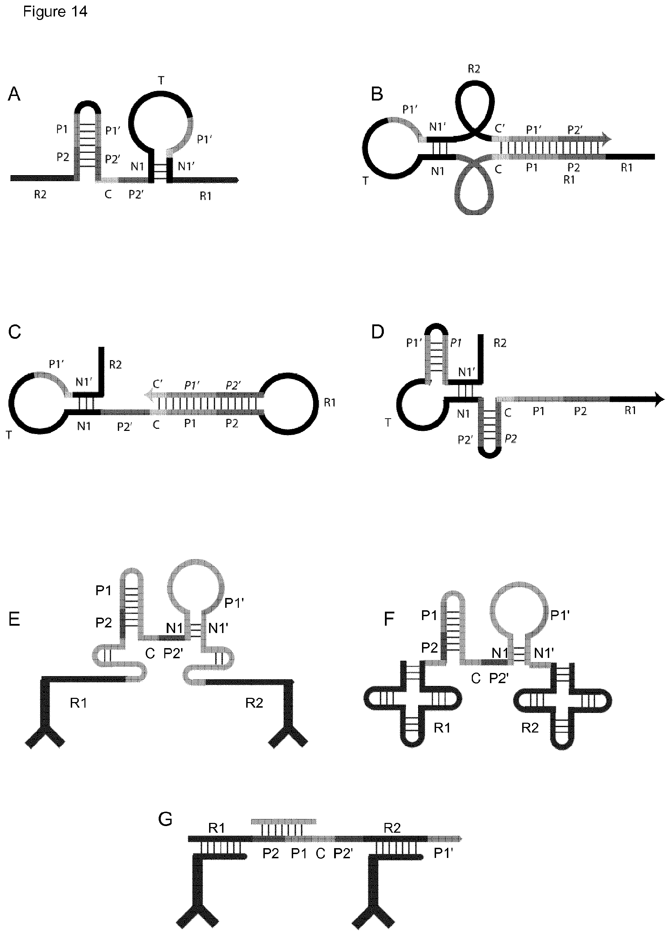

The exemplary embodiments described above refer to probes that interact with an analyte by binding to a nucleic acid molecule (e.g. directly to a nucleic acid analyte or indirectly by binding to a nucleic acid molecule that is attached to a molecule, e.g. antibody, that binds to the analyte). Accordingly, the probes comprise target binding domains that are target complementary domains, i.e. regions of complementarity to the target nucleic acid molecule. However, the probes of the invention may bind to non-nucleic acid target analytes directly. In this respect, the probes may comprise one or more target binding domains that are capable of binding to any analyte. For instance, the probe may be coupled to (i.e. conjugated to) one or more analyte-binding domains, e.g. one or more antibodies or other target-specific binding partner, to form target binding domains that are able of interacting specifically with a domain on the target analyte (see e.g. Figure 14E). In other embodiments, the target binding domains may be in the form of nucleic acid aptamers that are capable of binding to a domain on the target (see e.g. Figure 14F).

In embodiments where the probe contains a single target binding domain e.g. some circle probes, the target binding domain must interact with a nucleic acid molecule to enable the probe to release a RCA component. For instance, in some embodiments at least one target binding domain of the probe must interact with a nucleic acid molecule to enable the formation of a cleavage domain or to unfold the probe. Accordingly, in some embodiments at least one target binding domain must be a target complementary domain. Thus, in embodiments where the probe comprises more than one target binding domain, e.g. two or more, at least one of the target binding domains may be formed by coupling the probe to an analyte-binding domain. For example, circle probes may comprise two target binding domains, wherein a first binding domain is formed by coupling the probe to an analyte-binding domain and the second binding domain comprises a target complementary domain. Typically, the analyte-binding domain will be at the 5' end of a circle probe. In other embodiments (e.g. hairpin probes), each target binding domain may be comprise an analyte-binding domain, e.g. the probe may be coupled to more than one analyte-binding domain etc.

The exemplary embodiments described above demonstrate that the probe of the invention may require two types of ligation events to enable a RCA reaction to proceed. In other embodiments, no ligation events are required. A first ligation event may be defined as a "target dependent ligation", wherein the target complementary domains of the probe may hybridize to the target nucleic acid molecule(s) such that the 5' and 3' ends of probe are directly or indirectly ligatable using the target nucleic acid(s) as a ligation template (see Figure 3).

This may be particularly advantageous because it may allow the generation of the RCA product to be target dependent, i.e. a RCA product may be generated only in the presence of the target molecule. For instance, the target complementary domains of the probe may be designed so that the RCA components generated by cleavage of the probe will remain bound to (i.e. attached to or hybridized to) the target nucleic acid molecule only if the ends of the probe have been directly or indirectly ligated by virtue of a target templated ligation. This may be achieved, for example, by designing the probe to ensure that at least one of the target complementary domains may hybridize stably only in the presence of the other target complementary domain(s), e.g. at least one of the target complementary domains of the probe may be a short sequence. Prior to cleavage of the probe, the combination of the interaction of the target complementary domains (which are joined by the internal part of the nucleic acid molecule) with the target nucleic acid molecule is sufficient to attach the probe to the target nucleic acid. However, if the target complementary domains are not ligated (directly or indirectly), upon cleavage of the cleavable sites in the probe, at least one of the nucleic acid components of the probe required to enable RCA will dissociate from the target nucleic acid. Accordingly, the RCA reaction will not be able to proceed unless a target templated ligation has occurred to secure (i.e. attach) all of the RCA nucleic acid components to the target nucleic acid in proximity (i.e. to stabilize the interaction of the probe target complementary domains with the target nucleic acid molecule).

A second ligation event may be defined as a "probe dependent ligation", wherein the RCA template is provided as a circularisable nucleic acid molecule and its intramolecular ligation is templated by a domain of the probe, i.e. the ligation tempate domain of the probe. A probe dependent ligation is essential in embodiments where the RCA template is provided by the probe by cleavage of a hairpin structure, e.g. a stem loop structure, i.e. hairpin RCA probes. However, a probe dependent ligation event may also be required for circle RCA probes if the RCA template strand is a circularisable nucleic acid molecule.

In some embodiments, the probe requires both a target dependent ligation and a probe dependent ligation, preferably wherein the probe dependent ligation cannot occur unless there has been a target dependent ligation, e.g. when part of the probe required for the probe dependent ligation will dissociate from the target nucleic acid molecule after cleavage of the probe if a target dependent ligation has not occurred.

It will be seen, therefore, that the probe of the invention may be used in methods for the detection of a (target) nucleic acid molecule and the probe may enable the detection to proceed in controlled distinct (discrete or separable) stages. In practice it may be preferable to combine all or most of the reactants simultaneously, to allow the reaction to proceed without intervention. Nevertheless, the probe of the invention requires a sequence of events to occur in order to generate a RCA product that is indicative of the presence of a target nucleic acid molecule in a sample and, in the embodiments described above, the sequence of events (i.e. stages of the reaction) can be considered as:
(i) binding of the probe to the target nucleic acid molecule (this may include unfolding the probe as described below) and optionally ligating the target complementary domains (i.e. a target dependent ligation);
(ii) cleaving the cleavable site(s) in the probe (i.e. releasing at least one of the components of the RCA reaction);
(iii) optionally ligating the RCA template (ligating the circularisable nucleic acid molecule, i.e. a probe dependent ligation);
(iv) extending the RCA primer; and
(v) detecting the RCA product (detecting the extended primer, which is part of the probe).

Thus, the reagents of the assay may be contacted with the target nucleic acid containing sample simultaneously without the generation of the RCA product prior cleavage and/or unfolding of the probe to release the nucleic acid components that are required to enable RCA. The provision of the nucleic acid components to enable RCA in the form of a single probe is advantageous and it is particularly beneficial that the single probe also allows the detection reaction to proceed in stages whilst enabling the addition of the assay reagents simultaneously.

As described in more detail below, the present invention provides numerous probe variants. However, in its simplest form the invention can be seen to provide a single probe (a nucleic acid construct) that interacts with a target analyte, e.g. a nucleic acid molecule, and provides the nucleic acid components that are necessary and sufficient to enable a RCA reaction following cleavage and/or unfolding of the probe (i.e. the RCA nucleic acid components form part of, and/or are generated from, the nucleic acid construct). Accordingly, the present invention may be seen as providing a single RCA probe or self-contained RCA probe. Alternatively, the probe may be viewed as an unfolding RCA probe and/or a cleavable RCA probe. In particular embodiments the invention may be seen as providing a nucleic acid detection probe that must interact with a target nucleic acid and be cleaved and/or unfolded to release the components to enable a RCA reaction, e.g. a target nucleic acid molecule dependent RCA probe. The probe may also be viewed as a probe for target nucleic acid dependent production of a RCA product, wherein the sequence of the RCA product is not related to the sequence of the target nucleic acid molecule (i.e. the RCA product does not comprise a sequence that has significant sequence identity to a part of the target nucleic acid molecule). Since the probes are designed to result in an RCA reaction as a consequence of target binding, and the presence of target is detected by RCA, we have termed them "RCA reporters".

The probe of the invention will find utility in the detection of any analyte in a sample, e.g. a nucleic acid molecule, and may be particularly useful in the localized detection of an analyte, e.g. nucleic acid molecule, i.e. *in situ* detection, because the RCA product is an extension of the primer, which is localized on (e.g. hybridised to) the target analyte, e.g. nucleic acid molecule (directly or indirectly). In some embodiments the target analyte may be a nucleic acid molecule, particularly a RCA product and/or a nucleic acid molecule generated from a proximity probe detection assay. In further embodiments, the target nucleic acid molecule(s) may be a nucleic acid domain(s) conjugated to one or more proximity probes. In still further embodiments the target nucleic acid molecule may be a DNA molecule or a RNA molecule in a tissue or cell sample or a DNA or RNA molecule isolated therefrom.

Accordingly, at its broadest, the disclosure can be seen to provide a probe for use in detecting a target analyte (e.g. nucleic acid molecule) in a sample, wherein the probe provides or is capable of providing nucleic acid components sufficient to initiate a rolling circle amplification (RCA) reaction, said probe being a nucleic acid construct comprising:
(i) one or more target binding domains capable of binding to said target analyte or an intermediate molecule bound, directly or indirectly, to the target analyte;
(ii) one or more domains together comprising or capable of providing a circular or circularisable RCA template;
(iii) a domain comprising or capable of providing a primer for said RCA reaction that hybridizes to a region of said circular or circularisable RCA template; and, when the probe comprises or is capable of providing a circularisable RCA template,
(iv) one or more domains comprising or capable of providing a ligation template that templates the ligation (or circularisation) of the circularisable RCA template,
   wherein at least part of the probe must be cleaved and/or unfolded to release said primer to enable said rolling circle amplification reaction.

It will be understood that each probe generates a single circular RCA template (for the RCA reaction). Thus, it will be understood that in part (ii), where more than one domain provides a circular or circularisable RCA template, each such domain may contribute a part or portion of the ultimate RCA template, which parts or portions are ligated together to form the circle. Accordingly part (b)(ii) may alternatively be expressed as:
(ii) a domain which comprises or provides a circular or circularisable RCA template, or two or more domains which together are capable of providing a circularisable RCA template.

In some embodiments, the target analyte is, or comprises, a nucleic acid molecule and at least one of said target binding domains comprises a region of complementarity to said nucleic acid molecule (i.e. a target complementary domain) or an intermediate molecule bound, (e.g. hybridised) directly or indirectly, to the nucleic acid molecule.

In the probes of the invention and as described in more detail below, the probe of the invention comprises at least two target binding domains, and more particularly said at least two target binding domains are composed of nucleic acid e.g. are target-complementary domains. More particularly, in such embodiments whilst there is more than one target binding domain, only one RCA primer is provided by the probe. Such "multiple" target-binding domain probes may be both "circle" and "releasable RCA template" ("linear" or "hairpin") probes are described in more detail below.

In some embodiments, the probe is unable to generate a RCA product unless the probe is bound to said target or intermediate molecule, i.e. the probe may be viewed as a target dependent RCA probe, i.e. a RCA product is generated only in the presence of a target analyte, e.g. nucleic acid molecule. For instance, the primer and/or circular or circularisable RCA template may be unable to participate in, or initiate, the RCA reaction because, in the absence of the target or intermediate analyte, e.g. nucleic acid molecule, the RCA nucleic acid components are not in sufficient proximity to enable a RCA reaction to proceed, e.g. the probe may rely on a target dependent ligation reaction to generate a RCA product. In some embodiments the primer and/or circular or circularisable RCA template may be inaccessible (e.g. unavailable) for a rolling circle amplification reaction when the probe is not bound to said target or intermediate molecule, e.g. the probe may rely on a target dependent cleavage and/or unfolding to release one or more RCA nucleic acid components.

It will be evident that, in many embodiments, the probes of the invention will interact with a nucleic acid molecule, either as the target analyte or as a marker for the target analyte. Accordingly, the probes are described with respect to this type of interaction, i.e. the target binding domains are nucleic acid domains comprising a region of complementarity to a target nucleic acid molecule. However, as mentioned above, it is envisaged that some of the probes of the invention may comprise target binding domains that can interact with any analyte, i.e. the target binding domains may be formed by coupling the probe to one or more analyte-binding domains. The skilled person would readily be able to adapt the probes described herein, particularly the target binding domains, to incorporate one or more analyte-binding domains to enable the probe to detect an analyte as defined below.

Target dependent probes of the invention, i.e. probes that require an interaction with the target or intermediate molecule to generate a RCA product, may be particularly useful in, but not limited to, homogeneous reactions. Probes that do not rely on an interaction with the target nucleic acid molecule to generate a RCA product are particularly useful in heterogeneous assays, in which probes and/or RCA products that are not bound to target nucleic acid molecules (e.g. excess probes or non-target bound products) may be removed from the sample, e.g. by washing, prior to detection of the RCA product.

In some embodiments, the primer and ligation template may be provided by the same part of the probe, i.e. the same domain/sequence may function as the primer and ligation template. It will be evident that in embodiments that utilise a circularisable RCA template, the RCA template must be ligated to form a circular nucleic acid molecule in order that it can function as a template for RCA, i.e. in a probe dependent ligation.

It will be apparent from the description below that there are a large number of probe permutations and it is not feasible to provide a detailed description of each embodiment. Nevertheless, some exemplary embodiments are described below and it will be clear that other probes that fall within the scope of the claimed invention may be produced by combining features of these exemplary embodiments and this is readily achievable by a person of skill in the art, based on the description of the invention herein.

The probes of the invention are formed of, or may comprise, one or more nucleic acid molecules, i.e. one or more nucleic acid strands, e.g. one, two, three or more nucleic acid strands. Accordingly, whilst the probe of the invention may be viewed as a single nucleic acid molecule, it may more appropriately be considered to be (or to comprise) a nucleic acid construct, wherein when the probe comprises more than one nucleic acid strand, each strand comprises a region complementarity to at least one other strand in the construct such that the strands are prehybridized (hybridized before the probe is contacted with a sample, i.e. each nucleic acid strand of the probe is not added to the sample separately) so that the probe may be provided as (or may comprise) a single nucleic acid construct. Thus, the probe of the invention may be (or may comprise) a single stranded nucleic acid molecule (i.e. a continuous nucleic acid strand), comprising at least two hairpin structures, which are defined in detail below. Whilst the hairpin structures form regions in the probe that are double stranded, the probe may be regarded as single stranded because it is formed of a single continuous nucleic acid molecule, i.e. all of the nucleotides in the molecule are joined by covalent bonds, i.e. phosphodiester linkages. In other embodiments, the probe may be (or may comprise) a partially double stranded nucleic acid molecule, i.e. comprising at least two nucleic acid strands that are joined by one or more regions of complementarity (by hydrogen bonds, i.e. standard base pairing, as described below), wherein at least part of at least one of the nucleic acid strands is single stranded. In some embodiments, at least one of the strands of the partially double stranded construct is a circle or pre-circle oligonucleotide (a RCA template). In some embodiments, one strand of the partially double stranded molecule comprises at least one hairpin structure and a second strand is provided as an oligonucleotide that is hybridized to a part of the first strand that does not form a hairpin structure. More than two nucleic acid strands may be hybridized together to form a probe of the invention, e.g. 3, 4, 5 or more nucleic acid strands.

In some embodiments, the probe may be coupled to (i.e. conjugated to) one or more analyte-binding domains to form the target binding domains of the probe. Thus, in some embodiments the probe may comprise a nucleic acid construct. Alternatively viewed, the nucleic acid construct probe may comprise one or more analyte-binding molecules or domains, e.g. one or more antibodies.

The probes of the invention may fall into one of two categories, depending on the form in which the RCA template is provided by the probe.

In a first category, the probes comprise a RCA template, i.e. the RCA template is provided as one of the nucleic acid strands of the nucleic acid construct, i.e. the RCA template is provided as a pre-formed circle oligonucleotide or pre-circle (i.e. circularisable) oligonucleotide. Accordingly, these probes may be referred to generally as circle or pre-circle RCA probes, or RCA template probes, wherein the term "circle RCA probes" may be used to refer both to probes comprising a circular and circularisable RCA template.

In a second category the probes provide a RCA template, i.e. the RCA template is generated by the cleavage of the probe into multiple parts that are held in proximity by the target binding domain of each part of the probe. Thus cleavage of the probe, and optionally unfolding, is required to generate the RCA template, and not just the primer for RCA. Hence, these probes may be viewed as "multiple part probes", e.g. two-part probes, three-part probes etc. In some embodiments, the RCA template is generated by the cleavage of a hairpin structure, e.g. a stem loop domain. Accordingly, these probes may be referred to generally as hairpin or stem loop RCA probes. In other embodiments, the RCA template is generated by the cleavage of a linear domain, i.e. a domain without intramolecular complementary. Accordingly, these probes may be referred to generally as linear RCA probes. Both hairpin RCA probes and linear RCA probes may be referred to as pre-RCA template probes or releasable RCA template probes.

Whilst both circle RCA probes, linear RCA probes and hairpin RCA probes may require a probe dependent ligation event to generate the RCA template, it is preferred that the RCA template of circle RCA probes is in the form of a preformed circle oligonucleotide. Hence, in one embodiment circle RCA probes also may be viewed as probe templated ligation independent RCA probes (although these probes may still be target templated ligation dependent probes). In contrast, the hairpin RCA probes and linear RCA probes require a probe dependent ligation event to form the RCA template and hence also may be viewed as probe templated ligation dependent RCA probes.

As discussed below, in some embodiments the circle RCA probes may comprise a hairpin or stem loop structure. However, the hairpin or stem loop structure in the circle RCA probes is not required to generate the RCA template. In contrast, one of the hairpin structures in the hairpin RCA probes will release the RCA template (i.e. the RCA template is generated from a hairpin structure in a hairpin RCA probe).

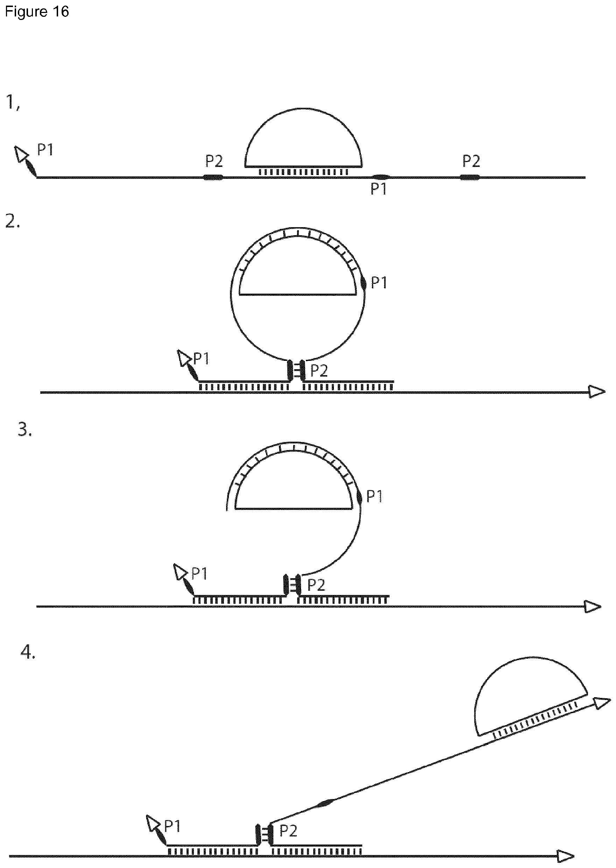

A variant of the "circle RCA probe" described above is shown in Figure 16, in which the probe forms a hairpin structure when the target complementary domains bind to the target nucleic acid molecule, e.g. a stem loop structure forms in between the target complementary domains. The stem loop structure forms a cleavage domain, wherein cleavage of the probe releases the RCA primer. Thus, the probe requires a target dependent cleavage to release a RCA component, although the cleavage recognition site does not need to involve the target nucleic acid molecule directly.

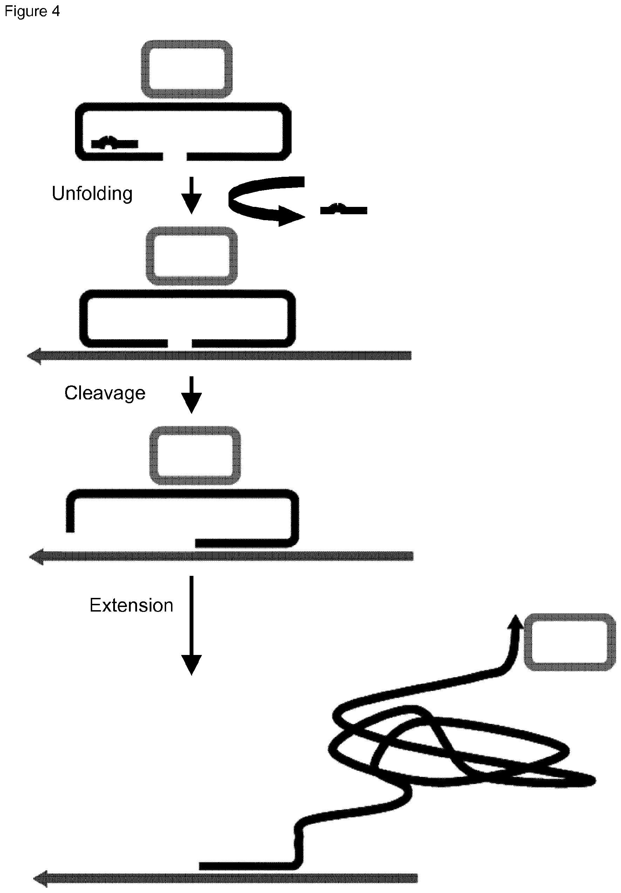

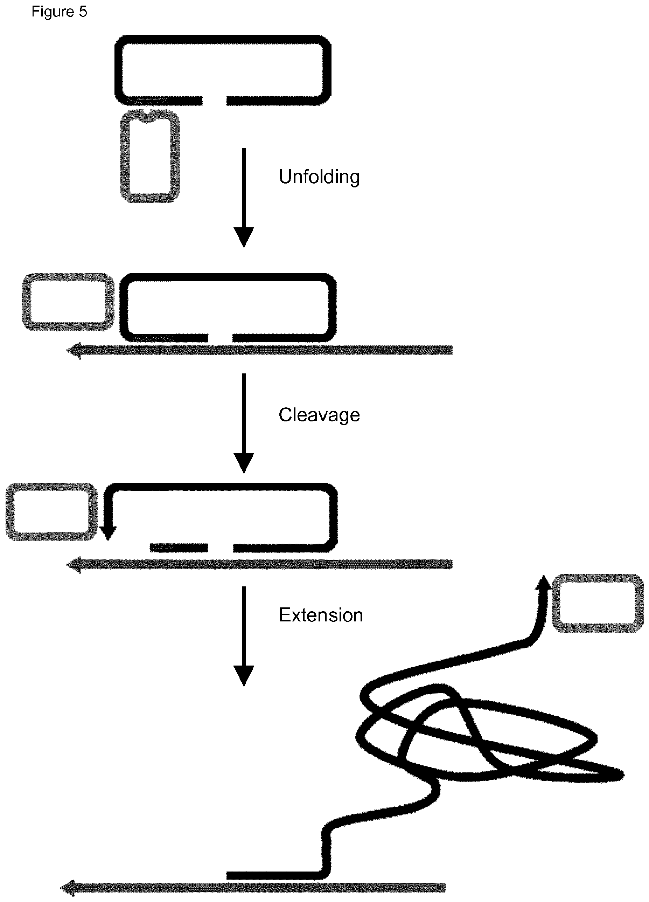

Two further exemplary embodiments of "circle RCA probes" are shown in Figures 4 and 5, which are variants of the embodiment described above and shown in Figure 1. Optionally, in these embodiments, the target complementary domains of the first nucleic acid strand (the primer strand) hybridize to the target nucleic acid molecule such that the 5' and 3' ends of probe are directly or indirectly ligatable, such that the probe may be ligated to form a circular nucleic acid molecule using the target nucleic acid(s) as a ligation template.

The target complementary domain at the 3' end of the first nucleic acid strand comprises a sequence that is recognised by a cleavage enzyme when bound to the target nucleic acid molecule, i.e. the domain comprises a cleavage recognition site. Preferably, the cleavage recognition site is functional, i.e. recognized and cleaved by a cleavage enzyme, when the domain is double stranded. Many enzyme cleavage recognition sites comprise symmetrical, i.e. palindromic, sequences and this can be problematic when multiple single stranded molecules comprising the same cleavage site are present in a sample, i.e. probes comprising palindromic cleavage recognition sequences are likely to form duplexes that are substrates for the cleavage enzyme. Accordingly, if the cleavage recognition site comprises a symmetrical, i.e. palindromric, sequence, it may be advantageous to block the cleavage recognition sites to avoid target independent cleavage of the probe, which may result in the generation of RCA products in the absence of target nucleic acids in the sample.

Thus, in some embodiments the probe may comprise a third nucleic acid strand, a protective or blocking strand (Figure 4), that is capable of hybridizing to the cleavage recognition site such that it does not form a site that is recognized or cleaved by the cleavage enzyme, i.e. the protective strand (an oligonucleotide) is complementary to the region of the target complementary domain comprising the cleavage recognition site but does not form a functional cleavage domain or cleavable site. Hence, the protective or blocking strand is partially complementary to the cleavage recognition site. The protective strand must interact with (hybridize to) the cleavage recognition site more strongly than the interaction between the palindromic sequences of two probes, e.g. the protective strand may comprise a region of complementarity to the probe that is longer than the cleavage recognition site.

In the presence of the target nucleic acid molecule, the target complementary regions of the probe hybridize to the target site. The target complementary domain comprising the cleavage recognition site forms a more stable interaction with the target nucleic acid molecule than it does with the protective strand, which is displaced (i.e. the probe is "unfolded"). The duplex between the target complementary domain and the target nucleic acid molecule forms a functional cleavage recognition site which may be cleaved by a cleavage enzyme, e.g. an endonuclease such as a nickase, or result in the cleavage of a sequence adjacent to the cleavage recognition site in the probe. In the presence of an enzyme that recognises the cleavage recognition site, the first strand of the probe is cleaved (in some embodiments, both the first nucleic acid strand of the probe and the target nucleic acid molecule may be cleaved), which results in an extendable 3' end that is not hybridized to the target nucleic acid molecule (i.e. a single stranded domain). The single stranded part of the first strand of the probe may be degraded, e.g. by exonuclease digestion, and thereby results in the release of the primer domain. The primer domain is extended using the circular nucleic acid molecule (RCA template) as a template for polymerisation to generate the RCA product which is attached or secured to the target nucleic acid by the 5' target complementary domain of the probe.

In some embodiments, the RCA template (i.e. the circle strand) may act as a protective or blocking strand (Figure 5). When the probe interacts with the target nucleic acid molecule to form the functional cleavage site, the RCA template is displaced to another region of the first nucleic acid strand (the primer strand), which is also complementary to the RCA template (the so-called displacement region or domain). This displacement may be viewed as an unfolding step. The RCA template interacts with the target complementary domain of the probe that comprises the cleavage recognition site more strongly than the displacement region of the probe, such that the RCA template is only displaced (the probe is only unfolded) when the probe interacts with the target nucleic acid sequence.

In some embodiments, the cleavage recognition site is not a symmetrical sequence. Accordingly, a blocking or protective strand may not be required.

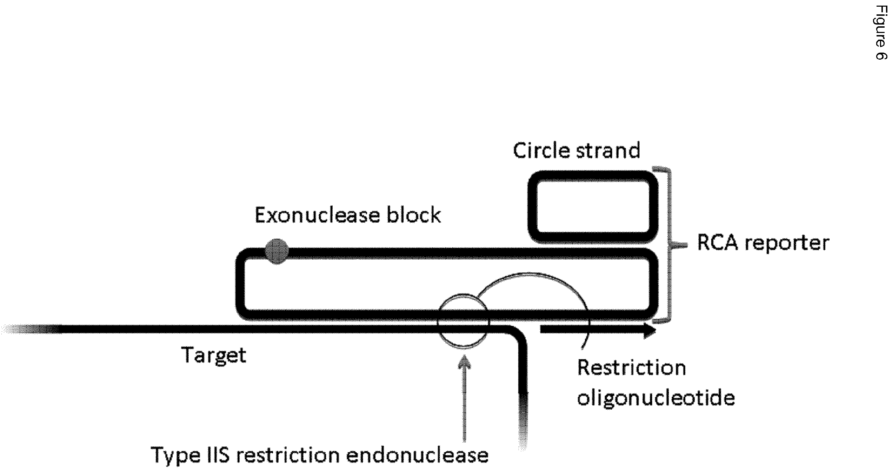

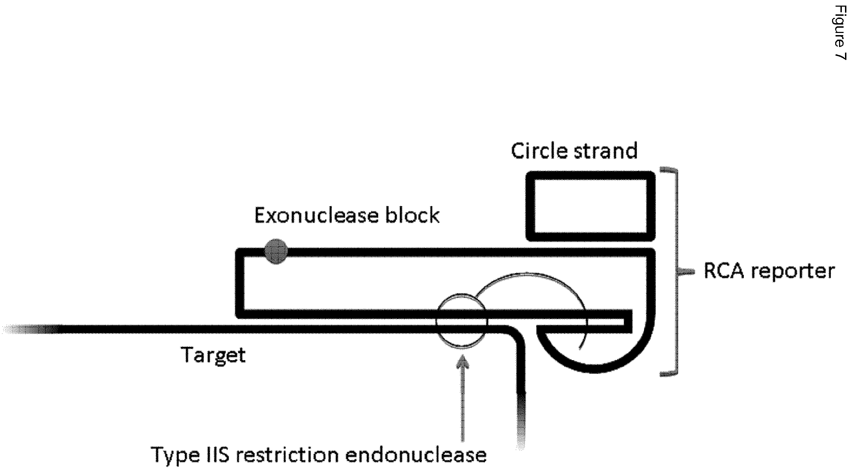

Still further embodiments of the circle RCA probes are shown in Figures 6 and 7. In these exemplary embodiments, both the primer strand and the circle strand are circular oligonucleotides. The primer strand comprises a target complementary region, which forms a functional cleavage recognition site when it hybridizes to the target nucleic acid molecule. In some embodiments, a cleavage enzyme that recognises the cleavage recognition site is able to cleave the primer strand at a site adjacent to the cleavage recognition site, e.g. using a Type IIS restriction endonuclease. This is particularly useful to avoid cleavage of the target nucleic acid molecule, which may be advantageous in some embodiments, e.g. when the target nucleic acid molecule comprises multiple probe binding sites, such as a RCA product. The site at which cleavage of the primer strand occurs (the cleavable domain) may be formed by a restriction oligonucleotide (defined below), which forms a third strand of the nucleic acid construct, i.e. a restriction strand or cleavage strand (Figure 6). Alternatively, the primer strand may comprise a hairpin structure that forms a cleavable domain (Figure 7). As described above, cleavage of the primer strand of the RCA probe, and subsequent exonuclease degradation, releases the primer which can be extended by RCA template dependent polymerisation. In some embodiments exonuclease degradation is not required and cleavage of the primer strand may release directly a portion of the primer strand that can function as a primer for the RCA reaction.

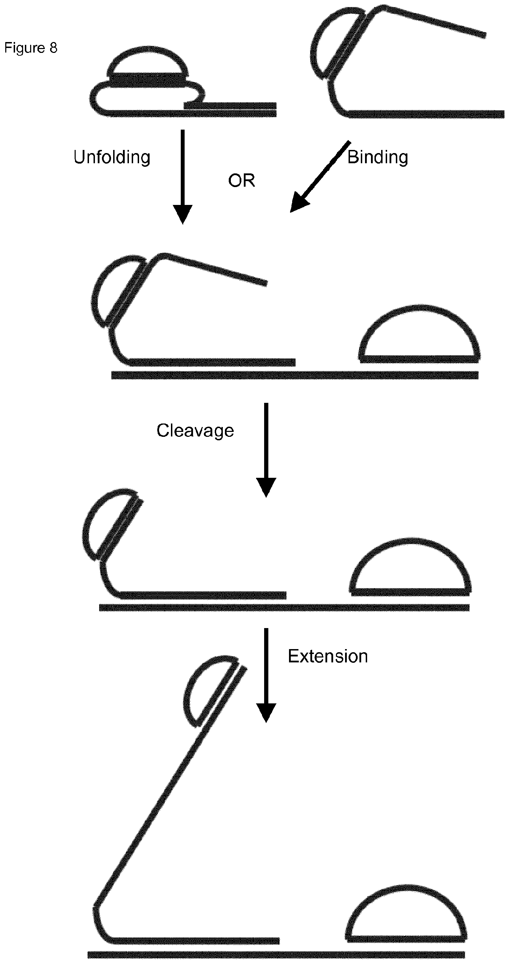

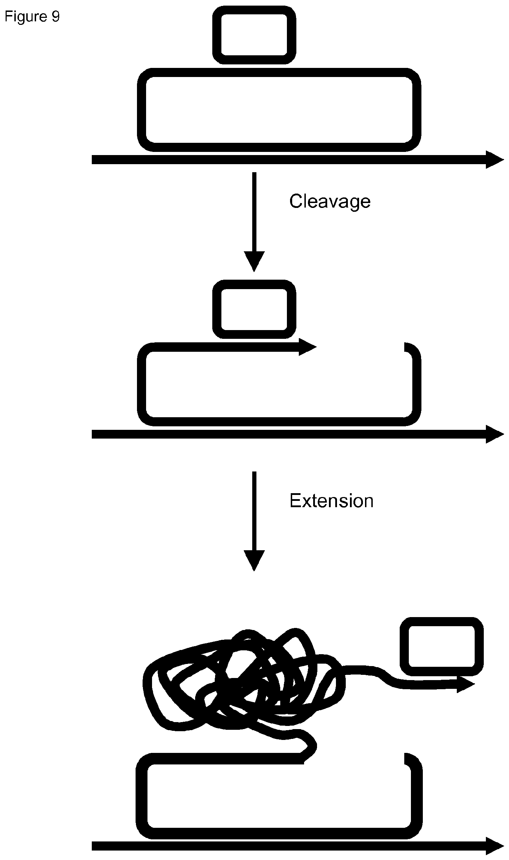

A still further exemplary embodiment is shown in Figure 8. The probe provides a first nucleic acid strand, the primer strand, which in some embodiments may be in the form of a hairpin structure. In other embodiments, the primer stand may be in the form of a circular oligonucleotide (Figure 9). The probe also provides a second nucleic acid strand, the circle strand, which is in the form of a circular nucleic acid molecule that is hybridized to a region of the first nucleic acid strand, e.g. the single stranded loop of the hairpin structure (Figure 8).

Before the probe interacts with, e.g. hybridizes to, the target nucleic acid molecule the 3' end of the first nucleic acid molecule (or a part of the nucleic acid molecule located towards the 3' end) may be hybridized to at least part of the 5' end of the first nucleic acid strand (i.e. to at least part of the target complementary domain) so as to form a hairpin structure, e.g. a stem loop. This may act to protect one or both ends of the primer strand from degradation, e.g. digestion by components in the sample with exonuclease activity. However, it is not essential that the first nucleic acid strand of the probe forms a hairpin structure, i.e. in some embodiments the primer strand may comprise no intramolecular complementarity.

In the presence of the target nucleic acid molecule, the target complementary region of the primer strand hybridizes to the target site. Where the primer strand of the probe is provided in the form of a hairpin structure, the interaction between the probe and the target nucleic acid is sufficient to destabilize the hairpin structure, i.e. the duplex between the target complementary region of the primer strand and the target nucleic acid is more stable than the duplex between the 5' and 3' ends of the primer strand. For instance, the duplex between the target complementary region of the primer strand and the target nucleic acid may be longer than the duplex between the 5' and 3' ends of the primer strand.

The primer strand comprises a cleavage site that forms part of, or is adjacent to, the site at which the RCA template is hybridized (i.e. 3' to the site at which the RCA template is hybridized). Cleavage of the cleavage site, e.g. exonuclease degradation of the 3' of the primer strand, results in the release of the RCA primer, which can be extended using the RCA template strand as a polymerisation template to generate the RCA product.

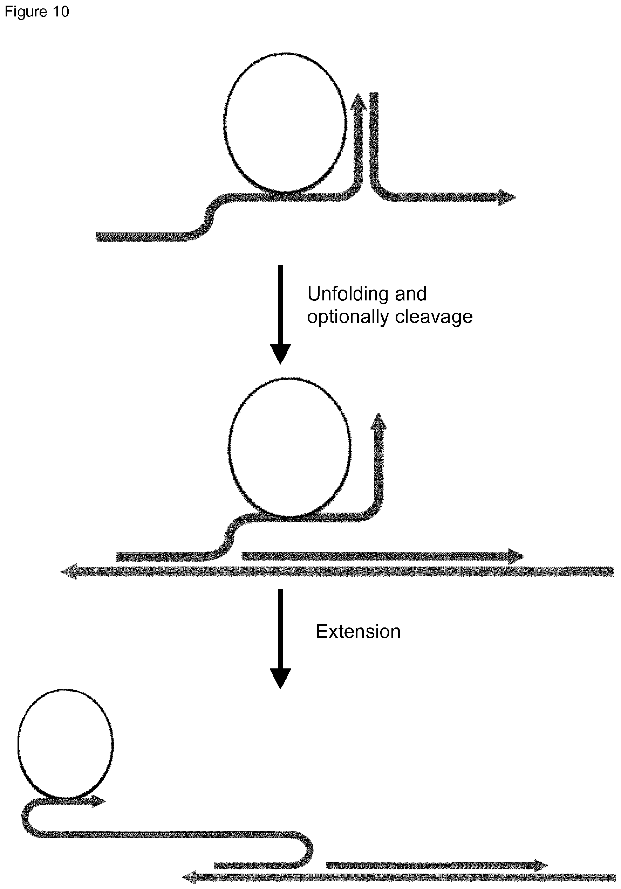

A further circle RCA probe exemplary embodiment is shown in Figure 10, wherein the probe comprises three nucleic acid strands: a primer strand, circle strand and a blocking strand (which may also be viewed as an invasion strand).

In a preferred embodiment the primer strand comprises a target complementary region at the 5' end and a domain at the 3' end comprising cleavage recognition site (a cleavage domain), which are joined by an internal region of complementarity to the RCA template (the RCA template complementary or binding domain, which may also be viewed as the primer domain or a part thereof). The cleavage domain comprises a region of sequence that is similar or identical to a region of the target sequence (the region of the target sequence directly or indirectly adjacent to the region to which the target complementary region the primer strand is able to hybridize).

At its 5' end the invasion strand comprises a region of complementarity to the cleavage domain and at its 3' end comprises a target complementary domain, wherein the region of complementarity to the cleavage domain is also complementary to a region of the target sequence (the region of the target sequence directly or indirectly adjacent to the region to which the target complementary region of the primer strand is able to hybridize). The invasion strand prevents, i.e. blocks, the cleavage domain from cleavage by a cleavage enzyme, e.g. an exonuclease.

In the presence of the target nucleic acid molecule, the target complementary regions of the probe bind to the target molecule (the probe binding domains of the target molecule). This displaces the invasion strand from the cleavage domain, i.e. the probe unfolds and the invasion strand invades the target nucleic acid, which exposes the cleavage domain to the cleavage enzyme, wherein cleavage of the domain (e.g. degradation of the single stranded 3' end of the probe up to the primer domain, to which the RCA template is hybridized) releases the primer for RCA templated extension.

In some embodiments, the primer strand does not comprise a cleavage domain. Instead, the primer strand comprises a target complementary region at the 5' end, a first region of complementarity to the RCA template (the RCA template complementarity domain) and a second region of complementarity to the RCA template; the second domain functions as the primer for the RCA reaction (the primer domain). The primer domain comprises a region of sequence that is similar or identical to a region of the target sequence (the region of the target sequence directly or indirectly adjacent to the region to which the target complementary region the primer strand is able to hybridize). The primer domain hybridizes more strongly to the invasion strand than it does to the RCA template.

In the presence of the target nucleic acid molecule, the target complementary regions of the probe bind to the target molecule. This displaces the invasion strand from the primer domain, i.e. the probe unfolds and the invasion strand invades the target nucleic acid, which releases the primer domain to bind to the RCA template for RCA templated extension. Hence, the primer may be released by target dependent unfolding of the probe.

Thus, in a second more particular aspect of the invention, the present invention can be seen to provide a probe for use in detecting a target analyte in a sample, wherein said analyte is, or comprises, a nucleic acid molecule and wherein the probe provides or is capable of providing nucleic acid components sufficient to initiate a rolling circle amplification (RCA) reaction, said probe being a nucleic acid construct comprising:
(a) a first strand (i.e. a primer strand) comprising:
   (i) a first domain comprising a target binding domain capable of binding to the target analyte or an intermediate molecule bound, directly or indirectly, to the target analyte (e.g. a target complementary domain);
   (ii) a second domain comprising a region of complementarity to a circular or circularisable RCA template, which domain may provide all or part of a primer for RCA of said RCA template (i.e. a RCA template complementary domain and/or primer domain); and
   (iii) a third domain comprising a cleavage recognition site (i.e. a cleavage domain) or comprising a region of complementarity to a circular or circularisable RCA template which may provide a primer for RCA of said RCA template (i.e. a primer domain),
      and
(b) a second strand (i.e. a circle strand) that is capable of hybridizing to the second domain of the first strand, said second strand comprising:
   (i) a circular RCA template comprising at least one region of complementarity to the first strand (i.e. at least the second domain of the first strand); or
   (ii) a circularisable RCA template comprising two regions of complementarity to the second domain of the first strand, wherein the first region of complementarity is at the 5' end of the circularisable RCA template and the second region of complementarity is at the 3' end of the circularisable RCA template, and wherein said regions of complementarity are capable of hybridizing to the second domain of the first strand such that the 5' and 3' ends are directly or indirectly ligatable,
      wherein cleavage and/or unfolding of at least part of the probe releases a domain to function as the primer for rolling circle amplification.

In some embodiments of this aspect of the invention, the third domain of the primer strand comprises a region of complementarity to the first domain, such that the third domain and first domain hybridize to form a nucleic acid duplex when the first domain is not hybridized to the target nucleic acid or the intermediate molecule. Thus, contacting the probe with the target nucleic acid molecule may be viewed as unfolding the probe. This embodiment may be particularly advantageous to prevent the cleavage of the probe when it is not bound to the target nucleic acid molecule, e.g. wherein the cleavage agent is an exonuclease, particularly a 3' exonuclease.

In some embodiments, the nucleic acid construct comprises a protection strand or protection oligonucleotide. The protection strand or oligonucleotide may prevent or protect the cleavage recognition site or domain of the oligonucleotide from being recognized by the cleavage enzyme. In some embodiments, the protection strand or oligonucleotide may prevent the primer strand from forming a functional cleavage recognition site with another nucleic acid molecule in a sample, e.g. with another probe. In yet other embodiments, the protection strand or oligonucleotide may prevent the primer strand from priming RCA templated extension. In some embodiments, the RCA template functions as a protection strand or oligonucleotide. In some embodiments, the ends of the primer strand may be directly or indirectly ligated.

In some embodiments, the primer strand is a circular nucleic acid molecule. In yet further embodiments, the circular primer strand comprises a hairpin structure. In some embodiments, the nucleic acid construct comprises a restriction oligonucleotide (i.e. a restriction strand or cleavage strand).

In some embodiments, the probe may comprise more than one target binding domain. When the probe comprises more than one target binding domains, one of the target binding domains may be formed by coupling the probe to an analyte-binding domain. This embodiment may be particularly useful when the target analyte comprises a nucleic acid molecule, e.g. where the target analyte is a protein:nucleic acid complex, wherein one target binding domain will bind to the protein component of the analyte and a second target binding domain will bind to the nucleic acid component of the analyte (i.e. the second target binding domain will be a target complementary domain).

In particular embodiments of the "circle RCA probes", where the RCA template (i.e. the circle strand) is in the form of a circle (i.e. a circular RCA template), it is preferred that the circle strand is not catenated to the first (primer) strand. In other words it is not topologically linked, or "padlocked" to the first strand. It is simply hybridises to the first strand.

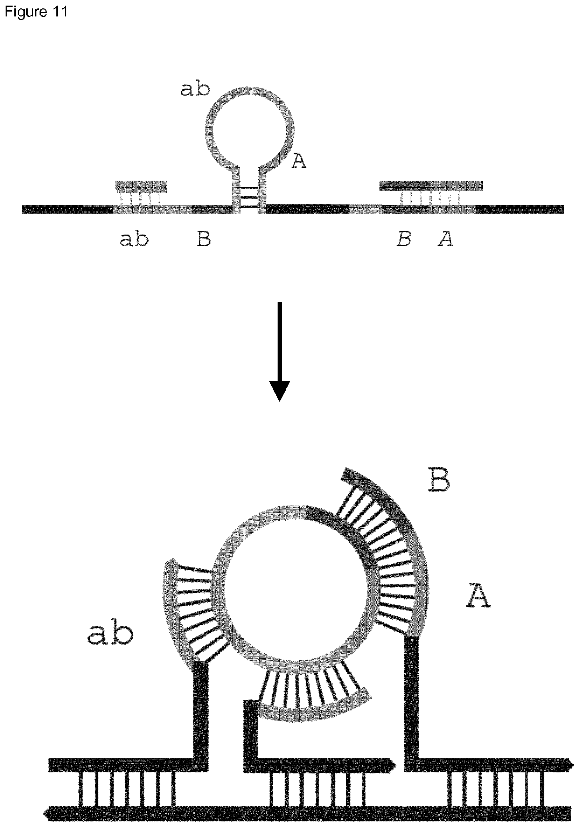

A further exemplary embodiment of a "hairpin RCA probe" is shown in Figure 11, which is a variant of the embodiment described above and shown in Figure 2.

The probe is in the form of a first nucleic acid strand that comprises a hairpin structure and two further nucleic acid strands (second and third strand, i.e. oligonucleotides) that are hybridized to the first nucleic acid strand on either side of the hairpin structure. The double stranded portions of the probe created by the second and third strands act as cleavage recognition sites, e.g. sites or domains that are recognised by one or more cleavage enzymes. Hence, the second and third strands may be referred to as cleavage strands or cleavage oligonucleotides. In some embodiments, these may be known as restriction oligonucleotides or restriction strands as defined below.

The first strand comprises three regions of complementarity to the target, wherein each target complementary domain is directly adjacent to a cleavage recognition site, i.e. directly adjacent to a region of the probe that forms the hairpin structure or a region to which a cleavage strand hybridizes. Each target complementary domain functions to attach or immobilize one of the RCA nucleic acid components to the target nucleic acid molecule.

Following contact with a sample under conditions that allow the probe to hybridize with the target nucleic acid molecule, one or more cleavage agents that recognize the cleavage recognition sites may be added to the sample which results in the cleavage of three sites in the probe, i.e. the loop of the hairpin structure and each double stranded portion of the probe or a region adjacent thereto. Upon cleavage of the probe the hairpin structure is able to unfold (by cleavage of part of the hairpin structure) to provide the RCA template that is attached to the target nucleic acid molecule via hybridization to a portion of the probe comprising a target complementary domain. The primer and ligation template are each provided by the portions of the probe that are attached to the target nucleic acid molecule via the other target complementary domains.

In the presence of the target nucleic acid molecule(s), the RCA nucleic acid components are directly or indirectly hybridized to the target nucleic acid molecule(s) (via the target complementary (i.e. target-binding) domains) thereby maintaining the RCA nucleic acid components in close proximity. The RCA template hybridizes to both the primer and ligation template. The ligation template part of the probe templates the circularisation of the RCA template in the presence of a ligase enzyme. The primer part of the probe can be extended using the circularised RCA template as a polymerisation template to generate the RCA product. It will be evident that in the absence of a target nucleic acid molecule the cleaved parts of the probe that form the RCA nucleic acid components are not maintained in proximity, thereby preventing the production of a RCA product in the absence of target nucleic acid.

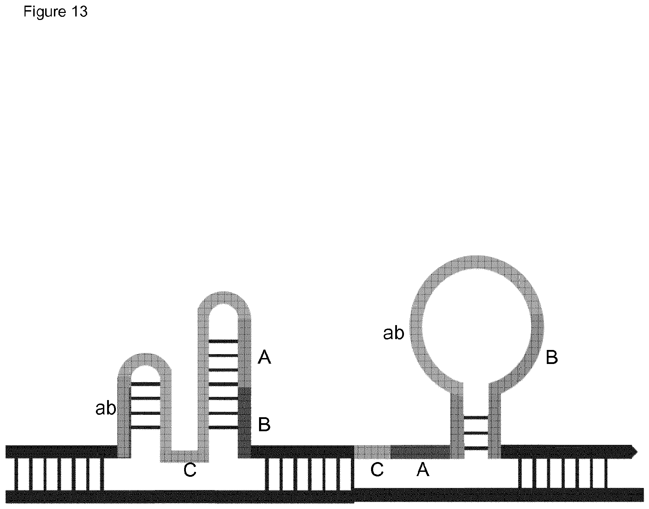

In yet further embodiments, one or both of the primer and ligation template domains may be provided as hairpin structures, see e.g. Figures 12 and 13. It will be evident that the primer and ligation domains may be provided by a combination of hairpin structures and cleavage oligonucleotides. However, it may be particularly advantageous for all of the RCA nucleic acid components to be provided as hairpin structures because this allows the probe nucleic acid construct of the invention to be provided as a single stranded nucleic acid molecule, i.e. a single continuous nucleic acid strand that comprises regions of intramolecular complementarity.

Thus, in one embodiment of the invention the probe may be provided as a "two-part" hairpin RCA probe (Figure 12). The probe is in the form of a single nucleic acid strand that comprises two hairpin structures, each comprising a cleavable site and linked to a target complementary domain. In Figure 12A the probe comprises a target complementary domain at each end. In Figure 12B the target complementary domains of the probe are capable of hybridizing to different nucleic acid molecules that are in close proximity, e.g. each end hybridizes to the nucleic acid domain of a proximity probe. It will be evident that the ends of the probe may hybridize to the target nucleic acid molecule(s) such that the 5' and 3' ends of probe are directly or indirectly ligatable, so that the probe may be ligated to form a circular nucleic acid molecule using the target nucleic acid(s) as a ligation template.

Following contact with a sample under conditions that allow the probe to hybridize with the target nucleic acid molecule, a cleavage agent may be added to cleave the cleavage sites in the hairpin structures. The hairpin structures unfold to yield the nucleic acid components needed for a RCA reaction. In one embodiment the primer and ligation template are provided by a first hairpin structure (i.e. the primer also functions as the ligation template, or *vice versa*) and the RCA template is provided by a second hairpin structure. In another embodiment, the ligation template is provided by a first hairpin structure and the primer and RCA template are provided by a second hairpin structure.

In the presence of the target nucleic acid molecule, and after cleavage of the probe, the primer and ligation template domains form part of the probe that comprise the target complementary domains. Accordingly, the primer and ligation template domains are hybridized to the target nucleic acid molecule thereby maintaining the RCA nucleic acid components in close proximity. The RCA template hybridizes to both the primer and ligation template. In embodiments where the primer domain also functions as the ligation template, the RCA template is hybridized to a portion of the probe comprising a target complementary domain. As described above, the ligation template part of the probe templates the circularisation of the RCA template in the presence of a ligase enzyme. The primer part of the probe can be extended using the circularised RCA template as a polymerisation template to generate the RCA product. It will be evident that in the absence of a target nucleic acid molecule the cleaved parts of the probe that form the RCA nucleic acid components are not maintained in proximity, thereby preventing the production of an RCA product in the absence of target nucleic acid.

A "three-part" probe is shown in Figure 13, which is form of a single nucleic acid strand that comprises three hairpin structures, each comprising a cleavable site and linked to a target complementary domain. It will be evident that this probe functions similarly to the embodiment shown Figure 11 and described above. The difference is that the cleavage oligonucleotides are replaced by hairpin structures.

An exemplary embodiment of a two-part "linear RCA probe" is depicted in Figure 14G, which is a variant of the embodiment described above and shown in Figure 2.

The probe is in the form of two nucleic acid strands and comprises a single cleavage domain. A first strand provides the RCA template and the second strand may be viewed as the cleavage strand. In Figure 14G the probe comprises a first target complementary domain at the 5' end and a second target complementary domain near the 3' end. In Figure 14G the target complementary domains of the probe are capable of hybridizing to different nucleic acid molecules that are in close proximity, e.g. each end hybridizes to the nucleic acid domain of a proximity probe, but it will be apparent that the target complementary domains could bind to adjacent probe binding site on the same nucleic acid molecule. Following contact with a sample under conditions that allow the probe to hybridize with the target nucleic acid molecule, a cleavage agent may be added to cleave the cleavage site between the target complementary domains. The parts of the probe that are not complementary to the target nucleic acid molecule form the nucleic acid components needed for a RCA reaction. For instance, part of the probe that is attached to the first target complementary domain forms the ligation template and RCA primer. The second target complementary domain forms the RCA template, i.e. the ends of domains on either side of the second target complementary domain may bind to the ligation template such that they are directly or indirectly ligatable.

In the presence of the target nucleic acid molecule, and after cleavage of the probe, the ligation template/RCA primer and RCA template domains form part of the probe that comprise the target complementary domains. Accordingly, the ligation template/RCA primer domain and RCA template domain are hybridized to the target nucleic acid molecule thereby maintaining the RCA nucleic acid components in close proximity. As described above, the ligation template/RCA primer part of the probe templates the circularisation of the RCA template in the presence of a ligase enzyme. The ligation template/RCA primer part of the probe can be extended using the circularised RCA template as a polymerisation template to generate the RCA product. It will be evident that in the absence of a target nucleic acid molecule the cleaved parts of the probe that form the RCA nucleic acid components are not maintained in proximity, thereby preventing the production of an RCA product in the absence of target nucleic acid.

The "two-part" and "three-part" aspects of the hairpin RCA probes and linear RCA probes described above refer to the number of target complementary domains, wherein cleavage of the probe acts to disconnect the RCA nucleic acid components that are directly or indirectly attached to each target complementary domain. Accordingly, it will be evident that the "three-part" probes cannot be involved in a target templated ligation, whereas this is a preferred aspect of the "two-part" probes.

Further examples of "two-part" probe designs are shown in Figure 14. As described above, each linear or hairpin RCA probe provides the nucleic acid components sufficient to initiate a RCA reaction, i.e. a primer, ligation template and RCA template and from the embodiments exemplified in the Figures it can be determined that a domain that provides a RCA template is adjacent to a domain that provides a ligation template. However, a domain that provides a primer can be adjacent to a domain that provides a RCA template or a domain that provides a ligation template.

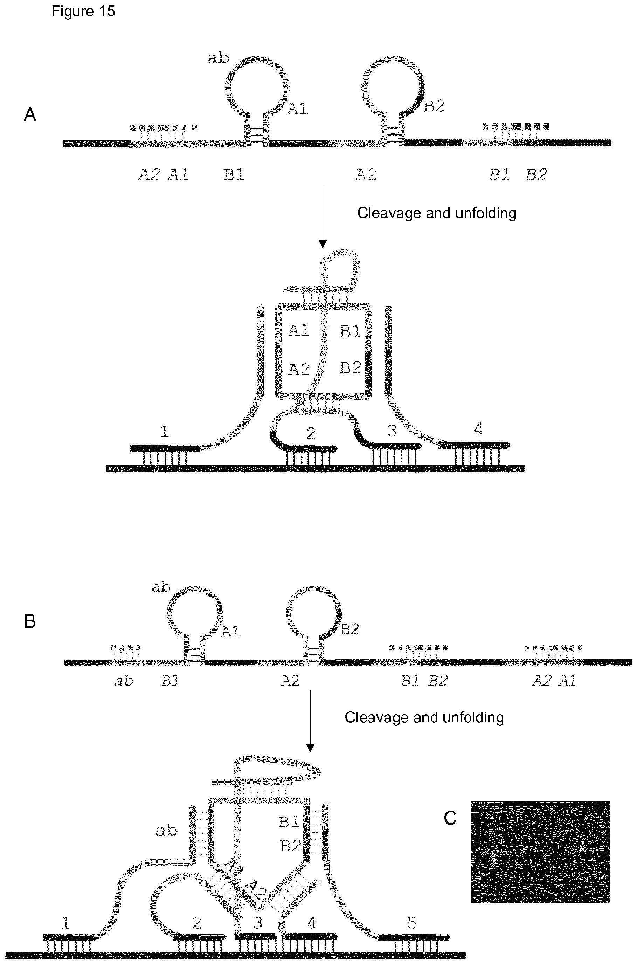

It will be apparent that the "two-part" and "three-part" probes described above are exemplary and it is possible to provide the RCA components in more parts, e.g. 4, 5 parts etc. Hence, the linear and hairpin RCA probes may be multiple part probes, wherein each RCA component is attached to a separate target complementary domain after probe cleavage. In this respect, the RCA template may be provided in more than one part, e.g. two or three parts etc, which may be ligated to form a circular oligonucleotide. In this respect, the number of parts in which the RCA template is provided will determine the number of ligation templates provided by the probe, i.e. if the RCA template is provided in two parts, e.g. two half-circles, the probe will provide two ligation templates, wherein one of the ligation templates may also function as the RCA primer (e.g. a four-part probe) or the RCA primer may be provided separately (e.g. a five-part probe). Four-part and five-part probes are shown in Figure 15.

Thus, in another more particular aspect of the invention, the present invention can be seen to provide a probe for use in detecting a target analyte (e.g. a nucleic acid molecule) in a sample, wherein the probe provides or is capable of providing nucleic acid components sufficient to initiate a rolling circle amplification (RCA) reaction, said probe being a nucleic acid construct comprising:
(i) at least two domains each comprising target binding domain capable of binding to the target analyte or an intermediate molecule bound, directly or indirectly, to the target nucleic acid molecule;
(ii) one or more domains together capable of providing a RCA template, ;
   and
(iii) at least one domain capable of providing a ligation template and/or primer, said domain comprising;
   (a) a region of complementarity to a sequence within the probe, such that it forms part of a hairpin structure that comprises a cleavage recognition site; or
   (b) a region of double stranded nucleic acid that comprises a cleavage recognition site,
      wherein
      (1) each domain capable of providing a RCA nucleic acid component is directly or indirectly attached to the target nucleic acid via a target binding domain;
      (2) the domain capable of providing the RCA template is adjacent to the domain capable of providing the ligation template; and
      (3) cleavage of the probe releases (or is sufficient to release) said RCA nucleic acid components to enable a RCA reaction when the target complementary domains are hybridized to the target analyte or intermediate molecule.

In some embodiments, the target analyte is, or comprises, a nucleic acid molecule and at least one of said target binding domains comprises a region of complementarity to said nucleic acid molecule (i.e. a target complementary domain) or an intermediate molecule bound, (e.g. hybridised) directly or indirectly, to the nucleic acid molecule.

In the hairpin RCA probe embodiments, the probe comprises one or more domains together capable of providing a RCA template, wherein one or more said domains comprise a region of complementarity to a sequence within the probe, such that it forms part of a hairpin structure that comprises a cleavage recognition site.

In some embodiments the target binding domains are target complementary domains that hybridize to the target or intermediate nucleic acid molecule such that the 5' and 3' ends of the probe are directly or indirectly ligatable.

In some embodiments, the domain capable of providing the RCA template also provides the primer for the RCA reaction, in which case the domain capable of providing the ligation template and/or primer only provides the ligation template. Accordingly, in some embodiments the ligation template and primer domains are provided as separate domains.

In an exemplary embodiment, each RCA nucleic acid component is provided by a separate domain, wherein the probe comprises a further domain capable of providing a ligation template or primer, said domain comprising;
(a) a region of complementarity to a sequence within the probe, such that it forms part of a hairpin structure that comprises a cleavage recognition site; or
(b) a region of double stranded nucleic acid that comprises a cleavage recognition site.

A region of double stranded nucleic acid that comprises a cleavage recognition site is provided by a nucleic acid strand that is hybridized to a region of the first strand of the probe, wherein the first strand may be viewed as the strand comprising the domain capable of providing a RCA template. Hence, as discussed above, the probe may comprise one or more cleavage oligonucleotides or restriction oligonucleotides.

In some embodiments, it may be desirable to prevent the probe from using the target nucleic acid molecule as an extension template. This may be particularly beneficial when the target nucleic acid molecule comprises multiple binding sites for the probe of the invention, i.e. multiple sites to which the target complementary domain(s) of the probes may bind (directly or indirectly), e.g. where the target nucleic acid molecule is a RCA product. If the probe comprises an extendable 3' end that can participate in a target templated extension reaction, the extension product may displace probes bound to the target nucleic acid molecule as it is extended. This may be particularly problematic in embodiments wherein it is desirable or necessary for the RCA product to be attached or immobilized to the target nucleic acid molecule, e.g. in heterogeneous embodiments, i.e. *in situ* or localized detection, or for signal amplification of a first RCA product, as described further below, where signal amplification is achieved by attaching a second RCA product to a first RCA product (a so-called super RCA (sRCA) reaction).

It can be seen from the Figures and the exemplary embodiments described above that the probe of the invention may comprise a 3' end that hybridizes to the target nucleic acid molecule, e.g. wherein the probe comprises a target complementary domain at its 3' end. Similarly, cleavage of the probe may result in one or more parts of the probe comprising an extendable 3' end that is hybridized to the target nucleic acid molecule. In the circle RCA probe embodiments it will be apparent that, if the RCA template strand is dissociated from the primer strand, the primer strand may initiate a target templated extension reaction, e.g. the probe may be degraded up to the target complementary domain at the 5' end of the probe such that the domain comprises an extendable 3' end that can be extended by a polymerase using the target as a template for extension. Accordingly, in some embodiments it may be useful to include one or more blocking groups in the probe to prevent unwanted target templated extensions. Additionally or alternatively, it may be useful to utilise non-displaceable oligonucleotides (displacement resistant or immobilizable oligonucleotides) in the methods of the invention, described below. The non-displaceable oligonucleotides ("blocking oligonucleotides") may bind to regions of the target nucleic acid molecule in between probe binding domains in the target. Accordingly, any target templated extension product/reaction would be blocked by the non-displaceable blocking oligonucleotides before the extension product displaces a probe of the invention.

The probes of the invention may include blocking groups in one or more positions, which may be dependent on the design of the probe. For instance, a portion of the target complementary domain at the 5' end and/or 3' end of the probe may be modified so that it cannot be displaced by a strand displacement polymerase, or by an extending strand, e.g. one or more of the target complementary domains of the probe may comprise an exonuclease block and/or a displacement block.

The 3' end of the probe may be modified so that it cannot function as a primer. For instance, the probe may be designed so that the 3' end is not complementary to the target nucleic acid molecule and therefore cannot participate in a target templated extension reaction. The non-target complementary part of the probe may also comprise an exonuclease block.

In some embodiments, particularly with regard to the circle RCA probes of the invention, it may be useful to include an exonuclease block in the portion of the primer strand between the 5' target complementary domain and the RCA template complementary domain/primer domain.

Thus, various means and procedures may be used, singly or in combination, depending on the precise nature of the method steps and probe design employed. For example, modifications (e.g. blocking groups or modified residues) can be incorporated into the probe, which inhibit polymerase and/or exonuclease action (i.e. which inhibit extension and/or degradation), or which inhibit strand displacement. To prevent unwanted exonuclease digestion of any hybridised probes or probe components (i.e. after cleavage of the probes) from creating a primer capable of priming on the target nucleic acid molecule, the presence of any reagents having exonucleolytic activity can be avoided, for example an exonuclease-deficient polymerase can be used. In certain embodiments of the method of the invention washing steps may be used. For example, in the case of a probe which is designed to have one or more ligatable ends which hybridise to the target nucleic acid molecule in juxtaposition for ligation, any probes which have hybridised but not ligated may be removed by stringent washing (according to principles well known in the art). This is particularly applicable in the case of heterogeneous, or solid phase-based methods. Any combination of such means may be employed.

In the case of probes or probe components (i.e. following cleavage of the probe) having a 3 'end which hybridises to the target nucleic acid molecule, where this 3' end is not required for ligation, a modification or block may be included at or near the 3' end which acts to inhibit extension (e.g. a "polymerase-block" or "extension block"). Alternatively or additionally a blocking oligonucleotide may be used, to prevent any extension which may occur from the 3' end from extending into and displacing any downstream probes. As noted above, such a blocking oligonucleotide will itself be modified to incorporate an extension and/or degradation block (e.g. at the 3 'end) and a displacement block (e.g. at the 5' end).

In certain embodiments a probe may comprise a 3 'end which hybridises to the target nucleic acid molecule and is required for ligation (e.g. to the 5' end of the probe). In such a situation it would not be appropriate to include an extension and/or degradation block at the hybridised 3 'end, in order to ensure that the 3' end is available for ligation. In this case, unwanted 3' extension of any unligated 3' ends may be inhibited by stringent washing to remove any unligated probes. Alternatively or additionally, in such a case the probe may be modified at or near the hybridised ligatable 5' end to include a displacement block. In such a case any extension which does take place from the 3 'end will not be to displace the hybridised 5 'end.

Blocking oligonucleotides which may inhibit unwanted extension reactions are described in the literature, for example in Olasagasti et al., 2010, Nature Nanotechnology, 5, 798-806 and in the Senior Thesis of Rashid, at the University of California, Santa Cruz, entitled Blocking Oligomer Design (3/10-3/11).

Any suitable blocking group may be used, such as a nucleotide modification, e.g. modification of a nucleotide with a group that prevents the polymerase from binding to the primer, e.g. by steric hindrance, e.g. biotin, or a group that cannot be processed by the enzyme. In representative embodiments, the nucleic acid molecule to be blocked, e.g. exonucelase blocked, may incorporate any suitable modification known in the art, such as 2'O-Me-RNA residues, Locked Nucleic Acids (LNA), Peptide Nucleic Acids (PNA), phosphothioate-modifed nucleic acids, Polyethylene-linker backbone stretches in between nucleic acids, acridine residues etc. There are several means of modifying nucleic acids so that they are exonuclease resistant and/or do not function as a primer and it is not intended that the methods of the invention are limited to the examples listed above.

The probes of the invention that are ligated using the target nucleic acid molecule as a ligation template (i.e. target templated ligation dependent probes) are particularly advantageous over RCA probes of the prior art, e.g. padlock probes. In this respect, target templated ligation of a padlock probe acts to lock the circularised nucleic acid molecule to the ligation template (i.e. target nucleic acid molecule), which may inhibit RCA templated by the circularised molecule due to topological inhibition. It can be necessary to resolve or release the topological inhibition, e.g. by partial digestion of the target nucleic acid molecule, to allow the RCA reaction to proceed. However, as the probe of the present invention provides the nucleic acid components to enable RCA and the probe is itself extended to generate the RCA product, there is no need to resolve or unlock the probe from the target nucleic acid molecule to generate the RCA product. In fact, target nucleic acid molecule templated ligation of probe may help to facilitate the localisation of the RCA product, i.e. to ensure the RCA product is linked to the target nucleic acid molecule.

Thus the probes of the invention are particularly advantageous for the detection of target nucleic acid molecules that comprise multiple probe binding sites, particularly when it is desirable that the RCA probes are localized to the target nucleic acid. In this respect, although multiple padlock probes may bind to a target nucleic acid molecule that comprises multiple binding sites, the target molecule must be cleaved to allow a RCA reaction to proceed for each probe. Accordingly, the RCA products are not linked to each other, i.e. each RCA product is an extension of a separate part of the target nucleic acid molecule. Alternatively, if the target nucleic acid molecule is not cleaved, only a single padlock probe may generate a RCA product (i.e. a single extension of the 3' end of the target nucleic acid molecule). In contrast, the probes of the present invention do not require the target nucleic acid molecule to be cleaved in order to generate the RCA product. Thus, all of the RCA products generated by the probes of the invention may be attached to the original target nucleic acid molecule. This allows all of the RCA products to be localized in the position of the target nucleic acid, which may result in a stronger signal than a padlock probe and/or to allow a detectable signal to be generated faster than a padlock probe. In embodiments where the target nucleic acid molecule is a RCA product, the probes of the invention may be used in a super-RCA.

In some embodiments it may be useful to design the probe such that the RCA components generated by cleavage of the probe will only remain bound to (i.e. attached to or hybridized to) the target nucleic acid molecule if the ends of the probe have been directly or indirectly ligated by virtue of a target templated ligation. This may be achieved, for example, by designing the probe to ensure that at least one of the target complementary domains (i.e. target binding domains) may hybridize stably only in the presence of another target complementary domain, e.g. one of the target complementary domains may comprise a short region of complementarity to the target molecule. Prior to cleavage of the probe, the combination of the interaction of the target complementary domains (which are joined by the intervening sequence) with the target nucleic acid molecule is sufficient to attach the probe to the target nucleic acid. However, if the target complementary domains are not ligated (directly or indirectly), upon cleavage of the cleavable sites in the probe, at least one of the nucleic acid components of the probe required to enable RCA will dissociate from the target nucleic acid. Accordingly, the RCA reaction will not be able to proceed unless a target templated ligation has occurred.

It will be apparent that a target templated ligation may be intramolecular or intermolecular. Intramolecular ligation is described above, wherein the 5' and 3' ends of the probe hybridize to the target nucleic acid molecule such that the ends may be directly ligated (wherein indirect ligation involves a gap oligonucleotide, as described below). Intermolecular ligation requires an additional nucleic acid molecule, e.g. a stabilization or ligation or "gap" oligonucleotide, which hybridizes to a region of the target nucleic acid molecule adjacent to the 5' or 3' end of a probe. The target complementary region of the probe may be ligated to the gap oligonucleotide using a ligase enzyme, which may prevent the RCA nucleic acid components provided by the probe from dissociating from the target nucleic acid molecule after cleavage of the probe.

As described above, in some embodiments the probe comprises multiple hairpin structures and the methods of using the probes of the invention may advantageously include a step of unfolding the domains of the probe to release the nucleic acid components that are necessary and sufficient to enable RCA. This may be achieved, for example, by altering the conditions of the sample to promote unfolding, e.g. altering the temperature or salt concentrations in the sample. Unfolding may involve displacing one or more strands of the nucleic acid construct, which may be an intramolecular or intermolecular displacement.

As mentioned above, the probe of the invention may find utility in the detection of a nucleic acid molecule in a sample. The nucleic acid molecule may be the target analyte for detection or may be indicative of the presence of the target analyte in a sample. For instance, the nucleic acid molecule may be attached to the target, e.g. a nucleic acid domain of an antibody:nucleic acid conjugate which is bound, directly or indirectly, to the target, e.g. a protein molecule. Similarly, the nucleic acid molecule to be detected may be a nucleic acid molecule generated from the interaction between proximity probes, which are bound to the target analyte, e.g. a protein. In this respect, for the probe to be able to hybridize to the target nucleic acid molecule, directly or indirectly, the nucleic acid molecule (or a nucleic acid molecule bound thereto) must be partially single stranded. In some embodiments, the probe may bind to an analyte or an intermediate molecule bound thereto (i.e. a non-nucleic acid molecule analyte or intermediate molecule) directly, wherein the probe is coupled to one or more analyte-binding domains.

Accordingly, the invention may be seen to provide the use of a probe as defined herein in the detection of an analyte in a sample, wherein said probe interacts with an analyte (or an intermediary molecule bound thereto), preferably an at least partially single stranded nucleic acid molecule, to generate a RCA product (i.e. wherein the probe, more particularly a released nucleic acid component of the probe, is extended to form a RCA product). In embodiments where the probe interacts with an at least partially single stranded nucleic acid molecule, said nucleic acid molecule is:
(i) the analyte;
(ii) directly or indirectly attached to the analyte; or
(iii) indicative of, or a proxy for, (i.e a marker for) the analyte in the sample.

Thus, the invention may also be seen to provide a method for detecting an analyte in a sample comprising:
(a) contacting said sample with a probe as defined herein, wherein said probe interacts with said analyte or an intermediary molecule bound thereto;
(b) releasing the nucleic acid components to directly enable a RCA reaction by cleavage and/or unfolding of the probe, wherein at least one of the released components of the cleaved and/or unfolded probe functions as the primer for the RCA reaction;
(c) extending the primer using the RCA template to produce a RCA product;
   and
(d) detecting said RCA product.

In some embodiments the probe interacts with an at least partially single stranded nucleic acid molecule, wherein the at least partially single stranded nucleic acid molecule is:
(i) the analyte;
(ii) directly or indirectly attached to the analyte; or
(iii) a marker for the analyte in the sample.

It will be evident from the description of the probe of the invention that the method may require additional steps depending on the features of the probe, e.g. a ligation step to circularise the RCA template or a wash step to remove probes and/or RCA products that are not attached to the analyte, e.g. nucleic acid molecule, to be detected.

As mentioned above, in a particular embodiment of the invention, the target analyte is a RCA product, i.e. the nucleic acid product of a RCA reaction. Accordingly in one preferred aspect, the present invention provides a method for performing a localised RCA reaction comprising at least two rounds of RCA, wherein the product of a second RCA reaction is attached, and hence localised, to a product of a first RCA reaction, said method comprising:
(a) providing a first RCA product;
(b) directly or indirectly hybridising to said first RCA product a probe, said probe being a nucleic acid construct comprising:
   (i) one or more domains comprising a region of complementarity to a first RCA product or to an intermediate molecule bound, (e.g. hybridized) directly or indirectly, to the first RCA product;
   (ii) one or more domains together comprising or capable of providing a circular or circularisable RCA template;
   (iii) a domain comprising or capable of providing a primer for RCA of said RCA template, wherein said domain hybridizes to a region of said circular or circularisable RCA template; and, when said RCA template is circularisable,
   (iv) one or more domains comprising or capable of providing a ligation template that templates the ligation (or circularisation) of the circularisable RCA template,
      wherein at least part of the probe must be cleaved and/or unfolded to release said primer to enable said RCA;
(c) cleaving and/or unfolding the probe to release the primer, and optionally the RCA template, and the ligation template if present;
(d) where said RCA template is circularisable, performing a ligation step to circularise the RCA template;
(e) performing a second RCA reaction using said RCA primer and RCA template of (b) to form a second RCA product, wherein in said reaction:
   (i) said probe and any unfolded or cleaved part thereof (e.g. any of the nucleic acid components comprised in or released from the probe) are not able to prime extension using said first RCA product as template or any such extension is limited to avoid displacement of any probe hybridised to the first RCA product;
   (ii) the direct or indirect hybridisation of the RCA primer of (b) to the first RCA product is maintained and, by virtue of said hybridisation, the second RCA product is attached to the first RCA product.

The present invention is predicated on surprising determination that it is possible to provide the nucleic acid components that are necessary and sufficient to perform a RCA reaction in a single nucleic acid molecule. Moreover, the RCA reaction can be controlled by requiring that at least one of the RCA components is released by a cleavage reaction and/or by unfolding the probe after the probe is contacted with target analyte, e.g. nucleic acid molecule.

Thus, the requirement that the probe of the invention provides nucleic acid components to directly enable a RCA reaction means that the probe must be capable of providing (after cleavage and/or unfolding) a circular or circularisable nucleic acid molecule (known herein as a RCA template) and a primer (a RCA primer). A third nucleic acid component, a ligation template, may be provided when the RCA template is provided as a circularisable nucleic acid molecule, as it is essential that the circularisable nucleic acid molecule is circularised before the RCA reaction can proceed. In some embodiments of the invention, the primer may also act as the ligation template. In particularly preferred embodiments of the invention, the target nucleic acid molecule does not function as the ligation template for the RCA template. However, the target nucleic acid molecule may function as the ligation template for the probe.

RCA templates, i.e. circular or circularisable nucleic acid molecules, e.g. oligonucleotides, are well known in the art. A RCA template typically may comprise about 20-1000 nucleotides, e.g. 26-1000, 30-900, 40-800, 50-700, 60-600, 70-500, 80-400, 90-300 or 100-200 nucleotides, such as at least 20, 25, 26, 27, 28, 29, 30, 35, 40, 50, 60, 70, 80, 90, 100, 120, 150, 200 or 250 nucleotides.

Circularisable nucleic acid molecules (commonly known as padlock probes and variants thereof) typically are linear nucleic acid molecules that comprise free ends which may hybridise to one or more nucleic acid domains (common template(s)) which act to template the ligation of the free ends to each other to generate a circular oligonucleotide. Such a ligation may be direct, i.e. where the free ends hybridise to the ligation template directly adjacent to each other. Alternatively, the ligation may be indirect, i.e. where the free ends hybridise to the ligation template with a space in between which is filled by a "gap" oligonucleotide such that each free end is ligated to one end of the gap oligonucleotide. In some embodiments, the space in between the free ends may be "filled-in" by extending the free 3' end, e.g. in a polymerase reaction, using the ligation template as an extension template. Once the free 3' end has been extended to be adjacent to the free 5' end, the two ends may be joined by a ligation reaction.

In embodiments in which the RCA template is provided as a preformed circle or circularisable strand of the probe (where the probe is a circle RCA probe) and cleavage and/or unfolding of the probe is not required to generate the RCA template, the RCA template is preferably provided as a preformed circle (a circular oligonucleotide). However, this is not an essential feature of the probe and the RCA template could be provided as a circularisable oligonucleotide, wherein the domain of the probe to which the RCA template is hybridised may function as the ligation template and/or extension template (based on the gap-fill embodiment described above).

In embodiments in which the RCA template is released by cleavage and optionally unfolding of the domains of the probe (where the probe is a hairpin RCA probe or linear RCA probe), the RCA template will be in the form of a circularisable nucleic acid molecule, i.e. one of the nucleic acid domains of the cleaved probe may release a 5' and/or 3' end, allowing the ligation of the ends to form a circular molecule (the RCA template). The release of the ligatable 5' and 3' ends of the domain can thus be viewed as the generation of a RCA template for circularisation by ligation. In a particularly preferred embodiment of the invention, the ligatable 5' and 3' ends of the domain hybridise to the ligation template directly adjacent to each other to obviate the need to provide a separate "gap" oligonucleotide (a gap oligonucleotide cannot be provided by the hairpin RCA probe of the invention). Nevertheless, in some embodiments the ligatable 5' and 3' ends of the domain may hybridise to the ligation template with a space in between, wherein the space is "filled in" by extension of the 3' end using the ligation template as an extension template, followed by ligation of the domain to form the RCA template.

The RCA template may comprise a reporter domain, which is a sequence that can be used to detect and/or identify the RCA product, i.e. the primer extension product templated by the RCA template. This is particularly advantageous in multiplex embodiments of the invention, i.e. where more than one analyte, e.g. nucleic acid analyte, is detected in a single assay. The RCA template provided by each probe (each probe is specific for a target analyte), may comprise a unique "marker" or identification sequence (e.g. a bar-code sequence, such as a site comprising the sequence of a specific detection probe, i.e. the RCA product is complementary to the RCA template and as such detection probes that hybridize to the RCA product will comprise a sequence that is identical to part of the RCA template) to allow the separate detection and/or quantification of each analyte in the sample. Thus, in multiplex assays each probe may comprise a different reporter domain and the detection of the interaction of the probe and the target analyte, i.e. the detection of each analyte, may be detected in parallel (i.e. at the same time), e.g. using oligonucleotides tagged with distinct fluorophores that may hybridise to the complement of the reporter domain. Alternatively, each marker (and therefore each analyte) may be detected using sequential visualisation reactions, wherein each reaction is separated by, e.g. stripping or bleaching steps. Methods of sequential visualisation reactions suitable for using the methods of the invention are known in the art, e.g. Göransson et al., 2009 (A single molecule array for digital targeted molecular analyses. Nucleic Acids Res. 2009 Jan;37(1):e7), Wählby et al., 2002 (Sequential immunofluorescence staining and image analysis for detection of large numbers of antigens in individual cell nuclei. Cytometry, 47(1):32-41, 2002). In some representative embodiments of the invention, multiple analytes may be detected in parallel. In other representative embodiments of the invention, multiple analytes may be detected sequentially. Combinatorial methods of labelling, e.g. ratio labelling, using different combinations and/or ratios of different labels are known in the art and may be used to increase the number of different molecules, and hence different analytes which may detected at one time, or in the same reaction. For example, combinations using different coloured and/or fluorescent labels and/or different ratios of different coloured and/or fluorescent labels may be used.

A primer or primer domain (a RCA primer) is a part or region of the probe that comprises a 3' end that can be extended, e.g. in a polymerization reaction, using the RCA template as the template for extension. Accordingly, the primer or primer domain comprises a region of complementarity (defined further below) to a part of the RCA template, which forms a duplex that is sufficiently stable under the conditions of the assay to facilitate RCA template dependent extension of the primer. The primer domain may also function as a ligation template, defined below. The primer domain of a probe will generally be at least 5 bp in length, typically at least 6, 8 or 10 bp in length, usually at least 15 bp in length and more usually at least 16 bp in length and may be as long as 30 bp in length or longer, where the length of the primer will generally range from 5 to 50 bp in length, e.g. 6, 8 or 10 to 50 bp, usually from about 10 to 35 bp in length. In some embodiments, the primer or primer domain may comprise a cleavage recognition site.

A ligation template or ligation template domain may be present in the probe, but is not essential in all embodiments, e.g. where the RCA template is provided as a preformed circle oligonucleotide. When present, the domain is a part or region of the probe to which the 5' and 3' ends of a circularisable RCA template may bind to template the direct or indirect intramolecular ligation of the RCA template to form a circular oligonucleotide. As mentioned above, in some embodiments, the ligation template may also function as an extension template. Accordingly, the ligation template or ligation template domain comprises a region of complementarity (defined further below) to parts (the 5' and 3' ends) of the RCA template, which forms a duplex that is sufficiently stable under the conditions of the assay to facilitate ligation template dependent ligation of the circularisable RCA template. The ligation template domain of a probe will generally be at least 2 bp in length, typically at least 5 bp in length and usually at least 10 bp in length, such as at least 15, 16, 17, 18, 19 or 20 bp in length and may be as long as 30 bp in length or longer, where the length of the ligation template will generally range from 2 to 50 bp in length, usually from about 5 to 35 bp in length or about 10 to 20 bp in length.

Thus the probe of the invention may be viewed as comprising various domains, which each function to interact with the target nucleic acid molecule or provide at least one of the nucleic acid components for RCA by releasing a domain upon cleavage of a cleavage site and/or unfolding of the probe.

Thus, the probe comprises at least one domain that is capable of binding to the target analyte, i.e. a target binding domain. In many embodiments the target binding domain comprises a region of complementarity to the target nucleic acid molecule, a so-called target complementary domain. In other embodiments, the target binding domain may require the probe to be conjugated to another molecule, e.g. a protein such as an antibody, comprising an analyte-binding domain that is capable of binding to the analyte, e.g. a non-nucleic acid analyte. In some embodiments, the target binding domain is capable of binding to a molecule that is bound directly or indirectly to a target analyte, e.g. the target complementary domain is complementary to a nucleic acid molecule bound directly or indirectly to the target nucleic acid molecule, a so-called "intermediate molecule" or "intermediary binding partner". For simplicity, the invention is defined with respect to direct interactions between the probe and a target analyte, particularly wherein the analyte is a nucleic acid molecule. However, it will be apparent to a person of skill in the art that the target molecule (i.e. analyte) may not be a nucleic acid molecule and the interaction between the probe and the target analyte may be direct or indirect. Accordingly, in some embodiments, the nucleic acid molecule with which the probe interacts may be viewed as the target nucleic acid molecule even though the objective of a method using the probe of the invention may be the detection of a nucleic acid molecule or other analyte with which the probe does not interact directly.

An analyte-binding domain may be any binding partner for the target analyte, and it may be a direct or indirect binding partner therefor. Thus it may bind to the target analyte directly or indirectly via an intermediary molecule or binding partner (as defined below) which binds to the target analyte, the analyte-binding domain binding to said intermediary molecule (binding partner). Particularly, the analyte-binding domain or the intermediary binding partner is a specific binding partner for the analyte.

An analyte binding domain may be selected to have a high binding affinity for a target analyte. By high binding affinity is meant a binding affinity of at least about 10⁻⁴ M, usually at least about 10⁻⁶ M or higher, e.g., 10⁻⁹ M or higher. The analyte binding domain may be any of a variety of different types of molecules, so long as it exhibits the requisite binding affinity for the target analyte when present as part of the probe. In other embodiments, the analyte binding domain may be a ligand that has medium or even low affinity for its target analyte, e.g. less than about 10⁻⁴ M.

Hence, the analyte binding domain of the probe may be any molecule capable of selectively binding to a target molecule. For example, the binding domain may be selected from a protein, such as a monoclonal or polyclonal antibody, lectin, soluble cell surface receptor, combinatorially derived protein from phage display or ribosome display, peptide, carbohydrate, nucleic acid, such as an aptamer or a nucleic acid molecule comprising the complementary sequence for a target nucleic acid, or combinations thereof. In a preferred embodiment of the invention, the analyte binding domain is a protein, preferably an antibody or derivative or fragment thereof. A region of complementarity to the target nucleic acid molecule refers to a portion of the probe that is capable of forming an intermolecular duplex with at least a region of the target nucleic acid molecule. In some embodiments the region of complementarity to the target nucleic acid molecule will be sufficient to form a stable duplex in the assay conditions in which the probe finds utility, such that the probe (or domains thereof) and target nucleic acid molecule will not dissociate even after cleavage and/or unfolding of the probe. In other embodiments the region of complementarity to the target nucleic acid molecule may be designed such that it is capable of forming a stable duplex with the target nucleic acid molecule only when at least one other target complementary domain of the probe forms a duplex with the target nucleic acid molecule (in the assay conditions in which the probe finds utility). Thus, it may be necessary to stabilise the duplex formed between the probe and the target nucleic acid molecule (e.g. by an intramolecular ligation of the domains of the probe that interact with the probe binding domains on the target nucleic acid molecule) to prevent the probe (or domains thereof) and target nucleic acid molecule from dissociating after cleavage and/or unfolding of the probe.

In embodiments where the probe comprises a hairpin structure that must be unfolded and/or cleaved to release a RCA nucleic acid component (e.g. a hairpin RCA probe), the hairpin structure may comprise any suitable number of nucleotide residues such that the hairpin can be unfolded. Preferably the hairpin structure will unfold only under suitable conditions, e.g. on the addition of a cleavage agent. It will be apparent that the structure of the hairpin will depend on the method used to promote its unfolding. In a representative example the portion of the nucleic domain forming the hairpin structure, i.e. the portion that provides the duplex and the loop of the hairpin structure, will be between from about 14 to about 1000 nucleotides in length, where in certain embodiments they may range from about 14 to about 500 nucleotides in length including from about 14 to about 250 nucleotides in length, e.g., from about 14 to about 160 nucleotides in length, such as from about 14 to about 150 nucleotides in length, from about 14 to about 130 nucleotides in length, from about 14 to about 110 nucleotides in length, from about 14 to about 90 nucleotides in length, from about 14 to about 80 nucleotides in length, from about 14 to about 75 nucleotides in length, from about 14 to about 70 nucleotides in length, from about 14 to about 60 nucleotides in length and any length between the stated ranges. Thus, the duplex part of the at least one hairpin structure (i.e. the stem of the stem loop structure) may be at least 3 base pairs in length, preferably at least 4, 5, 6, 7, 8, 9, 10, 11, 12, 13, 14, 15, 20, 25, 30, 40 or 50 base pairs in length. In other embodiments, the duplex part of the at least one hairpin structure of the probe may be at least 100, 200, 300 or 400 base pairs in length.

The single-stranded loop of the at least one hairpin structure preferably comprises at least 8 nucleotides, preferably at least 9, 10, 11, 12, 13, 14, 15, 20, 25, 30, 40 or 50 nucleotides. In other embodiments, the single-stranded loop of the at least one hairpin structure may be at least 100, 200, 300 or 400 nucleotides in length.

In preferred aspects of the invention the hairpin structure of the probes comprises at least one uracil residue, preferably at least 2, 3, 4, 5, 6, 7, 8, 9 or 10 uracil residues.

"Complementary" nucleotide sequences will combine with specificity to form a stable duplex under appropriate hybridization conditions. For instance, two sequences are complementary when a section of a first sequence can bind to a section of a second sequence in an anti-parallel sense wherein the 3'-end of each sequence binds to the 5'-end of the other sequence and each A, T(U), G and C of one sequence is then aligned with a T(U), A, C and G, respectively, of the other sequence. RNA sequences can also include complementary G=U or U=G base pairs. Thus, two sequences need not have perfect homology to be "complementary" under the invention. Usually two sequences are sufficiently complementary when at least about 85% (preferably at least about 90%, and most preferably at least about 95%) of the nucleotides share base pair organization over a defined length of the molecule.

In some of the embodiments described above it may be useful for one domain of the probe to share complementarity with more than one other nucleic acid molecule. It may be particularly advantageous for the domain to have a different complementarity for each nucleic acid molecule with which it interacts, i.e. to allow one interaction to occur preferentially over a different interaction. For instance, in some embodiments the target complementary domain may be complementary to the target nucleic acid molecule and to a second or further strand of the probe e.g. a protective strand (in this context the protective strand may be seen as a "blocking" strand), wherein the interaction between the probe and the target nucleic acid molecule is sufficient to displace the "blocking" (protective) strand (i.e. unfold the probe). In another exemplary embodiment, the primer domain may be complementary to the "blocking" strand (here an invasion strand) and the RCA template, and the invasion strand may be complementary to the target nucleic acid molecule. The interaction between the probe and the target nucleic acid molecule is sufficient to displace the invasion strand from its interaction with the primer domain, wherein the invasion strand binds to the target nucleic acid molecule and the primer strand binds to the RCA template, i.e. the probe is unfolded to release the primer for RCA. Thus, the sequences of complementary domains of probe may share at least about 85%, e.g. 90, 91, 92, 93, 94, 95, 96, 97, 98, 99 or 100% sequence identity with one or more nucleic acid molecules, e.g. domains of the probe or target nucleic acid molecules.

The regions of complementarity (i.e. hybridisation regions) between any domains of the probe and/or the target nucleic acid molecule may have a length in the range of 4-100 bp. In some embodiments it may be useful to use relatively short regions of complementarity e.g. 6-20, 6-18, 7-15 or 8-12 bp. However, other longer regions of complementarity may be useful, particularly for interactions between the probe and the target nucleic acid molecule, e.g. at least 20, 25, 35, 40, 50, 60, 70, 80, 90 or 100 bp such as 10-100, 20-90, 30-70 or 40-60 bp.

In many embodiments of invention the probe comprises at least one domain comprising a cleavage recognition site, e.g. a cleavage or cleavable domain. A cleavage recognition site is a sequence that is recognised by a cleavage enzyme, i.e. the cleavage enzyme is capable of interacting specifically with the cleavage recognition site, wherein said interaction results in the cleavage of a nucleic acid molecule. In some embodiments the cleavage enzyme may cleave the nucleic acid molecule at the cleavage recognition site, i.e. the cleavage recognition site may be a cleavage or cleavable domain. In other embodiments the cleavage enzyme may cleave at a position directly or indirectly adjacent to the cleavage recognition site, i.e. the cleavage or cleavable domain may form a domain that is distinct from the cleavage recognition site. Hence, the probe of the invention may comprise cleavage recognition sites and cleavable domains as separate features. In other embodiments, all of the cleavage recognition sites may be cleavable sites/domains.

In some embodiments, the probe comprises more than one cleavable domain, wherein cleavage of the cleavable domain releases a primer domain, RCA template and ligation template domain. In some embodiments, cleavage results in the unfolding of hairpin structures within the probe, which releases the RCA nucleic acid components. Thus, cleavage of a cleavable domain may result in the separation of the domains of the probe into distinct nucleic acid molecules (i.e. hairpin RCA probes), wherein the domains are held in proximity to each other by their interaction with the target nucleic acid molecule. If the probe is cleaved into separate nucleic acid domains that are not attached to the target nucleic acid molecule, the interaction between the domains (the regions of complementarity between the domains) is not sufficient to hold the domains in proximity to enable a RCA reaction to proceed.

"Cleavage" is defined broadly herein to include any means of breaking a nucleotide chain (i.e. a nucleotide sequence). Cleavage may thus involve breaking a covalent bond. This may involve cleavage of nucleotide chain (i.e. strand cleavage or strand scission), for example by cleavage of a phosphodiester bond.

In some embodiments, cleavage of the cleavage site of the probe concerns breaking at least one covalent bond linking adjacent nucleotide residues of the probe nucleic acid molecule, e.g. hydrolysis of the phosphodiester bond. Cleavage preferably involves the hydrolysis of one or more phosphodiester bonds, particularly wherein the cleavage site forms part of a hairpin structure. Thus, in some embodiments the cleavage recognition site (or cleavable domain) is in a hairpin structure.

In its simplest form, the cleavage recognition site may be a part of the probe that is available for cleavage (and/or susceptible to cleavage), preferably when the probe is bound to the target nucleic acid molecule. For instance, the cleavage recognition site may be a region at the end of the probe that is single stranded, e.g. a single stranded 3' end. In other words, in some embodiments the single stranded 3' end of a probe may be viewed as a cleavage domain. An exonuclease enzyme that is capable of degrading only single stranded nucleic acid may be used to degrade the single stranded end of the probe, wherein degradation will stop at a region of the probe that is double stranded and/or comprises a blocking domain, e.g. an exonuclease block. For instance, Figure 1 depicts an embodiment in which degradation of the 3' end of the probe releases the primer domain for RCA template directed extension. In a preferred embodiment, the unfolding and/or cleavage of the circle RCA probe releases a single stranded 3' end that may be degraded by an exonuclease enzyme.

In particular embodiments, the probe comprises regions of double stranded nucleic acid, which may be in the form of hairpin structures, that may comprise an endonuclease recognition sequence, i.e. the cleavage recognition site may be an endonuclease recognition site. In an exemplary embodiment, e.g. where the probe comprises an endonuclease recognition site, the endonuclease will cleave only a single strand of the duplex portion of the probe, e.g. one strand of the hairpin structure, thereby releasing a RCA nucleic acid component or a domain that may provide a RCA nucleic acid component.

The probe may comprise an endonuclease recognition sequence. For example, a nucleic acid strand of the probe may be "cleavage oligonucleotide" "restriction oligonucleotide", which hybridizes to another nucleic acid strand of the probe to provide an endonuclease recognition site. In some embodiments, a cleavage or restriction oligonucleotide may be hybridized to a single-stranded part or region of the probe, e.g. a single-stranded loop of a hairpin structure, to comprise a duplex within the probe. However, in embodiments where the cleavage oligonucleotide is provided separately it does not provide a RCA nucleic acid component, e.g. a ligation template.

In particular embodiments of the methods of the invention, e.g. linear and/or hairpin RCA probes that comprise at least one cleavage strand, it may be advantageous to include, i.e. add, an excess of cleavage strands in the reaction mix. This may help to ensure that all of the probes that bind to a target nucleic acid molecule can release the nucleic acid components sufficient to initiate a RCA reaction. For example, in some reaction conditions a proportion of cleavage strands may be dissociated from the probes. Thus, providing an excess of cleavage strands in the reaction mix, i.e. cleavage oligonucleotides, may be sufficient to replace and cleavage strands that have been displaced from the probe, i.e. to reassemble the probe *in situ.*

In some embodiments, the endonuclease may be a restriction endonuclease (a restriction enzyme), i.e. the cleavage recognition site may be a restriction endonuclease recognition site. Any suitable restriction endonuclease may be used to cleave the probe, i.e. the probe may comprise an suitable restriction endonuclease recognition site. As described above, in particular embodiments it may be useful to utilise a type II restriction endonuclease recognition sequence, and optionally a cleavage domain. Some type II restriction endonucleases, e.g. type IIS enzymes, may find particular utility in the methods of the invention. Type II restriction endonucleases either cleave within a specific cleavage recognition site or at an adjacent site (a cleavage domain), wherein the adjacent site may be a specific distance from the cleavage recognition site (e.g. a type IIS enzyme) and/or may comprise an additional cleavage recognition site (e.g. a type IIE enzyme).

In some embodiments the cleavage recognition site is achieved by providing a probe, e.g. a hairpin structure in the probe, that comprises one or more Uracil residues. The domain comprising the uracil residues, e.g. a hairpin structure, can be cleaved by treatment with a uracil-DNA glycosylase (UNG) enzyme in combination with an endonuclease enzyme capable of recognising apurinic/apyrimidinic (AP) sites of dsDNA, e.g. endonuclease IV, wherein cleavage releases one or more RCA nucleic acid components. Accordingly, the cleavage recognition site, e.g. the cleavable domain, may comprise one or more uracil residue, e.g. 2, 3, 4, 5, 6, 7, 8, 9, 10 or more uracil residues.

In some embodiments the cleavable domain, e.g. hairpin structure, may be cleaved, and thereby release one or more RCA nucleic acid components, using a nickase enzyme, which cleaves only one strand in the duplex of the cleavable domain, e.g. hairpin structure. Thus, the cleavage recognition site may be a site for a nickase enzyme. Nickases are endonucleases which cleave only a single strand of a DNA duplex. As described above, a cleavage recognition site may be provided in a single-stranded region of the probe, e.g. a loop of a hairpin structure, e.g. by annealing (hybridising) an oligonucleotide to said single-stranded region, e.g. loop, or when a target complementary domain comprising a cleavage recognition site binds to the target nucleic acid molecule. Alternatively viewed, the cleavage recognition site may become functional, i.e. may be in a form that is recognised and cleaved by a endonuclease (or enables the endonuclease to cleave the probe at a position adjacent to the cleavage recognition site, i.e. in a cleavable domain), when a cleavage oligonucleotide or target nucleic acid molecule interacts with the cleavage recognition site. In other embodiments, a blocking oligonucleotide may bind to the cleavage recognition site to prevent cleavage from occurring.

Some nickases introduce single-stranded nicks only at particular sites on a DNA molecule, by binding to and recognizing a particular nucleotide recognition sequence, i.e. a cleavage recognition sequence. Some nickases introduce single-stranded nicks at mis-match positions in a duplex. Hence, in some embodiments, the cleavage recognition site may be formed when the target complementary domain binds to the target nucleic acid molecule with a mis-match, i.e. the target complementary domain may not be 100% complementary to the target binding region in the target nucleic acid molecule, as defined above. A number of naturally-occurring nickases have been discovered, of which at present the sequence recognition properties have been determined for at least four. Nickases are described in U.S. Patent No. 6,867,028, and any suitable nickase recognition site may be used in the probes and methods of the invention.

In some preferred embodiments of the methods of the invention that utilise a nickase enzyme, the nickase enzyme is removed from the assay or inactivated following cleavage, and optionally unfolding, of the probe to prevent unwanted cleavage of ligation products.

In further embodiments of the invention an exonuclease enzyme may be used to degrade a portion of one strand of the probe, e.g. a single-stranded domain at the 3' end of the probe or a hairpin structure, thereby releasing a RCA nucleic acid component provided by the probe. Hence, the cleavage recognition site, or more particularly the cleavage domain, may be a single stranded part of the probe or a hairpin structure that is susceptible to exonuclease cleavage, i.e. unblocked. The exonuclease enzyme may have 5' or 3' exonuclease activity depending on the orientation of the hairpin structure or the design of the probe. In some embodiments, the exonuclease activity may be provided by a polymerase enzyme.

In some embodiments, one or more of the RCA nucleic acid components may be released by unfolding the probe and this may be achieved in a number of ways. In particular embodiments, one RCA nucleic acid components may be unfolded by cleavage, i.e. one or more RCA nucleic acid components may be released by cleavage and unfolding of the probe. In some embodiments, only cleavage of the probe is required to release one or more RCA nucleic acid components, wherein said cleavage may comprise cleaving one or more cleavage domains, e.g. 2, 3, 4, 5 or more cleavage domains. In some embodiments, e.g. the hairpin RCA probes, cleavage occurs in a hairpin structure of the nucleic acid domain (i.e. the cleavage domain is located in, or forms part of, a hairpin structure). As discussed above, cleavage is preferably enzymatic cleavage.

As described above, some of the probes of the invention comprise at least one hairpin structure. A hairpin structure may also be known as a hairpin-loop or a stem-loop and these terms are used interchangeably herein. A hairpin is an intramolecular base-pairing pattern that can occur in a single-stranded DNA or RNA molecule. A hairpin occurs when two regions of the same strand, usually complementary in nucleotide sequence when read in opposite directions, base-pair to form a double helix (a duplex) that ends in an unpaired, i.e. single-stranded, loop. The resulting structure can be described as lollipop-shaped.

In some aspects of the invention, a hairpin structure does not form the end of the probe, i.e. the duplex of at least one hairpin is flanked by a single-stranded region at the 5' and/or 3' ends of duplex. Thus, in some embodiments, a hairpin may be at one end of the probe, i.e. one end of the duplex (the 3' or 5' end) forms the end of the probe.

Unfolding of the probe may also be achieved by disrupting at least part of the double stranded element (portion or domain) of the probe, such as a hairpin structure, e.g. the hairpin structure in a circle RCA probe. This may be achieved by altering the conditions of the sample such that the hairpin structure is no longer a thermodynamically favourable structure, e.g. by altering the temperature or salt concentrations of the solution. In some embodiments, unfolding is achieved by contacting the probe with the target nucleic acid molecule, i.e. target dependent unfolding, wherein the interaction (the duplex formed) between the probe and the target is more stable than the intramolecular duplex formed by the domains of the probe, i.e. unfolding may include displacing a nucleic acid strand. Similarly, the hairpin structure may be destabilised by modification of one or more of the nucleotide bases in the duplex to disrupt the hydrogen bonds (so-called Watson-Crick base pairing) which anneal the two strands. For example, cleavage of the base from the nucleotide may be sufficient to disrupt the duplex enough to "unfold" the hairpin.

Thus cleavage and/or unfolding the probe results in the release of at least one of the nucleic acid components for an RCA reaction. As described above, prior to cleavage and/or unfolding of the probe at least one of the RCA nucleic acid components, i.e. the primer and/or circular or circularisable RCA template, is unable to participate in, or initiate, the RCA reaction. For instance, prior to unfolding and/or cleavage, at least one of the RCA nucleic acid components is inaccessible (e.g. unavailable or blocked) for a rolling circle amplification reaction, i.e. not in a form that will allow RCA. For example, the primer domain may not have a free 3' extendable end, e.g. the probe may comprise additional nucleotides downstream of, 3' to, the 3' end of the primer domain that do not have complementarity to the RCA template. Additionally or alternatively, the RCA template may not be circularised. Hence, release of one or more RCA nucleic acid components means that the probe is unfolded and/or cleaved to make said one or more components available, i.e. accessible, for a RCA reaction. In other words, the probe gives rise to, generates or allows to be generated one or more RCA nucleic acid components. Thus, release may involve unblocking said one or more components so that they are available to initiate, or participate in, a RCA reaction. Release may be converting or modifying said probe to generate or produce one or more components that will enable a RCA reaction to commence, i.e. in the presence of a suitable polymerase enzyme.

The domains of the RCA reporter probe may be made up of ribonucleotides and/or deoxyribonucleotides as well as synthetic nucleotide residues that are capable of participating in Watson-Crick type or analogous base pair interactions. Thus, the nucleic acid domains may be DNA and/or RNA or any modification thereof e.g. PNA or other derivatives containing non-nucleotide backbones. In some embodiments, the probe domain may comprise an exonuclease block, such that it cannot be used as a primer in a target templated nucleic acid extension reaction, i.e. cannot be recognised as a primer by a polymerase enzyme and/or cannot be degraded to produce a nucleic acid molecule capable of priming extension of the target nucleic acid molecule.

The possible lengths of the domains of the RCA probe are defined above and it will be apparent that when the probe comprises multiple nucleic acid strands, each strand may be of a different length, which may vary widely. For instance, a blocking strand and/or cleavage strand typically may comprise between 4-100 nucleotides, e.g. 5-90, 6-80, 7-70, 8-60, 9-50, 10-40 nucleotides, such as at least 12, 15, 18, 20, 25, 30 or 35 nucleotides, whereas a RCA template typically may comprise 20-1000 nucleotides, e.g. 26-1000, 30-900, 40-800, 50-700, 60-600, 70-500, 80-400, 90-300, 100-200 nucleotides, such as at least 20, 25, 26, 27, 28, 29, 30, 35, 40, 45, 50, 60, 70, 80, 90, 100, 120, 150, 200 or 250 nucleotides. Typically, the length of the primer strand of a circle RCA probe is similar to the range of lengths that are typical for an RCA template. The length of a hairpin RCA probe (i.e. the strand that provides the RCA template) typically may comprise 40-1500 nucleotides, e.g. 50-1400, 60-1300, 70-1200, 80-1100, 90-1000 nucleotides, such as at least 40, 45, 50, 55, 60, 65, 70, 75, 80, 85, 90, 95, 100, 125, 150, 175, 200, 250 or 300 nucleotides. Where the RCA probe comprises more than one nucleic acid strand, the length of the probe may be defined according to the longest strand, e.g. the primer strand, circle strand, RCA template strand.

As mentioned above, when the probe interacts directly with a nucleic acid moelcule, the target nucleic acid molecule is at least partially single stranded. The target nucleic acid molecule may be the analyte to be detected in a sample, i.e. a nucleic acid analyte, or may serve as a proxy or marker for a different analyte, as described further below. The target nucleic acid molecule may comprise a single probe binding site, i.e. a single domain or region that is complementary to the target complementary domain of the probe. In embodiments where the probe comprises more than one target complementary domain, the target nucleic acid molecule may be viewed as comprising a probe binding site for each complementary domain. Alternatively, each target complementary domain may be viewed as binding to a different portion (region or part) of the probe binding domain. In some embodiments, the target nucleic acid molecule (nucleic acid analyte) may comprise multiple probe binding sites. For instance, the target nucleic acid molecule may comprise a repetitive sequence, wherein each repeat comprises one or more probe binding sites, e.g. the target nucleic acid molecule may be a RCA product. In preferred embodiments, the target complementary domain(s) and the probe binding site(s) share at least 85% sequence identity, preferably 90%, e.g. 95, 96, 97, 98, 99 or 100%. In some embodiments, it may be useful to have at least one mis-match in the duplex to create a functional cleavage recognition site (a cleavable domain), e.g. which may be cleaved by a nickase enzyme. The length of the probe binding site may be the same as the length of the target complementary domain of the probe, as defined above, or may be longer, e.g. if multiple target complementary domains bind to the probe binding domain.

In some embodiments, the target nucleic acid molecule (e.g. nucleic acid analyte) is fully single stranded. The target nucleic acid molecule may be rendered partially or fully single stranded by any suitable means known in the art, e.g. enzymatic digestion/degradation, denaturation by heat, etc. The target nucleic acid molecule may be rendered partially or fully single stranded before, after or contemporaneously with the contact of the sample with the RCA probe. Preferably the target nucleic acid molecule is rendered partially or fully single stranded before the RCA probe is contacted with said sample.

As described above, the probe may be useful for the detection of any target analyte. In some embodiments, the one or more target binding domains may be formed by coupling the probe to one or more analyte-binding domain, as defined above, to allow the probe to interact directly with a non-nucleic acid target analyte (or a binding partner thereof). In other embodiments where the target analyte is not a nucleic acid molecule (i.e. a non-nucleic acid target analyte), the RCA probe may be viewed as an indirect binding partner for the target analyte, i.e. the probe may bind to an intermediate or intermediary molecule (i.e. a binding partner) that is bound directly or indirectly to the target analyte. Particularly, the intermediary binding partner is, or comprises, a nucleic acid molecule and is a specific binding partner for the analyte. A binding partner is any molecule or entity capable of binding to its target, e.g. target analyte, and a specific binding partner is one which is capable of binding specifically to its target (e.g. the target analyte), namely that the binding partner binds to the target (e.g. analyte) with greater affinity and/or specificity than to other components in the sample. Thus binding to the target analyte may be distinguished from non-target analytes; the specific binding partner either does not bind to non-target analytes or does so negligibly or non-detectably or any such non-specific binding, if it occurs, may be distinguished. The binding between the target analyte and its binding partner is typically non-covalent.

In some embodiments where the RCA probe binds to the analyte via an intermediary molecule, the probe may be pre-incubated with the intermediary molecule. For example, in embodiments where the probe binds to the nucleic acid domain of a proximity probe that binds target analyte directly, the probe may be pre-hybridized to the nucleic acid domain of the proximity probe. In this embodiment, the probe may be seen as forming part of the nucleic acid domain of the proximity probe. In a preferred embodiment, the probe is not pre-hybridized to the nucleic acid domain of a proximity probe.

The "analyte" may be any substance (e.g. molecule) or entity it is desired to detect by the method of the invention. The analyte is the "target" of the assay methods and uses of the invention. The analyte may accordingly be any biomolecule or chemical compound it may be desired to detect, for example a peptide or protein, or nucleic acid molecule or a small molecule, including organic and inorganic molecules. The analyte may be a cell or a microorganism, including a virus, or a fragment or product thereof. It will be seen therefore that the analyte can be any substance or entity for which a specific binding partner (e.g. an affinity binding partner) can be developed. All that is required is that the analyte is capable of binding a RCA probe or a binding partner comprising a nucleic acid molecule to which the RCA probe may bind, i.e. comprising a probe binding site. Analytes of particular interest may thus include nucleic acid molecules, such as DNA (e.g. genomic DNA, mitochondrial DNA, plastid DNA, viral DNA etc), RNA (e.g. mRNA, microRNA, rRNA, snRNA, viral RNA etc) and synthetic and/or modified nucleic acid molecules (e.g. including nucleic acid domains comprising or consisting of synthetic or modified nucleotides such as LNA, PNA, morpholino etc), proteinaceous molecules such as peptides, polypeptides, proteins or prions or any molecule which includes a protein or polypeptide component, etc., or fragments thereof. The analyte may be a single molecule or a complex that contains two or more molecular subunits, e.g. including but not limited to protein-DNA complexes, which may or may not be covalently bound to one another, and which may be the same or different. Thus in addition to cells or microrganisms, such a complex analyte may also be a protein complex or protein interaction. Such a complex or interaction may thus be a homo- or hetero-multimer. Aggregates of molecules, e.g. proteins may also be target analytes, for example aggregates of the same protein or different proteins. The analyte may also be a complex between proteins or peptides and nucleic acid molecules such as DNA or RNA, e.g. interactions between proteins and nucleic acids, e.g. regulatory factors, such as transcription factors, and DNA or RNA. Advantageously, where the analyte is a nucleic acid molecule, the nucleic acid may be detected *in situ*, i.e. without removing or extracting the nucleic acid from the cell. However, isolated and amplified nucleic acid molecules also represent appropriate target analytes.

All biological and clinical samples are included, e.g. any cell or tissue sample of an organism, or any body fluid or preparation derived therefrom, as well as samples such as cell cultures, cell preparations, cell lysates etc. Environmental samples, e.g. soil and water samples or food samples are also included. The samples may be freshly prepared or they may be prior-treated in any convenient way e.g. for storage.

Representative samples thus include any material which may contain a biomolecule, or any other desired or target analyte, including for example foods and allied products, clinical and environmental samples. The sample may be a biological sample, which may contain any viral or cellular material, including all prokaryotic or eukaryotic cells, viruses, bacteriophages, mycoplasmas, protoplasts and organelles. Such biological material may thus comprise all types of mammalian and non-mammalian animal cells, plant cells, algae including blue-green algae, fungi, bacteria, protozoa etc. Representative samples thus include whole blood and blood-derived products such as plasma, serum and buffy coat, blood cells, urine, faeces, cerebrospinal fluid or any other body fluids (e.g. respiratory secretions, saliva, milk, etc), tissues, biopsies, cell cultures, cell suspensions, conditioned media or other samples of cell culture constituents, etc. The sample may be pre-treated in any convenient or desired way to prepare for use in the methods and uses of the invention, for example by cell lysis or purification, isolation of the analyte, etc.

Since the length of the probes, and particularly the length of the nucleic acid molecule between the target binding domains of the probe, can be constructed to span varying molecular distances, binding sites for the probe on the analyte or intermediary binding partner need not be on the same molecule. They may be on separate, but closely positioned, molecules. For example, each probe binding domain may be part of a nucleic acid domain of a proximity probe, wherein the proximity probes bind to the multiple binding domains of an organism, such as a bacterium or cell, or a virus, or of a protein complex or interaction, such that they can be targeted by the probes and methods of the present invention.

The detection of the target analyte depends upon the presence of an analyte in a sample and detecting the interaction between the probe and the analyte or intermediary binding partner, which is bound to the analyte. The interaction between the probe and the analyte may release one or more RCA nucleic acid components, e.g. via target dependent cleavage and/or unfolding of the probe. In some embodiments, the detection of the interaction between the probe and the target analyte may be via a proximity dependent ligation (i.e. ligation of the RCA template), wherein the ligation is dependent on the RCA nucleic acid components interacting, directly or indirectly, with the target. In other embodiments, the detection of the target analyte relies on the removal of probes or probe products that are not bound, directly or indirectly, to the target analyte, e.g. by washing.

Thus, in general terms the interaction between the probe and the target nucleic acid molecule may lead to the generation of a nucleic acid product, which may be detected in order to detect the analyte. Accordingly, in the methods of the invention, the detection step involves detecting an extension product, e.g. the extension of the primer domain of the probe templated by the RCA template provided by the probe, wherein by detecting the extension product the analyte may be detected.

Upon the addition of an appropriate polymerase (and if necessary other enzymes, e.g. cleavage and/or ligase enzymes), the presence of analyte in the sample may be detected by rolling circle amplification (RCA) of the RCA template (circularised oligonucleotide). The concatemeric RCA products, which typically can only be formed when the probes interacts with (binds to) the target nucleic acid molecule, provide the marker "signal" for detection of the analyte. Said signal may be detected by any appropriate means known in the art (see below for further examples) and as taught in US 7,320,860, e.g. by hybridisation of labelled probes to the reporter domain sequence, which is repeated throughout the concatemeric RCA products. As mentioned above, at least one of the RCA nucleic acid components of the probes must be released by cleavage and/or unfolding to enable a RCA product to be generated. Accordingly, in representative embodiments, reagents that are required to detect the interaction of the probe and the target nucleic acid molecule, e.g. amplify the RCA product, may be added to the reaction at the same time as the probe, thereby avoiding the need for the addition of specific detection reagents in a separate step. Minimising the number of steps in the assay may facilitate the reduction in the overall time needed to carry out the assay, i.e. increase the efficiency of the assay, and contribute to the enhanced signal to noise ratio, i.e. help to reduce non-specific background.

In some embodiments, the RCA template may be circularised by a ligation reaction (i.e. akin to a padlock probe as described above), i.e. on addition of an appropriate ligase. It is preferred that all of the nucleic acid components for the RCA are provided by the probe. However, ligation of the RCA template may encompass the use of a gap oligonucleotide. Hence, in embodiments that utilise a circle RCA probe, the probe may comprise a gap oligonucleotide or gap strand. A gap strand may be defined as an oligonucleotide that hybridizes to the primer strand of the RCA probe in between the 5' and 3' end of the circularisable RCA template. Each end of the RCA template is ligated to an end of the gap oligonucleotide to generate the circularised RCA template. It will be evident that a gap oligonucleotide may be provided separately to the RCA probes of the invention, i.e. in some embodiments a gap oligonucleotide is not part of the RCA probe. Hence, in the methods of the invention, a gap oligonucleotide may be added to the sample before, after, or contemporaneously with the RCA probe. In some embodiments, several different gap oligonucleotides may be added, wherein each type of gap oligonucleotide is added at a different concentration. Each type of gap oligonucleotide may comprise common sequences at the 5' and 3' ends that are complementary to the ligation template domain (in between the ends of the circularisable RCA template) and a different intervening sequence, which may act as a reporter domain as defined above. The resultant RCA products will comprise different reporter domain sequences depending on which gap oligonucleotide was ligated into the RCA template and can be detected separately. This may be utilised to extend the dynamic range of the assay methods described herein, as described in WO2012/049316.

The term "detecting" is used broadly herein to include any means of determining the presence of the analyte (i.e. if it is present or not) or any form of measurement of the analyte. Thus "detecting" may include determining, measuring, assessing or assaying the presence or absence or amount or location of analyte in any way. Quantitative and qualitative determinations, measurements or assessments are included, including semi-quantitative. Such determinations, measurements or assessments may be relative, for example when two or more different analytes in a sample are being detected, or absolute. As such, the term "quantifying" when used in the context of quantifying a target analyte(s) in a sample can refer to absolute or to relative quantification. Absolute quantification may be accomplished by inclusion of known concentration(s) of one or more control analytes and/or referencing the detected level of the target analyte with known control analytes (e.g., through generation of a standard curve). Alternatively, relative quantification can be accomplished by comparison of detected levels or amounts between two or more different target analytes to provide a relative quantification of each of the two or more different analytes, i.e., relative to each other.

The sequences of the various domains of the probes (i.e. primer domain, ligation template domain, RCA template, target complementary domains etc and the intervening (i.e. connecting) sequences) may be chosen or selected with respect to the sequence of each domain in the probe and the target nucleic acid molecule. Thus, the sequence of the various domains is not critical as long as the domains that are required to interact to enable the production of a RCA product can hybridise to each other under the appropriate conditions, e.g. in the presence of the target nucleic acid molecule. However, with the exception of the sequences required for the hairpin structures of the probes, the sequences of the domains should be chosen to avoid the occurrence of intramolecular hybridization (i.e. hybridization events between domains of the same strand). For example, the primer domain should not be capable of hybridising to the ligation template domain. Once the sequence of the domains is selected or identified, the probe may be synthesized using any convenient method.

The term "hybridisation" or "hybridises" as used herein refers to the formation of a duplex between nucleotide sequences which are sufficiently complementary to form duplexes via Watson-Crick base pairing. Two nucleotide sequences are "complementary" to one another when those molecules share base pair organization homology. Hence, a region of complementarity in a domain of a RCA probe refers to a portion of that domain that is capable of forming an intra- or intermolecular duplex, i.e. either a duplex within the same molecule (a hairpin structure) or a duplex with a different molecule or a different strand of the probe construct. These terms are also used to refer to base pair interactions which are analogous to Watson-Crick base pairing, including Hoogsteen base pairing which is a rarely observed variation of base pairing which also allows for a third strand to wind around a double-helix assembled in a Watson-Crick pattern to form a triplex.

The amount of probe that is added to a sample may be selected to provide a sufficiently low concentration of probe in the reaction mixture to minimise non-target specific interactions, i.e. to ensure that the probe will not randomly bind to non-target molecules in the sample to any great or substantial degree. As such, it is intended that only when the probe binds the target nucleic acid molecule are the RCA nucleic acid components released and allowed to generate a RCA product. In representative embodiments, the concentration of each probe in the reaction mixture following combination with the sample ranges from about 1 fM to 1µM, such as from about 1 pM to about 1 nM, including from about 1 pM to about 100 nM, e.g. 1, 2, 5, 10, 20, 50 nM.

A number of different probes may be added to a sample for a multiplex assay. Multiplex assays may involve the detection of hundreds, thousand or even tens of thousands of analytes in a sample. Accordingly, multiplex assays may comprise at least 2 distinct probes, i.e. probes capable of detecting different analytes. For instance, multiplex assays may utilised at least 3, 4, 5, 10, 20, 30, 40 or 50 probes, such as 100, 200, 500, 1000, 10000 or more probes.

Following combination of the sample and RCA probe(s), the reaction mixture may be incubated for a period of time sufficient for the probe(s) to bind target analyte, if present, in the sample. As described above, once the probe has bound to the analyte the probe is unfolded and/or cleaved to release at least one RCA nucleic acid component so as to allow the domains of the probe to interact, i.e. for the primer to interact with the RCA template so as to be able to be extended using the RCA template as a template for polymerisation. Where more than one type of RCA probe is used in the assay, each different type of RCA probe may be cleaved and/or unfolded separately, e.g. the first probe may be unfolded by cleavage and the second probe may be unfolded by target binding. In some representative embodiments, e.g. *in situ* assays or other assays in which the analyte is immobilised, wash steps may be included between the addition of probe and the detection of the RCA product, e.g. the analyte may be captured or immobilised on a substrate, which may be washed to remove unbound or non-specifically bound probe or RCA products that are not attached to the target nucleic acid molecule. In some embodiments, wash steps may be included between cleaving the probe and the detection of the analyte, e.g. to remove cleaved domains of the probe that are not bound to the target nucleic acid molecule. Alternatively, or additionally, a washing step may be included after the probe has been added to the sample and allowed to bind, but before the unfolding and/or cleavage step.

In representative embodiments, the probe and sample may be pre-incubated for a period of time ranging from 5 minutes to about 24 hours prior to the addition of the addtional probes. Preferably said pre-incubation is from about 20 minutes to 12 hours at a temperature ranging from 4 to about 50°C e.g. 10-40°C or 20-37°C. Conditions under which the reaction mixture is maintained should be optimized to promote specific binding of the probe to the target nucleic acid molecule, while suppressing unspecific interaction.

Following pre-incubation, if such a step is included, the probe is cleaved and/or unfolded and the product mixture may be incubated for a period of time ranging from about 5 minutes to about 48 hours, including from about 30 minutes to about 12 hours, at a temperature ranging from about 4 to about 105°C, including from about 4 to about 80 °C, such as about 10 to about 70°C, about 15 to about 60°C, typically about 20 to about 37 °C. Incubation at high temperatures, e.g. above about 40-50°C, may utilise thermophilic or hyperthermophilic enyzmes, e.g. ligases and/or polymerases. Conditions should allow for efficient and specific hybridization between the RCA nucleic acid components, as described above.

Following the combination of the sample with the probe, the gap oligonucleotide(s) may be added, if used, and allowed to hybridise. Alternatively or additionally, one or more gap oligonucleotides may be added with the probe. In some embodiments, the gap oligonucleotides may be prehybridized to the probe.

In general, any convenient protocol that is capable of detecting the presence of the RCA product may be employed. The detection protocol may or may not require a separation step.

In some embodiments, a ligation template domain stabilises the ends of a circularisable RCA template, which are ligated by contacting the reaction mixture with a nucleic acid ligating activity, e.g. provided by a suitable nucleic acid ligase, and maintaining the mixture under conditions sufficient for ligation of the nucleic acid domains to occur.

As is known in the art, ligases catalyze the formation of a phosphodiester bond between juxtaposed 3'-hydroxyl and 5'-phosphate termini of two immediately adjacent nucleic acids when they are annealed or hybridized to a third nucleic acid sequence to which they are complementary (i.e. a ligation template). Any convenient ligase may be employed, where representative ligases of interest include, but are not limited to: Temperature sensitive and thermostable ligases. Temperature sensitive ligases include, but are not limited to, bacteriophage T4 DNA ligase, bacteriophage T7 ligase, and *E. coli* ligase. Thermostable ligases include, but are not limited to, Taq ligase, Tth ligase, Ampligase® and Pfu ligase. Thermostable ligase may be obtained from thermophilic or hyperthermophilic organisms, including but not limited to, prokaryotic, eukaryotic, or archael organisms. Certain RNA ligases may also be employed in the methods of the invention.

A suitable ligase and any reagents that are necessary and/or desirable may be combined with the reaction mixture and maintained under conditions sufficient for ligation of the hybridized oligonucleotides to occur, e.g. ligation of the RCA template via the probe ligation template, ligation of one or more of the target complementary domains templated by the target nucleic acid molecule. Ligation reaction conditions are well known to those of skill in the art. During ligation, the reaction mixture in certain embodiments may be maintained at a temperature ranging from about 4°C to about 105°C, about 4 to about 80 °C, such as about 10 to about 70°C, about 15 to about 60°C, typically such as from about 20°C to about 37°C for a period of time ranging from about 5 seconds to about 16 hours, such as from about 1 minute to about 1 hour. In yet other embodiments, the reaction mixture may be maintained at a temperature ranging from about 35°C to about 45°C, such as from about 37°C to about 42°C, e.g., at or about 38°C, 39°C, 40°C or 41°C, for a period of time ranging from about 5 seconds to about 16 hours, such as from about 1 minute to about 1 hour, including from about 2 minutes to about 8 hours. In a representative embodiment, the ligation reaction mixture includes 50 mM Tris pH7.5, 10 mM MgCl₂, 10 mM DTT, 1 mM ATP, 25 mg/ml BSA, 0.25 units/ml RNase inhibitor, and T4 DNA ligase at 0.125 units/ml. In yet another representative embodiment, 2.125 mM magnesium ion, 0.2 units/ml RNase inhibitor; and 0.125 units/ml DNA ligase are employed.

It will be evident that the ligation conditions may depend on the ligase enzyme used in the methods of the invention. Hence, the above-described ligation conditions are merely a representative example and the parameters may be varied according to well known protocols. For example, a ligase that may be utilized in the methods of the invention, namely Ampligase®, may be used at temperatures of greater than 50°C. However, it will be further understood that the alteration of one parameter, e.g. temperature, may require the modification of other conditions to ensure that other steps of the assay are not inhibited or disrupted, e.g. binding of the probe to the target nucleic acid molecule. Such manipulation of RCA assay methods is routine in the art.

Following ligation (if ligation is required, e.g. if the probe comprises or provides a circularisable RCA template or the probe must be ligated to stabilize its interaction with the target nucleic acid molecule) the RCA template (which may be the ligation product), is detected as an indication of the presence, or as a measure of the amount and optionally the location, of analyte in the sample. The RCA template may be viewed as a single stranded circular nucleic acid molecule that is hybridized to at least one domain of the probe comprising a target complementary domain.

The next step of the method following ligation step (if required) is to generate the RCA product, i.e. to extend the RCA primer using the RCA template in a polymerisation reaction. Rolling-circle amplification (RCA) is well known in the art, being described in Dean et al., 2001 (Rapid Amplification of Plasmid and Phage DNA Using Phi29 DNA Polymerase and Multiply-Primed Rolling Circle Amplification, Genome Research, 11, pp. 1095-1099). In representative RCA reactions, the circularised RCA template is able to interact with the RCA primer, which is provided by the RCA reporter probe. The RCA primer is employed in a primer extension reaction, e.g., RCA is templated by the RCA primer to generate a single concatameric product. The RCA primer will be of sufficient length, as described above, to provide for hybridization to the RCA template under annealing conditions (described in greater detail below).

In addition to the above nucleic acid components, the reaction mixture produced in the subject methods typically includes a polymerase, e.g. phi29 DNA polymerase and other components required for a DNA polymerase reaction as described below. The desired polymerase activity may be provided by one or more distinct polymerase enzymes. In some embodiment the polymerase has exonuclease activity, e.g. 5' and/or 3' exonuclease activity.

In preparing the reaction mixture of this step of the subject methods, the various constituent components may be combined in any convenient order. For example, all of the various constituent components may be combined at the same time to produce the reaction mixture.

The amplified products of the RCA reaction may be detected using any convenient protocol, where the particular protocol employed may detect the RCA products non-specifically or specifically, as described in greater detail below. For instance, the RCA product may be detected directly, e.g. the concatemer may be cleaved to generate monomer which may be detect using gel electrophoresis or by hybridizing labelled detection nucleotides that hybridize to the reporter domain in the RCA product. Alternatively, the RCA product may be detected indirectly, e.g. the product may be amplified by PCR and the amplification products may be detected.

Representative non-specific detection protocols of interest include protocols that employ signal producing systems that selectively detect single or double stranded DNA products, e.g., via intercalation. Representative detectable molecules that find use in such embodiments include fluorescent nucleic acid stains, such as phenanthridinium dyes, including monomers or homo- or heterodimers thereof, that give an enhanced fluorescence when complexed with nucleic acids. Examples of phenanthridinium dyes include ethidium homodimer, ethidium bromide, propidium iodide, and other alkyl-substituted phenanthridinium dyes. In another embodiment of the invention, the nucleic acid stain is or incorporates an acridine dye, or a homo- or heterodimer thereof, such as acridine orange, acridine homodimer, ethidiumacridine heterodimer, or 9-amino-6-chloro-2-methoxyacridine. In yet another embodiment of the invention, the nucleic acid stain is an indole or imidazole dye, such as Hoechst 33258, Hoechst 33342, Hoechst 34580 (BIOPROBES 34, Molecular Probes, Inc. Eugene, Oreg., (May 2000)) DAPI (4',6-diamidino-2-phenylindole) or DIPI (4',6-(diimidazolin-2-yl)-2-phenylindole). Other permitted nucleic acid stains include, but are not limited to, 7-aminoactinomycin D, hydroxystilbamidine, LDS 751, selected psoralens (furocoumarins), styryl dyes, metal complexes such as ruthenium complexes, and transition metal complexes (incorporating Tb³⁺ and Eu³⁺, for example). In certain embodiments of the invention, the nucleic acid stain is a cyanine dye or a homo- or heterodimer of a cyanine dye that gives an enhanced fluorescence when associated with nucleic acids. Any of the dyes described in U.S. Pat. No. 4,883,867 to Lee (1989), U.S. Pat. No. 5,582,977 to Yue et al. (1996), U.S. Pat. No. 5,321,130 to Yue et al. (1994), and U.S. Pat. No. 5,410,030 to Yue et al. (1995) may be used, including nucleic acid stains commercially available under the trademarks TOTO, BOBO, POPO, YOYO, TO-PRO, BO-PRO, PO-PRO and YO-PRO from Molecular Probes, Inc., Eugene, Oreg. Any of the dyes described in U.S. Pat. No. 5,436,134 to Haugland et al. (1995), U.S. Pat. No. 5,658,751 to Yue et al. (1997), and U.S. Pat. No. 5,863,753 to Haugland et al. (1999) may be used, including nucleic acid stains commercially available under the trademarks SYBR Green, EvaGreen, SYTO, SYTOX, PICOGREEN, OLIGREEN, and RIBOGREEN from Molecular Probes, Inc., Eugene, Oreg. In yet other embodiments of the invention, the nucleic acid stain is a monomeric, homodimeric or heterodimeric cyanine dye that incorporates an aza- or polyazabenzazolium heterocycle, such as an azabenzoxazole, azabenzimidazole, or azabenzothiazole, that gives an enhanced fluorescence when associated with nucleic acids, including nucleic acid stains commercially available under the trademarks SYTO, SYTOX, JOJO, JO-PRO, LOLO, LO-PRO from Molecular Probes, Inc., Eugene, Oreg.

In yet other embodiments, a signal producing system that is specific for the amplification product, as opposed to nucleic acid molecules in general, may be employed to detect the amplification. In these embodiments, the signal producing system may include a probe nucleic acid that specifically binds to a sequence found in the amplification product (i.e. a reporter domain sequence), where the probe nucleic acid may be labelled with a directly or indirectly detectable label. A directly detectable label is one that can be directly detected without the use of additional reagents, while an indirectly detectable label is one that is detectable by employing one or more additional reagents, e.g., where the label is a member of a signal producing system made up of two or more components. In many embodiments, the label is a directly detectable label, where directly detectable labels of interest include, but are not limited to: fluorescent labels, radioisotopic labels, chemiluminescent labels, and the like. In many embodiments, the label is a fluorescent label, where the labelling reagent employed in such embodiments is a fluorescently tagged nucleotide(s), e.g. fluorescently tagged CTP (such as Cy3-CTP, Cy5-CTP) etc. Fluorescent moieties which may be used to tag nucleotides for producing labelled probe nucleic acids (i.e. detection probes) include, but are not limited to: fluorescein, the cyanine dyes, such as Cy3, Cy5, Alexa 555, Bodipy 630/650, and the like. Other labels, such as those described above, may also be employed as are known in the art.

In certain embodiments, the specifically labelled probe nucleic acids (detection probes) are labelled with "energy transfer" labels. As used herein, "energy transfer" refers to the process by which the fluorescence emission of a fluorescent group is altered by a fluorescence-modifying group. Energy transfer labels are well known in the art, and such labelled oligonucleotide probes include the TaqMan® type probes, as described in U.S. Patent No. 6,248,526, (as well as Held et al., Genome Res. (1996) 6:986-994; Holland et al., Proc. Natl Acad. Sci. USA (1991) 88:7276-7280; and Lee et al., Nuc. Acids Res. (1993) 21:3761-3766). Further examples of detection probes include: Scorpion probes (as described in Whitcombe et al., Nature Biotechnology (1999) 17:804-807; U.S. Patent No. 6,326,145, Sunrise probes (as described in Nazarenko et al., Nuc. Acids Res. (1997)25:2516-2521; U.S. Patent No. 6,117,635, Molecular Beacons (Tyagi et al., Nature Biotechnology (1996) 14:303-308; U.S. Patent No. 5,989,823, and conformationally assisted probes.

Thus, determining the presence of the RCA product may be achieved using any convenient protocol in order to detect the target analyte in the sample. In other words, the reaction mixture is screened etc. (i.e., assayed, assessed, evaluated, tested, etc.) for the presence of any resultant RCA products in order to detect the presence of the target analyte in the sample being assayed. The particular detection protocol may vary depending on the sensitivity desired and the application in which the method is being practiced. In certain embodiments, the RCA product may be directly detected without any further amplification, while in other embodiments the detection protocol may include an amplification component, in which the copy number of the RCA product is increased or the RCA product is used as a template for further RCA reactions, e.g., to enhance sensitivity of the particular assay.

Where detection without amplification is practicable, the RCA product may be detected in a number of different ways. For example, the nucleotides incorporated in the RCA product may be directly labelled, e.g., fluorescently, or otherwise spectrophotometrically, or radioisotopically labelled or with any signal-giving label, such that the RCA product is directly labelled. In some embodiments detection probes, e.g., fluorescently labelled probes, molecular beacons (as described above) may be employed to detect to the presence of the RCA product, where these probes are directed to a sequence (reporter domain sequence, i.e. a sequence that is identical to the reporter domain sequence in the RCA template) that is repeated in the RCA concatemer and therefore only exists in its entirety in the RCA product.

As indicated above, in certain embodiments of the subject methods, the detection step includes a further amplification step, where the copy number of RCA product (or a portion thereof, e.g. monomers derived therefrom) is increased, e.g., in order to enhance sensitivity of the assay. The amplification may be linear or exponential, as desired, where representative amplification protocols of interest include, but are not limited to: polymerase chain reaction (PCR); isothermal amplification, rolling-circle ampification (RCA) etc. Thus, in some embodiments, the RCA product may be detected by a RCA reporter as defined herein, i.e. in a so-called super-RCA reaction.

Where the detection step includes an amplification step (more specifically a step of *in vitro* amplification of the RCA product), the amplified product (or amplification product) may be detected, to detect the analyte.

The polymerase chain reaction (PCR) is well known in the art, being described in U.S. Pat. Nos.: 4,683,202; 4,683,195; 4,800,159; 4,965,188 and 5,512,462. In representative PCR amplification reactions, the reaction mixture that includes the above RCA product (which may also be viewed as a template nucleic acid in an amplification reaction) is combined with one or more primers that are employed in the primer extension reaction, e.g., the PCR primers (such as forward and reverse primers employed in geometric (or exponential) amplification or a single primer employed in a linear amplification). The oligonucleotide primers with which the template nucleic acid (hereinafter referred to as template DNA for convenience) is contacted will be of sufficient length to provide for hybridization to complementary template DNA under annealing conditions (described in greater detail below). The primers will generally be at least 5 bp in length, typically at least 6, 8 or 10 bp in length, usually at least 15 bp in length and more usually at least 16 bp in length and may be as long as 30 bp in length or longer, where the length of the primers will generally range from 5 to 50 bp in length, e.g. 6, 8 or 10 to 50 bp, usually from about 10 to 35 bp in length. The template DNA may be contacted with a single primer or a set of two primers (forward and reverse primers), depending on whether primer extension, linear or exponential amplification of the template DNA is desired.

In addition to the above components, the reaction mixture produced in the subject methods typically includes a polymerase and deoxyribonucleoside triphosphates (dNTPs). The desired polymerase activity may be provided by one or more distinct polymerase enzymes. In many embodiments, the reaction mixture includes at least a Family A polymerase, where representative Family A polymerases of interest include, but are not limited to: *Thermus aquaticus* polymerases, including the naturally occurring polymerase (Taq) and derivatives and homologues thereof, such as Klentaq (as described in Barnes et al, Proc. Natl. Acad. Sci USA (1994) 91:2216-2220); *Thermus thermophilus* polymerases, including the naturally occurring polymerase (Tth) and derivatives and homologues thereof, and the like. In certain embodiments where the amplification reaction that is carried out is a high fidelity reaction, the reaction mixture may further include a polymerase enzyme having 3'-5' exonuclease activity, e.g., as may be provided by a Family B polymerase, where Family B polymerases of interest include, but are not limited to: *Thermococcus litoralis* DNA polymerase (Vent) as described in Perler et al., Proc. Natl. Acad. Sci. USA (1992) 89:5577-5581; *Pyrococcus* species GB-D (Deep Vent); *Pyrococcus furiosus* DNA polymerase (Pfu) as described in Lundberg et al., Gene (1991) 108:1-6, *Pyrococcus woesei* (Pwo) and the like. Where the reaction mixture includes both a Family A and Family B polymerase, the Family A polymerase may be present in the reaction mixture in an amount greater than the Family B polymerase, where the difference in activity will usually be at least 10-fold, and more usually at least about 100-fold. Usually the reaction mixture will include four different types of dNTPs corresponding to the four naturally occurring bases present, i.e. dATP, dTTP, dCTP and dGTP. In the subject methods, each dNTP will typically be present in an amount ranging from about 10 to 5000 µM, usually from about 20 to 1000 µM.

The reaction mixture prepared in this detection step of the subject methods may further include an aqueous buffer medium that includes a source of monovalent ions, a source of divalent cations and a buffering agent. Any convenient source of monovalent ions, such as KCI, K-acetate, NH₄-acetate, K-glutamate, NH₄Cl, ammonium sulphate, and the like may be employed. The divalent cation may be magnesium, manganese, zinc and the like, where the cation will typically be magnesium. Any convenient source of magnesium cation may be employed, including MgCl₂, Mg-acetate, and the like. The amount of Mg²⁺ present in the buffer may range from 0.5 to 10 mM, although higher or lower amounts may be used and may depend on the type of reaction. For instance, for PCR the amount of Mg²⁺ present in the buffer may be about 1.5mM, whereas for RCA, the amount of Mg²⁺ present in the buffer may about 10mM. Representative buffering agents or salts that may be present in the buffer include Tris, Tricine, HEPES, MOPS and the like, where the amount of buffering agent will typically range from about 5 to 150 mM, usually from about 10 to 100 mM, and more usually from about 20 to 50 mM, where in certain preferred embodiments the buffering agent will be present in an amount sufficient to provide a pH ranging from about 6.0 to 9.5, where most preferred is pH 7.3 at 72 °C. Other agents which may be present in the buffer medium include chelating agents, such as EDTA, EGTA and the like.

The next step in the subject methods is signal detection from the labelled RCA or amplification products of interest, where signal detection may vary depending on the particular signal producing system employed. In certain embodiments, merely the presence or absence of detectable signal, e.g., fluorescence, is determined and used in the subject assays, e.g., to determine or identify the presence or absence of the target nucleic acid molecule. Depending on the particular label employed, detection of a signal may indicate the presence or absence of the target nucleic acid molecule.

In those embodiments where the signal producing system is a fluorescent signal producing system, signal detection typically includes detecting a change in a fluorescent signal from the reaction mixture to obtain an assay result. In other words, any modulation in the fluorescent signal generated by the reaction mixture is assessed. The change may be an increase or decrease in fluorescence, depending on the nature of the label employed, but in certain embodiments is an increase in fluorescence. The sample may be screened for an increase in fluorescence using any convenient means, e.g., a suitable fluorimeter, such as a thermostable-cuvette or plate-reader fluorimeter, or where the sample is a tissue sample on a microscope slide, fluorescence may be detected using a fluorescence microscope. Fluorescence is suitably monitored using a known fluorimeter. The signals from these devices, for instance in the form of photo-multiplier voltages, are sent to a data processor board and converted into a spectrum associated with each sample tube. Multiple tubes, for example 96 tubes, can be assessed at the same time. Thus, in some embodiments multiple analytes may be detected in parallel, whereas in other embodiments multiple analytes may be detected sequentially, e.g. one analyte at a time or one group of analytes at a time.

Where the detection protocol is a real time protocol, e.g., as employed in real time PCR reaction protocols, data may be collected in this way at frequent intervals, for example once every 3 minutes, throughout the reaction. By monitoring the fluorescence of the reactive molecule from the sample during each cycle, the progress of the amplification reaction can be monitored in various ways. For example, the data provided by melting peaks can be analyzed, for example by calculating the area under the melting peaks and these data plotted against the number of cycles.

The spectra generated in this way can be resolved, for example, using "fits" of pre-selected fluorescent moieties such as dyes, to form peaks representative of each signalling moiety (i.e. fluorophore). The areas under the peaks can be determined which represents the intensity value for each signal, and if required, expressed as quotients of each other. The differential of signal intensities and/or ratios will allow changes in labelled probes to be recorded through the reaction or at different reaction conditions, such as temperatures. The changes are related to the binding phenomenon between the oligonucleotide probe and the target sequence or degradation of the oligonucleotide probe bound to the target sequence. The integral of the area under the differential peaks will allow intensity values for the label effects to be calculated.

Screening the mixture for a change in fluorescence provides one or more assay results, depending on whether the sample is screened once at the end of the primer extension reaction, or multiple times, e.g., after each cycle, of an amplification reaction (e.g., as is done in real time PCR monitoring).

The data generated as described above can be interpreted in various ways. In its simplest form, an increase or decrease in fluorescence from the sample in the course of or at the end of the amplification reaction is indicative of an increase in the amount of the target analyte present in the sample, e.g., as correlated to the amount of amplification product detected in the reaction mixture, suggestive of the fact that the amplification reaction has proceeded and therefore the target analyte was in fact present in the initial sample. Quantification is also possible by monitoring the amplification reaction throughout the amplification process. Quantification may also include assaying for one or more nucleic acid controls in the reaction mixture, as described above.

In this manner, a reaction mixture may readily be screened (or assessed or assayed etc.) for the presence of target analyte(s), e.g. nucleic acid analytes. The methods are suitable for detection of a single target analyte as well as multiplex analyses, in which two or more different target analytes are assayed in the sample. In these latter multiplex situations, the number of different sets of probes that may be employed typically ranges from about 2 to about 20 or higher, e.g., as up to 100 or higher, 1000 or higher, etc. wherein the multiple analytes in a sample may be detected in parallel or sequentially.

The analysis of many analytes simultaneously and in a single reaction using several different probes (multiplexing) is enhanced by the increased specificity and sensitivity obtained when using the single RCA probes of the invention. Each probe set can be designed to produce a RCA product that can be used to determine the presence or absence, quantity and/or location of the analytes being interrogated by the RCA probe (or the intermediary binding partner(s) with which the probe interacts). The RCA product may be detected directly or after amplification using any of the well established methods for analysis of nucleic acid molecules known from the literature including liquid chromatography, electrophoresis, mass spectrometry, microscopy, real-time PCR, fluorescent probes, microarray etc.

The probes and methods of the present invention may be employed homogeneously (i.e. in solution) as described above, or alternatively heterogeneously, using a solid phase, for example, in which analyte becomes immobilised on a solid phase, permitting the use of washing steps. The use of solid phase assays offers advantages, particularly for the detection of difficult samples: washing steps can assist in the removal of inhibiting components, and analytes can be enriched from an undesirably large sample volume. Higher concentrations and greater amounts of probes can be used, as unbound analytes, probes and RCA products can be removed by washing.

Immobilisation of the analyte on a solid phase may be achieved in various ways. Accordingly, several embodiments of solid phase assays are contemplated. In one such embodiment, the analyte can first be captured by an immobilised (or immobilisable) capture probes and then bound by subsequently added probe(s).

The immobilised capture probe may be immobilised, i.e. bound to the support, in any convenient way. Thus the manner or means of immobilisation and the solid support may be selected, according to choice, from any number of immobilisation means and solid supports as are widely known in the art and described in the literature. Thus, the capture probe may be directly bound to the support (e.g. chemically crosslinked), it may be bound indirectly by means of a linker group, or by an intermediary binding group(s) (e.g. by means of a biotin-streptavidin interaction). Thus, a capture probe may be provided with means for immobilisation (e.g. an affinity binding partner, e.g. biotin or a hapten or a nucleic acid molecule, capable of binding to its binding partner, i.e. a cognate binding partner, e.g. streptavidin or an antibody or a nucleic acid molecule) provided on the support. The probe may be immobilised before or after binding to the analyte. Further, such an "immobilisable" capture probe may be contacted with the sample together with the support.

The capture probe may be, for example, an antibody or nucleic acid molecule that is capable of binding to the target analyte specifically. In other words the capture probe may be an immobilised (or immobilisable) analyte-specific probe comprising an analyte binding domain (i.e. an analyte capture probe). Thus in such an embodiment the analyte is first captured by the immobilised or immobilisable capture probe which serves only to immobilise the analyte on the solid phase, and subsequently the immobilised analyte is incubated with the probe of the invention. In such an embodiment, the capture probe may be any binding partner capable of binding the analyte, directly or indirectly, as discussed above in related to intermediary binding partners. More particularly, such a capture probe binds specifically to the analyte.

The solid support may be any of the well known supports or matrices which are currently widely used or proposed for immobilisation, separation etc. These may take the form of particles (e.g. beads which may be magnetic or nonmagnetic), sheets, gels, filters, membranes, fibres, capillaries, or microtitre strips, tubes, plates or wells etc.

The support may be made of glass, silica, latex or a polymeric material. Suitable are materials presenting a high surface area for binding of the analyte. Such supports may have an irregular surface and may be for example porous or particulate e.g. particles, fibres, webs, sinters or sieves. Particulate materials e.g. beads are useful due to their greater binding capacity, particularly polymeric beads.

Conveniently, a particulate solid support used according to the invention will comprise spherical beads. The size of the beads is not critical, but they may for example be of the order of diameter of at least 1 and preferably at least 2 µm, and have a maximum diameter of preferably not more than 10, and e.g. not more than 6 µm.

Monodisperse particles, that is those which are substantially uniform in size (e.g. size having a diameter standard deviation of less than 5%) have the advantage that they provide very uniform reproducibility of reaction. Representative monodisperse polymer particles may be produced by the technique described in US-A-4336173.

However, to aid manipulation and separation, magnetic beads are advantageous. The term "magnetic" as used herein means that the support is capable of having a magnetic moment imparted to it when placed in a magnetic field, i.e. paramagnetic, and thus is displaceable under the action of that field. In other words, a support comprising magnetic particles may readily be removed by magnetic aggregation, which provides a quick, simple and efficient way of separating the particles following the analyte binding steps.

In a further embodiment, the analyte itself may be immobilised (or immobilisable) on the solid phase e.g. by non-specific absorption. In a particular such embodiment, the analyte may be present within cells, being optionally fixed and/or permeabilised, which are (capable of being) attached to a solid support, e.g. a tissue sample comprising analyte may be immobilised on a microscope slide.

The above-described methods typically result in detection of target dependent RCA products (i.e. RCA products that are only produced in the presence of the target analyte) that are present in the reaction mixture, which in turn provides a measure of the amount of target analyte in the sample being assayed. The measure may be qualitative or quantitative.

Accordingly, the above described probes and methods for detecting the presence of one or more target analytes in a complex sample find use in a variety of different applications.

The subject probes and methods may be used to screen a sample for the presence or absence of one or more target analytes in a sample. As indicated above, the invention provides probes and methods for detecting the presence or quantifying the amount of one or more target analytes in a sample.

The subject probes and methods can be employed to detect the presence of one or more target analytes in a variety of different types of samples, including complex samples having large amounts of non-target entities, where the probe of the invention allows for superior detection of the target analyte(s) over equivalent methods that utilise the RCA probes, e.g. padlock probes. As such, the subject probes provide methods that are highly sensitive for detecting one or more target analytes in a simple or complex sample. The sample that is assayed in the subject methods is, in many embodiments, from a physiological source, as discussed in more detail above.

It will be evident from the description above and the representative examples described below that the probes and methods of the invention have numerous advantages over existing methods. Advantageously, the use of single RCA probes that provide the nucleic acid components sufficient to initiate RCA renders the probes and methods of the invention particularly useful for the simultaneous detection of multiple analytes in a sample, i.e. multiplex assays. Furthermore, each probe may result in a RCA product that is unique, which allows multiple analytes to be detected in parallel. Alternatively, for assays used to detect a large number of analytes, it may be useful to detect (e.g. visualise) the RCA products sequentially, e.g. one at a time or one group at a time. The probe of the invention enable other reagents to be added to the assay at the same time as the probes. As the RCA nucleic acid components provided by the probes cannot initiate a RCA reaction until the probe has been cleaved and/or unfolded thereby minimising RCA products that arise from non-specific interactions. Reducing the number of steps in the assay minimises potential errors and renders the protocol more suitable for automation.

The invention will be further described with reference to the following nonlimiting Examples with reference to the following drawings in which:
Figure 1 shows a circle RCA probe, wherein binding of the probe to the target nucleic acid molecule forms a cleavage recognition site (A), which is cleaved (B) to release the primer for extension (C).
Figure 2 shows a two-part hairpin RCA probe bound to the nucleic acid domains of proximity probes that are bound to an analyte immobilized on a substrate. A and A', and B and B', represent complementary sequences, wherein the domain comprising A and B functions as the ligation template for circularisation of the RCA template by hybridizing to A' and B' after cleavage of the probe. C represents a cleavage domain.
Figure 3 shows three variants of two-part hairpin RCA probes. Probes 1 and 2 require a target templated ligation to allow the RCA components to be maintained in proximity after their release by cleavage. Probe 3 may be involved in a target templated ligation, but this is not essential. A and A', and B and B', represent complementary sequences, wherein the domain comprising A and B functions as the ligation template for circularisation of the RCA template by hybridizing to A' and B' after cleavage of the probe. C represents a cleavage domain.
Figure 4 shows a circle RCA probe comprising a protection strand. The protection strand is displaced when the probe binds to the target nucleic acid molecule.
Figure 5 shows a circle RCA probe, wherein the RCA template functions as a protection strand. The RCA template protection strand is displaced to a second RCA template complementary domain when the probe binds to the target nucleic acid molecule.
Figure 6 shows a circle RCA probe comprising a cleavage strand. A cleavage recognition site is formed when the probe binds to the target nucleic acid molecule, which allows the cleavage domain formed by the cleavage strand to be cleaved. Cleavage releases the RCA primer.
Figure 7 shows a circle RCA probe as described in figure 6, wherein the cleavage domain is formed by a hairpin structure.
Figure 8 shows two variants of a circle RCA probe. The probe on the left is unfolded by binding to the target nucleic acid molecule and subsequently cleaved. The probe on the right does not require a target interaction for cleavage to occur. The probe is depicted as detecting a primary RCA product.
Figure 9 shows a circle RCA probe, wherein the primer strand is also a circular oligonucleotide.
Figure 10 shows a circle RCA probe comprising an invasion strand. When the probe binds to the target, the invasion strand is displaced from the probe to release the RCA primer.
Figure 11 shows a three-part RCA hairpin probe comprising two cleavage strands, wherein ab is the primer domain, the domain labelled A in the hairpin is complementary to the domain labelled A in the duplex and the domain labelled B directly adjacent to the stem-loop structure is complementary to the domain labelled B in the duplex. The domains in between ab and B, and to the right of the AB duplex, are cleavage domains.
Figure 12 shows a two-part hairpin RCA probe bound to (A) a nucleic acid molecule and (B) the nucleic acid domains of proximity probes, wherein domains labelled A and B are complementary and C represents a cleavage domain.
Figure 13 shows a three-part hairpin RCA probe bound to a nucleic acid molecule, wherein domains labelled A, B and ab are complementary and C represents a cleavage domain.
Figure 14 shows a variety of two-part probes in (A)-(G), wherein the following domains are complementary: P1 and P1'; P2 and P2'; N1 and N1'; and C and C'. R1 and R2 are used to label target binding domains. The target binding domains in (E) are formed by coupling the probe to antibodies. The target binding domains in (F) are formed by coupling the probe to nucleic acid aptamers. C represents a cleavage domain.
Figure 15 shows four-part (A) and five-part (B) hairpin RCA probes bound to nucleic acid molecules, wherein domains labelled A1, A2, B1, B2 and ab are complementary. (C) shows dual labelled blobs resulting from the detection of an immobilized target nucleic acid molecule.
Figure 16 shows a circle RCA probe wherein a cleavage domain is formed when the probe binds to the target nucleic acid molecule to form a stem loop structure. Domains labelled P2 represent complementary sequences.
Figure 17 shows a bar chart of the comparative results of a protein detection assay using a padlock probe or a two-part probe, which interact with the nucleic acid domains of proximity probes.
Figure 18 shows a bar chart of the comparative results of a nucleic acid detection assay using a continuous three-part probe or a three-part probe comprising cleavage strands.
Figure 19 shows (A) a graph of the signal:noise ratio of a two-part hairpin cleavage strand probe in the detection of a nucleic acid molecule immobilized to a substrate via a biotin/streptavidin interaction and (B) a bar chart of the comparative results of a nucleic acid detection assay using a continuous two-part probe or a two-part probe comprising a cleavage strand.
Figure 20 shows (A) a two-part linear RCA probe bound to the nucleic acid domains of proximity probes that are bound to an analyte immobilized on a substrate and (B) a graph of a the signal:noise ratio of the two-part linear RCA probe in the detection of IL6.
Figure 21 shows the simultaneous *in situ* detection of beta-actin mRNA and protein using two-part hairpin RCA probes. Only a selection of the RCA products are circled, wherein the blobs in the large circles represent protein and the blobs in the small circles represent mRNA.
Figure 22 shows the detection of a single nucleotide polymorphism (SNP) in a nucleic acid molecule using a target ligation dependent two-part hairpin probe, wherein (A) shows detection of a nucleic acid molecule comprising the correct sequence, (B) shows a reduced signal in the detection of a nucleic acid molecule comprising the wrong sequence and (C) shows no signal in the absence of target nucleic acid.
Figure 23 shows the detection of a RCA product using a circle RCA probe, wherein (A) shows no secondary RCA products are generated in the absence of primary RCA product and (B) shows that the primary and secondary RCA products co-localise.
Figure 24 shows the signal generated from the hybridisation of detection oligonucleotides to RCA products in the presence or absence of a polymerase with strand displacement activity. Detection oligonucleotides hybridised to a RCA product are depicted, wherein the label is illustrated as a star, oligonucleotides with a free 3' end are illustrated as an arrow and oligonucleotides blocked with three 2'-O-methylated RNA bases are illustrated as a dot.
Figure 25 shows a secondary RCA reaction using a padlock probe (A) or two-part hairpin RCA probe (B).

### EXAMPLES

### Example 1 - Two-part hairpin RCA probe

As described above, the probes of the invention may be used in the detection of any analyte, wherein an intermediary binding partner that can bind to the analyte is coupled to (or comprises) a nucleic acid molecule. The nucleic acid molecule(s) coupled to the intermediary molecule may be detected by the probe and acts as a proxy or marker for the analyte. The protocol described below utilizes proximity probes as intermediary binding molecules for the target analyte, wherein the target complementary domains of the RCA probe bind to the nucleic acid domains of the proximity probes (see e.g. Figure 2). The RCA probe was compared to a padlock probe, utilizing a gap oligonucleotide, which is also able to bind to the nucleic acid domains of the proximity probes to generate a RCA template. The RCA reaction for the padlock probe RCA template was primed by one of the proximity probe nucleic acid domains.

Fifty µl of biotinylated mouse IgG (Sigma F9291) in concentration series (10 nM, 1 nM, 0.1 nM) was incubated in 1x PBS on a streptavidin coated glass slide (SurModics), with no IgG spiked in 1xPBS as negative control. After incubation at 37°C for 60 min, the slides were blocked with blocking buffer at 37°C for 60 min. After blocking, 50 µl of 5 nM PLA (proximity ligation assay) probes (goat polyclonal IgG against IgG (R&D system) conjugated to DNA oligonucleotides) were added to reaction chambers followed by incubation at 37°C for 60 min.

Prior to the experiment, the streptavidin coated slide was compartmentalized with secure-Seal 8 (Grace Bio-labs). A washing step, with two repetitions of 50 µl 1x PBS 0.05% Tween20, was performed before every addition of new reagent mixes, for removal of previous incubation reagents.

The "two-part" RCA probes were prepared by incubation of the cleavage strand with the RCA template strand in 100:1 molar ratio in Mg hybridization buffer at 37°C for 60 min. After the PLA probe incubation step, the two-part probes were diluted in blocking buffer to 20 nM and 50 µl of the resultant solution was applied to the reaction chambers comprising the immobilized biotinylated mouse IgG. After incubation at 37°C for 60 min, the unbound probes were removed by washes. A series of reactions were carried out to release/generate the RCA nucleic acid components and amplification of RCA template.

First 50 µl unfolding and cleavage mix (i.e. primer and RCA tempalte release mix) (1x NEB4 buffer (New England Biolabs (NEB), 0.5 µg/µl BSA (NEB), 50 units of Nb Btsl (NEB), 50 units of Mlyl (NEB)) and 500 pmol cleavage strands were added to each well. In embodiments where the primer domain (which is also the ligation template domain) is provided by a hairpin structure, the presence of additional cleavage strands is not required.

After incubation at 37°C for 60 min followed by washes, 50 µl of a further cleavage mix (i.e. to remove the cleavage strand) (1x NEB4 buffer (NEB), 0.5 µg/µl BSA (NEB) and 5 units of UNG (Fermentas), 1 µg/µl BSA (NEB), 5 units of UNG (Fermentas) and 5 units of EndoIV (Fermentas)) was added. The reaction was incubated at 37°C for 30 min to release the RCA nucleic acid components. After washes, 50 µl of ligation mix (1x NEB4 buffer (NEB), 0.5 µg/µl BSA (NEB), 15 units of T4 DNA ligase (Fermentas) and 0.5 mM ATP (Fermentas)) was added to the reaction chambers. The ligation reaction was incubated at 37°C for 30 min, followed by washes and addition of 50 µl of rolling circle amplification (RCA) mix (1x phi29 buffer (Fermentas), 0.2 µg/µl purified BSA (NEB), 0.25 mM dNTPs (Fermentas), 25 units of phi29 (Fermentas)). The RCA reactions were carried out at 37°C for 60 min. The RCA products were visualized by hybridization of 10nM fluorescently labelled probes (detection probes), by incubating at 37°C for 30 min, followed by washes and an ethanol series. Thereafter slides were mounted with VectaShield (Immunkemi) and a cover slip and imaged. The microscopic images were analyzed with ImageJ and the blobs were enumerated.

In the case of padlock probe (S3) system, a ligation mix (1x NEB4 buffer (NEB), 0.5 µg/µl BSA (NEB), 15 units of T4 DNA ligase (Fermentas), 0.5 mM ATP (Fermentas)) and 100 nM of two DNA oligonucleotides (for sequences see Söderberg et al, 2006 Nature Methods, Vol. 3(12), pp. 995-1000,) were added instead of the two-part probe. After incubation at 37°C for 60 min, RCA reactions were initiated by addition of 50 µl RCA mix (described above). The RCA products generated from the two-part probes and padlock probes were visualized and enumerated by the protocol described above.

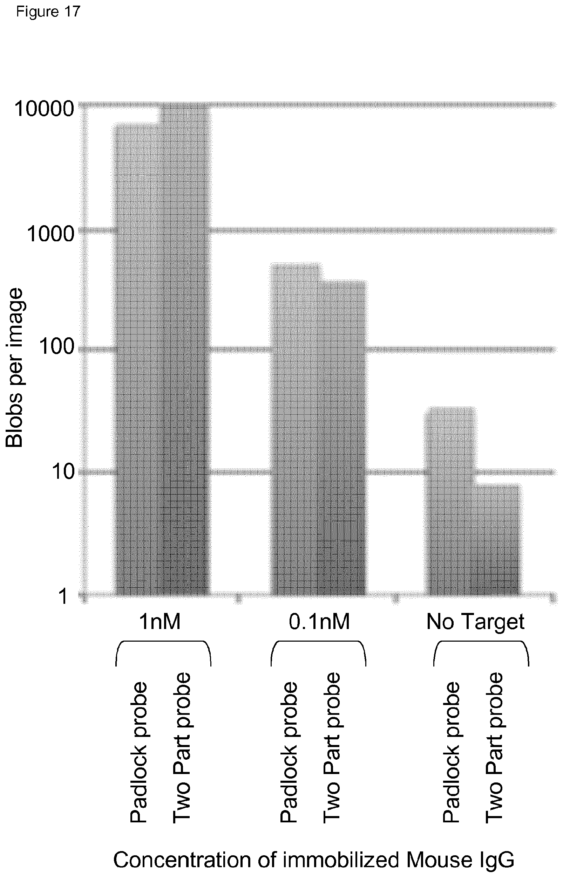

Figure 17 shows that the two-part probe generates a similar or better signal than the padlock probe in the presence of the target analyte and shows a reduced signal in comparison the padlock probe in the absence of target analyte, i.e. the two-part probe shows an improvement in the signal to noise ratio in comparison to a padlock probe.

### Example 2 - Three part hairpin RCA probe

The utility of the "three-part" RCA probe was shown using a nucleic acid analyte, i.e. the target analyte was a nucleic acid molecule. Two variants of the "three-part" probe were tested, i.e. a single stranded probe comprising three hairpin structures and a partially double stranded probe comprising a hairpin structure and two cleavage strands.

Fifty µl of 10 pM synthetic biotinylated DNA template was incubated in 1x PBS on a streptavidin coated glass slide (SurModics) at 37°C for 60 min, with no DNA spiked in 1×PBS as negative control. After incubation, the slides were blocked with blocking buffer (0.1% BSA (Sigma), 100 nM goat IgG (Sigma), 1 mM biotin (Sigma), 10 µg/µl salmon sperm DNA (Sigma), 5 mM EDTA, 1x PBS and 0.05% Tween 20) at 37°C for 60 min.

Prior to the experiment, the streptavidin coated slide was compartmentalized with secure-Seal 8 (Grace Bio-labs). A washing step, with two repetitions of 50 µl 1× PBS, 0.05% Tween20, was added before every new addition of reagent mixes, for removal of previous incubation reagents.

The three-part probe comprising cleavage strands was prepared by incubation of the cleavage strands with the RCA template strand in 100:1 molar ratio in Mg hybridization buffer (50 mM KAc, 20 mM TrisAc, 10 mM MgAc and 1 mM DTT) at 37°C for 60 min. The single stranded (continuous) probe was incubated in 1 M NaCl at 95°C for 5 min and cooled to 20°C with temperature decrease rate at 1°C/sec to allow the formation of the hairpin structures. The probes were then diluted in blocking buffer to 20 nM and 50 µl of each probe was applied to the reaction chamber comprising the immobilized target nucleic acid molecules. After incubation at 37°C for 60 min, the unbound probes were removed by washes. A series reactions were carried out to release/generate the RCA nucleic acid components and amplification of RCA template.

First 50 µl unfolding and cleavage mix (i.e. primer and RCA template release mix) (1x NEB4 buffer (New England Biolabs (NEB), 0.5 µg/µl BSA (NEB), 50 units of Nb Btsl (NEB), 50 units of Mlyl (NEB)) and 500 pmol cleavage strands were added to each well. The unfolding mix did not contain cleavage strands in the reaction comprising the continuous (single stranded) three-part RCA reporter.

After incubation at 37°C for 60 min followed by washes, 50 µl of additional cleavage mix (e.g. to remove the cleavage strands or to release RCA components) was added. The cleavage mix for the three-part probe comprising cleavage strands contained: 1x NEB4 buffer (NEB), 0.5 µg/µl BSA (NEB) and 5 units of UNG (Fermentas), 1 µg/µl BSA (NEB), 5 units of UNG (Fermentas) and 5 units of EndoIV (Fermentas). The additional cleavage mix for the continuous three-part probe contained: 1x unfolding buffer (20 mM Tris-HCl, 30 mM NaCl, 1 mM EDTA, 100 mM KCI and 1 mM DTT), 1 µg/µl BSA (NEB), 5 units of UNG (Fermentas) and 5 units of EndoIV (Fermentas). The reaction was incubated at 37°C for 30 min to release the RCA nucleic acid components. After washes, 50 µl of ligation mix (1x NEB4 buffer (NEB), 0.5 µg/µl BSA (NEB), 15 units of T4 DNA ligase (Fermentas) and 0.5 mM ATP (Fermentas)) was added to the reaction chambers. The ligation reaction was incubated at 37°C for 30 min, followed by washes and the addition of 50 µl of rolling circle amplification (RCA) mix (1x phi29 buffer (Fermentas), 0.2 µg/µl purified BSA (NEB), 0.25 mM dNTPs (Fermentas), 25 units of phi29 (Fermentas)). The RCA reactions were carried out at 37°C for 60 min. The RCA products were visualized by hybridization of 10nM fluorescently labelled probes (detection probes), by incubating at 37°C for 30 min, followed by washes and ethanol series. Thereafter slides were mounted with VectaShield (Immunkemi) and a cover slip and imaged. The microscopic images were analyzed with ImageJ and the blobs were enumerated.

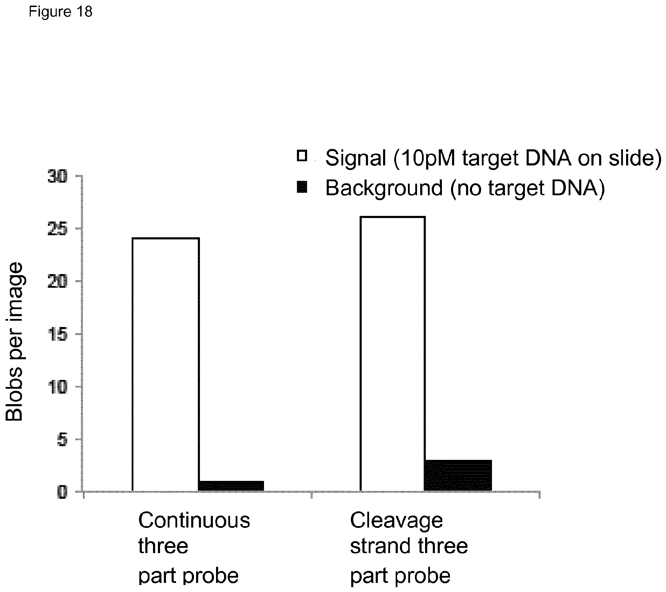

Figure 18 shows that the continuous three-part probe generates a similar signal to the three-part probe comprising cleavage strands in the presence of the target analyte. Both probes shows a very low signal in the absence of target analyte. The continuous the three-part probe shows a better signal to noise ratio than the cleavage strand three-part probe.

### Example 3 - Continuous and cleavage strand two part probes in the detection of a nucleic acid target

The utility of the "two-part" RCA probes was shown using a nucleic acid analyte, i.e. the target analyte was a nucleic acid molecule. The target nucleic acid was immobilized on a glass slide using two different techniques. Two variants of the "two-part" probe were tested, i.e. a continuous probe comprising two hairpin structures and a partially double stranded probe comprising a hairpin structure and a cleavage strand.

### Synthetic DNA template immobilization

Synthetic biotinylated DNA template in 10 µl 1x PBS was contacted with a streptavidin coated glass slide (SurModics) at 55°C for 10 min. Alternatively, DNA template was hybridized to slides in 50 µl 1x PBS at 37°C for 60 min with 1xPBS as a negative control. After incubation, the slides were blocked with blocking buffer (0.1% BSA (Sigma), 100 nM goat IgG (Sigma), 1 mM biotin (Sigma), 10 nµ/µl salmon sperm DNA (Sigma), 5 mM EDTA, 1x PBS and 0.05% Tween 20) at 37°C for 60 min. Prior to the experiment, the streptavidin coated slide was compartmentalized with secure-Seal 8 (Grace Bio-labs). A washing step, with two repetitions of 50 µl 1x PBS 0.05% Tween20, was performed before every addition of new reagent mixes, for removal of previous incubation reagents.

### Assembly of continuous probes

Limited by the available DNA synthesis service (single stranded DNA are delivered up to 200 bp), two part (and three part) continuous probes were ordered in their constituent parts. Probes were assembled by incubation of all parts with equal molar ratio in ligation mix (1X NEB4 buffer (NEB), 0.5 mM ATP (Fermantas), 0.6 unit/µl T4 DNA ligase) at 37°C for 30 min. The reaction mix was applied in 6% TBE-Urea gel (Life Technologies) according to manufacture's manual. The probes were then recovered by purifying DNA fragments at the correct sizes from the gel.

### Preparation and hybridization of two-part probes

Two-part probes with hybridized cleavage strands were prepared by incubation of cleavage strands with the RCA template strand in 100:1 molar ratio in Mg hybridization buffer (50 mM KAc, 20 mM TrisAc, 10 mM MgAc and 1mM DTT) at 37°C for 60 min. Two-part continuous probes were prepared by incubating the probes in 1 M NaCl at 95°C for 5 min and cooling down to 20°C with temperature decrease rate at 1°C/sec. The probes were then diluted in blocking buffer to 20 nM of which 50 µl were applied to the reaction chamber and immobilized with synthetic templates.

### Unfolding and cleavage reactions

After incubation at 37°C for 60 min, the unbound probes were removed by washes. The cleavage and unfolding reactions were carried out as described in Examples 2 and 3, wherein additional cleavage strands were not included in the assay using the continuous two part probe.

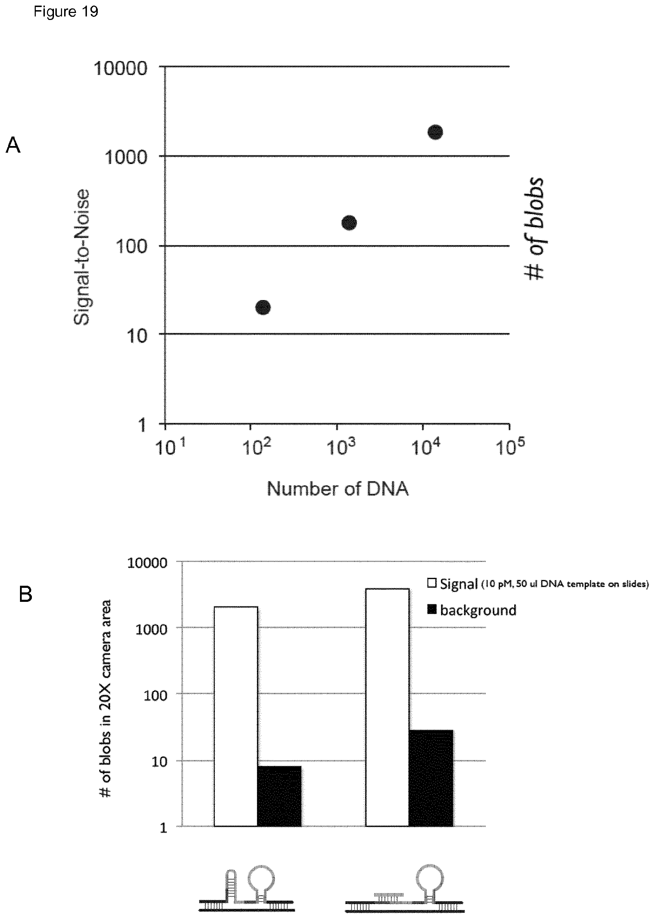

Figure 19A shows the signal obtained from the two-part hairpin cleavage strand probe using target DNA immobilized via a biotin/streptavidin interaction. Figure 19B shows the signal obtained from target DNA hybridised to a glass slide and demonstrates that the continuous two-part probe generates a similar signal to the two-part probe comprising cleavage strands in the presence of the target analyte. Both probes shows a low signal in the absence of target DNA. The continuous the two-part probe shows a better signal to noise ratio than the cleavage strand two-part probe.

### Example 4 - Two-part linear probe

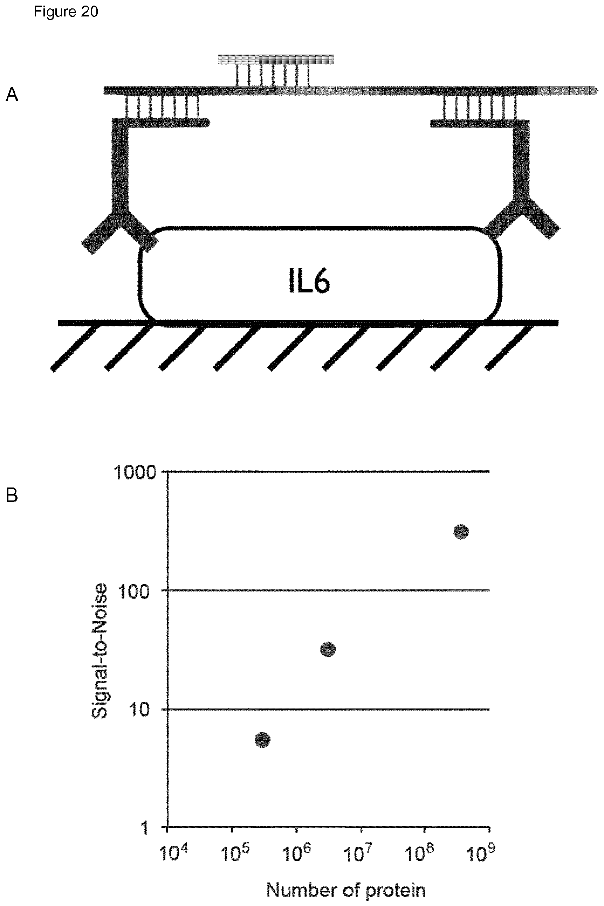

A linear two-part probe was used to detect interleukin-6 (IL6) using proximity probes as the intermediary binding partner (see Figure 20A).

In this experiment antigen IL6 was immobilized on a streptavidin coated slide by drying in 10 µl 1x PBS at 55°C for 10 min with 1xPBS as a negative control. After incubation at 37°C for 60 min, the slides were blocked with blocking buffer at 37°C for 60 min. After blocking, 50 µl of 5 nM PLA (proximity ligation assay) probes (goat polyclonal IgG against IL6 conjugated to DNA oligonucleotides) were added to reaction chambers followed by incubation at 37°C for 60 min.

The cleavage and detection reactions were performed as described in Example 1. Figure 20B shows the signal:noise ratio for three different concentrations of IL6.

### Example 5 - In situ detection of actin protein and mRNA using cleavage strand two-part hairpin probes

### Cell preparation

BJhTERT cells were seeded on the slides and allowed to attach and expand to the desired confluence. Cells were then fixed in 3% (w/v) paraformaldehyde (PFA) (Sigma) in PBS for 30 min at room temperature. After fixation, slides were washed twice in DEPC-treated PBS and stored in 70% ethanol at 4°C for at least 8 hours before being used. Fixed cells, attached on slides, were incubated with 0.1 M HCl for 2 min followed by washing in 1x PBS.

### Antibody hybridization

Mouse monoclonal beta-actin antibody (Abcam, ab6277), diluted 1:100 in 50 µl of blocking buffer, containing 5 Units of RNase Inhibitor (Fermentas), was incubated with cells at 37°C for 30 min. After washes, 5 nM of antibodies against mouse IgG conjugated with DNA oligonucleotides was applied to the cells in 50 µl blocking buffer with 5 Units of RNase Inhibitor. Incubation was carried out at 37°C for 30 min, followed by washes and post-fixation with 3.7% PFA at room temperature for 5 min.

### Addition and processing of two-part probes

Twenty nM of two-part probes, targeting either beta-actin transcripts or the DNA oligonucleotides conjugated to the antibodies, were applied to the cells in 50 µl of blocking buffer containing 5 Units of RNase Inhibitor. Incubation was carried out at 37°C for 60 min. After washes, a series of reactions were carried out for the formation and amplification of RCA reporters, following the protocol described in Example 1. Upon a 1 h RCA reaction at 37°C, a common primer motif was hybridized to the blobs representing detection of beta-actin protein or mRNA.

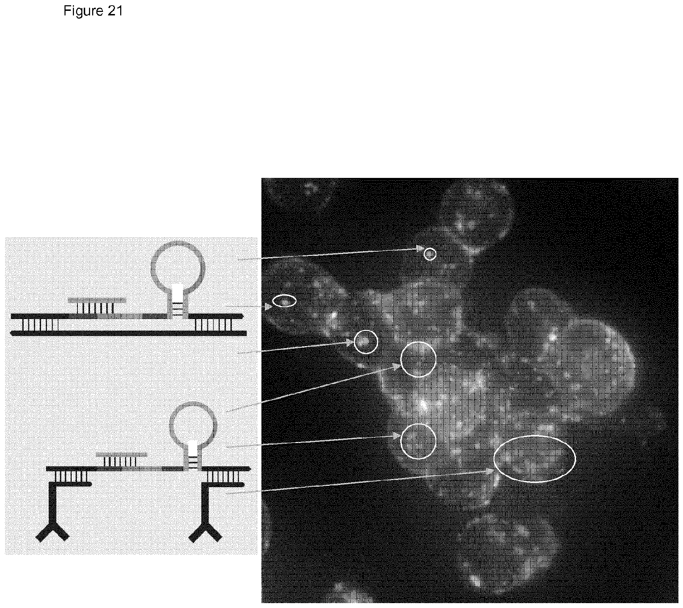

Figure 21 shows that the two-part probes were able to detect beta-actin protein (large circles) and mRNA (small circles) in the same cells simultaneously. Only a selection of the RCA products are circled in Figure 21.

### Example 6 - Five-part hairpin probe

A five-part hairpin probe (as shown in Figure 15B) was used to detect DNA immobilized to a glass slide.

Steps for the immobilization of a synthetic DNA target, preparation and hybridization of the probes, and the cleavage and detection steps were performed following the protocols described in Example 1-3.

Similarly to Example 3, the probes were ordered in parts: the RCA primer domain, first part of the RCA template (padlock 1), second part of the RCA template (padlock 2) and two ligation template domains (ligation 1 and 2) along with their connection templates. The RCA primer part was biotinylated at its 5' end. The five part probes were assembled by first immobilizing the RCA primer part oligonucleotides on streptavidin coated beads (MyOne T1 beads, Invitrogen). Approximately 1/8 coating positions per bead were occupied by the RCA primer part oligonucleotides. The other four parts (padlock 1, padlock 2, ligation 1 and ligation 2) were sequentially added to the RCA primer part oligonucleotides by an iterating protocol with addition of ligation mix containing a two fold molar excess of probe parts and a 20 fold molar excess of connection templates in 1x ligation buffer (400 mM Tris-HCl, 100 mM MgCl₂), 0.1 u/µl T4 DNA ligase (Fermantas) and 0.5 mM ATP, incubating at 37°C for 30 min. Before every addition of a new mix, a washing step was applied to the beads, comprising incubation in 1× PBS 0.05% Tween-20 at room temperature and 0.1× SSC at 46 °C for 30 min. After the last ligation reaction, the DNA ligation products were released from the beads by incubating the beads with 95% formamide at 90°C for 5 min. Supernatants were applied to a 6% TBE-Urea gel (Life Technologies) according to manufacture's manual. The five-part probes were then recovered by purifying DNA fragments at an approximate size of 500 bp from the gel.

Figure 15C shows that RCA products could be detected using a five part probe. Two "blobs" are seen for each RCA product as each ligation domain acts as a reporter domain, meaning that the RCA product was dual labelled.

### Example 7 - Target ligation dependent two-part hairpin probes for the detection of SNPs

This experiment demonstrates detection of a SNP using two-part probes that use the target nucleic acid molecule as a ligation template for an intramoleular ligation (see e.g. Figure 3). Two potential target molecules were immobilized on a glass slide, wherein a first "correct" target provided a probe binding site with perfect complementarity to the target bindings domain of the probe. A second "wrong" target contained a single mis-match (from C to A) in the probe binding site. The probe binding site acts at the ligation template for the intramolecular ligation of the probe.

The steps for the synthetic DNA target immobilization, probe preparation, cleavage and detection followed the protocols described in Examples 1 to 3.

Probe hybridization in this experiment was performed as follows. Twenty nM of two-part probes comprising cleavage strands was applied to the reaction chambers in 50 µl of ligation mix (1x NEB4 buffer (NEB), 0.5 µg/µl BSA (NEB), 15 units of T4 DNA ligase (Fermentas) and 0.5 mM ATP (Fermentas)). The ligation reaction was incubated at 37°C for 60 min.

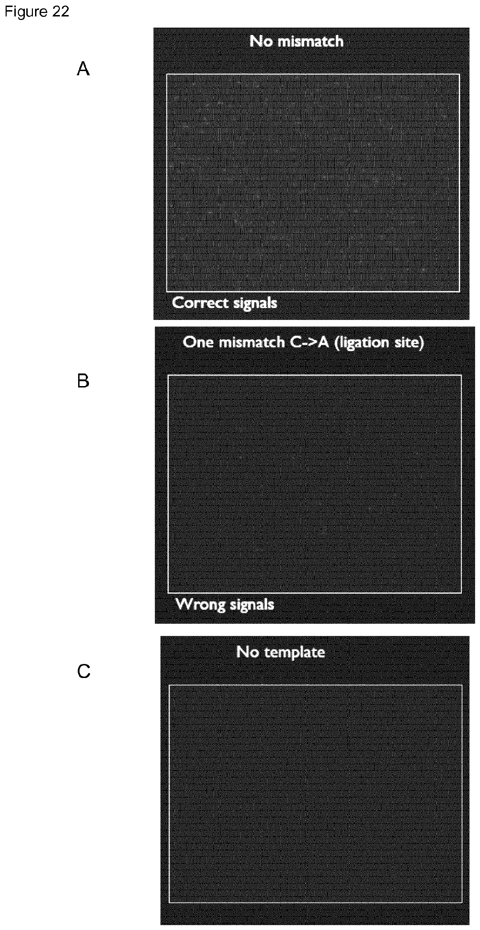

Figure 22 shows that target dependent ligation of the probe enables the discrimination between two targets containing a SNP. The percentage of RCA products generated from the wrong target was only 6% of the number of RCA products generated from the correct target.

### Example 8 - Circle RCA probe for the detection of a RCA product

The Example demonstrates the utility of a circle RCA probe using a RCA product as the target in a so-called "super RCA (sRCA)" reaction, as depicted in the unfolding embodiment of Figure 8.

A pre-ligated padlock was amplified via RCA using phi29 polymerase for 1 h at 37°C. The circle RCA probe was then spiked-in and the reaction was allowed to proceed for 1 h at 37°C followed by 10 min at 65°C to inactivate the phi29 polymerase. Cy-3 or FITC-labeled oligos targeting the primary and secondary RCA products, respectively were subsequently added to the sRCA reaction and allowed to hybridize for 20 min at 55°C. The sRCA products were allowed to adhere to poly-L-lysine coated slides and then visualized via epifluorescent microscopy with a 20X objective, exposure time 1500 ms.

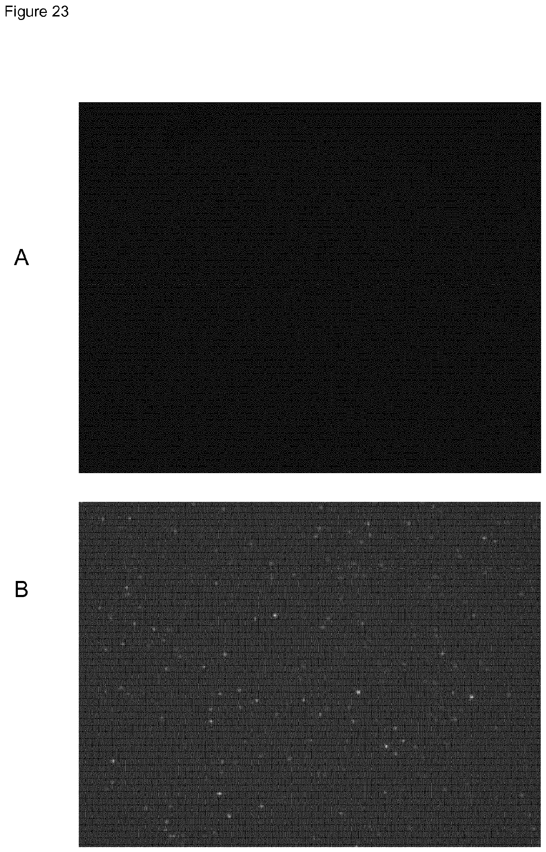

Figure 23A shows that no secondary RCA products were generated in the absence of a primary RCA product. Figure 23B shows that the primary and secondary RCA products co-localise.

### Example 9 - Importance of blocking 3' end of probes to avoid target templated extension and subsequent displacement of probes on targets with multiple probe binding sites

Prior to experiment, the streptavidin coated glass slide (SurModics) was compartmentalized with secure-Seal 8 (Grace Bio-labs). Fifty µl of 1 pM synthetic biotinylated DNA template was incubated in 1x PBS in each reaction chamber at 37°C for 60 min. After incubation, the slides were blocked with blocking buffer (0.1% BSA (Sigma), 100 nM goat IgG (Sigma), 1 mM biotin (Sigma), 10 µg/µl salmon sperm DNA (Sigma), 5 mM EDTA, 1x PBS and 0.05% Tween 20) at 37°C for 60 min. A washing step, with two repetitions of 50 µl 1× PBS 0.05% Tween20, was performed before every addition of a new mix, to remove of previous incubation reagents.

Fifty µl ligation mix (1x NEB4 buffer (NEB), 0.5 µg/µl BSA (NEB), 15 units of T4 DNA ligase (Fermentas) and 0.5 mM ATP (Fermentas)) and 100 nM of padlock probe was added to the reaction chambers. The ligation reaction was incubated at 37°C for 30 min, followed by washes and the addition of 50 µl rolling circle amplification (RCA) mix (1x phi29 buffer (Fermentas), 0.2 µg/µl purified BSA (NEB), 0.25 mM dNTPs (Fermentas), 25 units of phi29 (Fermentas)). The RCA reactions were carried out at 37°C for 60 min.

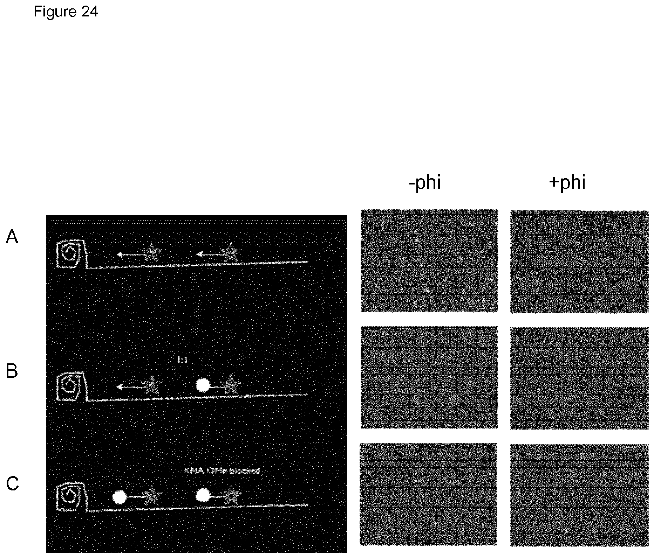

Fifty µl detection mix containing 100 nM detection oligonucleotides in 20% Formamide (Sigma) and 2x SSC (300 mM NaCl, 30 mM Na-citrate) was added in each chamber. Incubation was carried out at 37°C for 30 min. Detection oligonucleotides labelled with Alex555 (illustrated as a star in Figure 24) used in this experiment were identical in their binding sequence to the RCA products but different in their 3' ends, which were either free (illustrated as an arrow in Figure 24) or blocked with three 2'-O-methylated RNA bases (illustrated as a dot in Figure 24). RCA products were labelled with one of the two detection oligonucleotides (Figure 24A and C) or both detection oligonucleotides with 1:1 ratio (Figure 24B). The labelling reactions were done in duplicates.

After hybridization of detection oligonucleotides, chambers were washed. Fifty µl of 1XPBS, 0.05% Tween20 was added in one of the replicated chambers (Figure 24 column labelled -phi), whereas 50 µl of phi29 mix (1x phi29 buffer (Fermentas), 0.2 µg/µl purified BSA (NEB), 0.25 mM dNTPs (Fermentas), 25 units of phi29 (Fermentas)) was added in the other replicated chamber (Figure 24 column labelled phi+). Incubation was carried out at 37°C for 60 min. Thereafter slides were washed, dried, mounted with VectaShield (Immunkemi) and a cover slip and imaged.

Figure 24 demonstrates that the nucleic acid molecules hybridized to a RCA product are displaced when target templated extension is allowed to occur. In this example detection oligonucleotides were displaced, but the principle is applicable to detection probes, e.g. RCA reporters, that are required to remain attached to the target nucleic acid molecule.

### Example 10 - A comparison of secondary RCA using a padlock probe and a RCA reporter

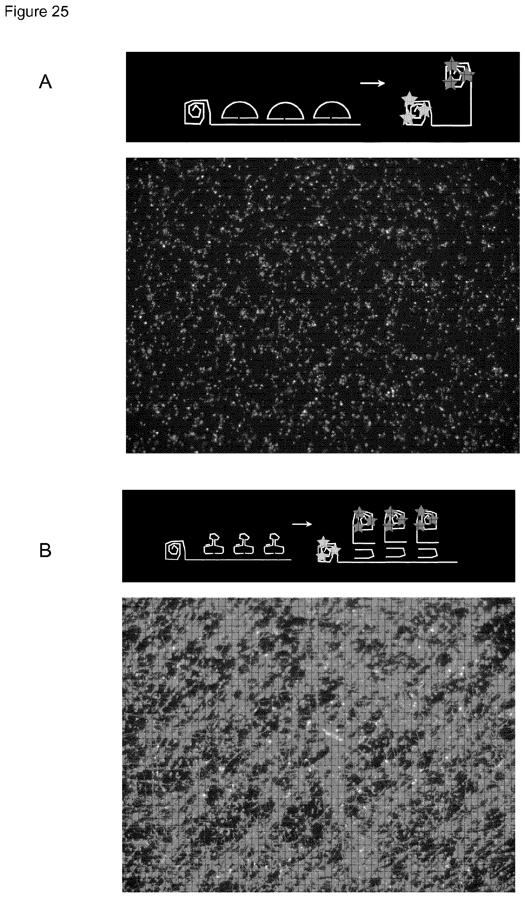

Primary RCA products were generated on a slide by the protocol described in Example 9. Fifty µl of ligation mix (1x NEB4 buffer (NEB), 0.5 µg/µl BSA (NEB), 15 units of T4 DNA ligase (Fermentas) and 0.5 mM ATP (Fermentas)) and 100 nM of padlock probes or 20 nM of target ligation dependent two-part hairpin probes (comprising cleavage strands, e.g. as shown in Figure 3) were added to the reaction chambers. The ligation reaction was incubated at 37°C for 60 min. After washes, 50 µl of 3' end termination mix (1X terminal transferase buffer (NEB), 0.25 mM CoCl2 100 nM dUTP, 10 units terminal transferase) was added and incubated in each reaction chamber at 37°C for 30 min. After washes, 50 µl UNG mix (1x NEB4 buffer, 0.5 µg/µl BSA, 5 units of UNG) was added to the chamber, which was incubated at 37°C for 30 min. Thereafter 50 µl 1XPBS, 0.05% Tween20 was added to the chamber applied with padlock probes (Figure 25A), whereas unfolding and cleavage reactions were carried out in the chamber applied with two-part probes (Figure 25B): Firstly, 50 µl of unfolding and cleavage mix (1x NEB4 buffer (New England Biolabs (NEB), 0.5 µg/µl BSA (NEB), 50 units of Nb Btsl (NEB), 50 units of Mlyl (NEB)) and 500 pmol of additional cleavage strands were added to each well. After incubation at 37°C for 60 min followed by washes, 50 µl of additional cleavage mix (1x NEB4 buffer (NEB), 0.5 µg/µl BSA (NEB) and 5 unit of UNG (Fermentas) was added to remove the cleavage strands. The reaction was incubated at 37°C for 30 min. After washes, 50 µl of ligation mix (1x NEB4 buffer (NEB), 0.5 µg/µl BSA (NEB), 15 units of T4 DNA ligase (Fermentas) and 0.5 mM ATP (Fermentas)) was added to the reaction chambers. The ligation reaction was incubated at 37°C for 30 min. Finally, secondary RCA reactions in both chambers were initiated by the addition of 50 µl of rolling circle amplification (RCA) mix (1x phi29 buffer (Fermentas), 0.2 µg/µl purified BSA (NEB), 0.25 mM dNTPs (Fermentas), 25 units of phi29 (Fermentas)). The RCA reactions were carried out at 37°C for 60 min. The primary and secondary RCA products were visualized by hybridization of 10 nM detection oligonucleotides labelled with different fluorophores, by incubation at 37°C for 30 min. Thereafter slides were washed, dried, mounted with VectaShield (Immunkemi) and a cover slip and imaged.

Figure 25 shows that the signal generated using two-part RCA reporter probes is significantly enhanced in comparison to a secondary RCA performed using padlock probes.

| Table 1 - list of oligonucleotides used in the Examples described above | | | | |
|---|---|---|---|---|
| *Oligonucleotide name* | 5' *modification* | 3' *modification* | *Purification* | *Sequence (5'-3)* |
| **Example 1** | | | | |
| Target | 5' Biotin | - | HPLC | |
| Continuous RCA two part probe part 1 | - | - | Standard | |
| Continuous RCA probe two part probe part 2 | - | 3' Biotin | HPLC | |
| Cleavage strand RCA probe RCA template strand | - | - | PAGE | |
| Cleavage strand | - | - | Standard | UUUUUGACTCUUCCUUGUUUCCCCUCCGCUUUGCCUGUCU (SEQ ID NO:5) |

| **Example 4** | | | | |
|---|---|---|---|---|
| Prox probe domain 1 | 5' thiol | 3' RNA Ome | | AAAAAAAAAAGACGCTAATAGTTAAGACGCTTUUU (SEQ ID NO:6) |
| Prox probe domain 2 | 5' thiol | - | | AAAAAAAAAATATGACAGAACTAGACACTCTT (SEQ ID NO:7) |
| Two part linear RCA template strand | - | - | | |
| Cleavage strand | - | - | | UUUUUGACTCUUCCUUGUUUCCCCUCCGCUUUGCCUGUCU (SEQ ID NO:5) |

| **Example 6** | | | | |
|---|---|---|---|---|
| Target | 5' Biotin | 3' RNA Ome | Standard | |
| RCA primer part | 5' Biotin | - | Standard | |
| Connection template 1 | - | - | Standard | GGCAAAGCGGUUUUUGACUC (SEQ ID NO:11) |
| Padlock 1 | - | - | Standard | |
| Connection template 2 | - | - | Standard | CAAGCGGACCUUUUUGACUC (SEQ ID NO:13) |
| Padlock 2 | - | - | Standard | |
| Cleavage strand 1 | - | - | Standard | CCUGCUUGUCGUGUCCCGCUUUGCCUGUCUUUUUUGACUC (SEQ ID NO:15) |
| Ligation 1 | - | - | Standard | |
| Cleavage strand 2 | - | - | Standard | UGUUGUGCGUUUGGCGGUCCGCUUGUCUUGUUUUUGACUC (SEQ ID NO:17) |
| Ligation 2 | - | - | Standard | |
| Cleavage strand 3 | - | - | Standard | UUUUUGACUCCGACGAACGUGAUAC (SEQ ID NO:19) |
| Detection oligo 1 | 5' Cy3 | - | HPLC | TGCGTTTGGCGGTCCGCTTG (SEQ ID NO:20) |
| Detection oligo 2 | 5' Cy5 | - | HPLC | TTGTCGTGTCCCGCTTTGCC (SEQ ID NO:21) |

| **Example 7** | | | | |
|---|---|---|---|---|
| Correct target | 5' Biotin | 3' RNA Ome | HPLC | TCTCTCTCTCTCTCT CCGCGCTCGTCGTCG CGCGTCCGCCCCGCG UUU (SEQ ID NO:22) |
| Wrong target | 5' Biotin | 3' RNA Ome | HPLC | TCTCTCTCTCTCTCT CCGCGCTCGTCGTCG AGCGTCCGCCCCGCG UUU (SEQ ID NO:23) |
| Two-part RCA template strand | 5' phosphoryl ation | | PAGE | |
| Detection oligonucleotide | 5'Cy3 | | HPLC | TTCCTTGTTTCCCCTCCGCTTTGCCTGTCT (SEQ ID NO:25) |
| Cleavage strand | - | | Standard | UUUUUGACTCUUCCUUGUUUCCCCUCCGCUUUGCCUGUCU (SEQ ID NO:5) |

## Claims

1. A probe for use in detecting a target analyte in a sample, wherein the probe provides or is capable of providing nucleic acid components sufficient to initiate a rolling circle amplification (RCA) reaction, said probe being a nucleic acid construct comprising:
(i) at least two domains each comprising a target binding domain capable of binding to the target analyte or to an intermediate molecule bound, directly or indirectly, to the target analyte;
(ii) one or more domains together capable of providing a RCA template; and
(iii) at least one domain capable of providing a primer for said RCA reaction that hybridises to a region of said RCA template and optionally a ligation template that templates the ligation of the RCA template, said domain comprising;
(a) a region of complementarity to a sequence within the probe, such that it forms part of a hairpin structure that comprises a cleavage recognition site; or
(b) a region of double stranded nucleic acid that comprises a cleavage recognition site,
wherein at least part of the probe must be cleaved and/or unfolded to release said primer to enable said rolling circle amplification reaction, and wherein
(1) each domain capable of providing a RCA nucleic acid component is directly or indirectly attached to the target analyte via a target binding domain;
(2) the domain capable of providing the RCA template is adjacent to the domain capable of providing the ligation template; and
(3) cleavage of the probe releases said RCA nucleic acid components to enable a RCA reaction when the target binding domains are bound to the target analyte or intermediate molecule.

2. The probe of claim 1, wherein:
a) at least one of said target binding domains comprises a region of complementarity to a target nucleic acid molecule or an intermediate nucleic acid molecule bound, directly or indirectly, to the target analyte, preferably wherein the target nucleic acid molecule is the target analyte or a marker for the target analyte, and/or wherein the target binding domains are nucleic acid domains that hybridize to the target or intermediate nucleic acid molecule such that the 5' and 3' ends of the probe are directly or indirectly ligatable; or
b) wherein the probe comprises a nucleic acid molecule coupled to one or more analyte-binding domains to form the target binding domains, preferably wherein the analyte-binding domain is selected from a protein, such as a monoclonal or polyclonal antibody, a lectin, a soluble cell surface receptor, a combinatorially derived protein from phage display or ribosome display, a peptide, a carbohydrate, a nucleic acid aptamer, or a combination thereof, preferably wherein the analyte-binding domain is an antibody or derivative or fragment thereof.

3. The probe of claim 1 or 2, wherein at least one of said one or more domains together capable of providing a RCA template comprises a region of complementarity to a sequence within the probe, such that it forms part of a hairpin structure that comprises a cleavage recognition site.

4. The probe of any one of claims 1 to 3, wherein
(i) the ligation template and primer domain are provided as separate domains; or
(ii) the primer domain and ligation template are provided by the same part of the probe.

5. The probe of claim 4(i), wherein each RCA nucleic acid component is provided by a separate domain and wherein the domains capable of providing a ligation template and primer comprise;
(a) a region of complementarity to a sequence within the probe, such that it forms part of a hairpin structure that comprises a cleavage recognition site; or
(b) a region of double stranded nucleic acid that comprises a cleavage recognition site.

6. The probe of any one of claims 1 to 5, wherein the probe comprises a single stranded nucleic acid molecule comprising at least two hairpin structures.

7. The probe of any one of claims 1 to 6, wherein cleavage and/or unfolding of the probe releases a single stranded 3' end that may be degraded by an enzyme having exonucleolytic activity, preferably wherein said enzyme degrades a portion of one strand of the probe thereby releasing a RCA nucleic acid component provided by the probe.

8. The probe of any one of claims 1 to 5 or 7, wherein the probe comprises a partially double stranded nucleic acid molecule and wherein at least part of at least one of the nucleic acid strands is single stranded, preferably wherein
a) at least one of the strands of the partially double stranded construct is a pre-circle oligonucleotide; or
b) one strand of the partially double stranded molecule comprises at least one hairpin structure and a second strand is provided as an oligonucleotide that is hybridized to a part of the first strand that does not form a hairpin structure.

9. The probe of any one of claims 1 to 8, wherein
(i) the cleavage recognition site comprises an endonuclease recognition sequence; or
(ii) said probe comprises a domain comprising one or more uracil residues.

10. The probe of claim 9, wherein
(i) the endonuclease recognition sequence is a nickase or type IIS endonuclease recognition sequence; or
(ii) the domain comprising said one or more uracil residues can be cleaved with a uracil-DNA glycosylase enzyme in combination with an endonuclease enzyme capable of recognising apurinic/apyrimidinic (AP) sites of dsDNA.

11. The probe of any one of claims 1 to 10, wherein the probe comprises one or more blocking groups, preferably wherein said one or more blocking groups is in a target binding domain of the probe; and/or wherein said one or more blocking groups is in a domain of the probe other than a target binding domain.

12. The probe of claim 11, wherein the blocking group is selected from the group consisting of a 2'O-Me-RNA residue, a Locked Nucleic Acid (LNA), a Peptide Nucleic Acids (PNA), a phosphothioate-modifed nucleic acid, a Poly-ethylene-linker backbone stretch in between nucleic acids and an acridine residue.

13. The probe of any one of claims 1 to 12, wherein the probe is unable to generate a RCA product unless the probe is bound to said target or intermediate molecule.

14. Use of a probe as defined in any one of claims 1 to 13 in the detection of an analyte in a sample, wherein said probe interacts with an analyte or an intermediary molecule bound thereto to generate a RCA product.

15. A method for detecting an analyte in a sample comprising:
(a) contacting said sample with a probe as defined in any one of claims 1 to 13, wherein said probe interacts with said analyte or an intermediary molecule bound thereto;
(b) releasing the nucleic acid components to directly enable a RCA reaction by cleavage and/or unfolding of the probe, wherein at least one of the released components of the cleaved and/or unfolded probe functions as the primer for the RCA reaction;
(c) extending the primer using the RCA template to produce a RCA product; and
(d) detecting said RCA product.

## Patentansprüche

1. Sonde zur Verwendung im Nachweisen eines Targetanalyten in einer Probe, wobei die Sonde Nukleinsäurekomponenten, die ausreichend sind, um eine Rolling-Circle-Amplifikations-(RCA)-Reaktion zu initiieren, bereitstellt oder in der Lage ist, diese bereitzustellen, wobei die Sonde ein Nukleinsäurekonstrukt ist, umfassend:
(i) wenigstens zwei Domänen, jeweils umfassend eine Targetbindungsdomäne, die in der Lage ist, an den Targetanalyten oder an ein Intermediatmolekül zu binden, welches direkt oder indirekt an den Targetanalyten gebunden ist;
(ii) eine oder mehrere Domänen, die zusammen in der Lage sind, ein RCA-Templat bereitzustellen; und
(iii) wenigstens eine Domäne, die in der Lage ist, einen Primer für die RCA-Reaktion bereitzustellen, welcher an eine Region des RCA-Templats hybridisiert, und gegebenenfalls ein Ligationstemplat, welches die Ligation des RCA-Templats templiert, wobei die Domäne umfasst:
(a) eine Region mit Komplementarität zu einer Sequenz innerhalb der Sonde, so dass diese Teil einer Haarnadelstruktur bildet, die eine Spalterkennungsstelle umfasst; oder
(b) eine Region einer doppelsträngigen Nukleinsäure, die eine Spalterkennungsstelle umfasst,
wobei wenigstens ein Teil der Sonde gespalten und/oder entfaltet werden muss, um den Primer freizusetzen, um die Rolling-Circle-Amplifikations-Reaktion zu ermöglichen, und wobei
(1) jede Domäne, die in der Lage ist, eine RCA-Nukleinsäurekomponente bereitzustellen, direkt oder indirekt über eine Targetbindungsdomäne an dem Targetanalyten befestigt ist;
(2) die Domäne, die in der Lage ist, ein RCA-Templat bereitzustellen sich neben der Domäne befindet, die in der Lage ist, das Ligationstemplat bereitzustellen; und
(3) ein Spalten der Sonde, die RCA-Nukleinsäurekomponenten freisetzt, um eine RCA-Reaktion zu ermöglichen, wenn die Targetbindungsdomänen an den Targetanalyten oder das Intermediatmolekül gebunden sind.

2. Sonde nach Anspruch 1, wobei:
a) wenigstens eine der Targetbindungsdomänen wenigstens eine Region mit Komplementarität zu einem Targetnukleinsäuremolekül oder einem Intermediatnukleinsäuremolekül umfasst, welches direkt oder indirekt an den Targetanalyten gebunden ist, bevorzugt wobei das Targetnukleinsäuremolekül der Targetanalyt oder ein Marker für den Targetanalyten ist, und/oder wobei die Targetbindungsdomänen Nukleinsäuredomänen sind, die an das Target- oder das Intermediatnukleinsäuremolekül hybridisieren, so dass die 5'- und 3'-Enden der Sonde direkt oder indirekt ligierbar sind; oder
b) wobei die Sonde eine Nukleinsäuremolekül umfasst, welches an eine oder mehrere analytenbindende Domänen gekoppelt sind, um die Targetbindungsdomänen zu bilden, bevorzugt wobei die analytenbindende Domäne ausgewählt ist aus einem Protein wie z.B. einem monoklonalen oder polyklonalen Antikörper, einem Lektin, einem löslichen Zelloberflächenrezeptor, einem kombinatorisch abgeleiteten Protein aus einem Phagedisplay oder Ribosomendisplay, einem Peptid, einem Kohlenhydrat, einem Nukleinsäureaptamer oder einer Kombination davon, bevorzugt wobei die analytenbindende Domäne ein Antikörper oder ein Derivat oder ein Fragment davon ist.

3. Sonde nach Anspruch 1 oder 2, wobei wenigstens eine der ein oder mehr Domänen, die zusammen in der Lage sind, ein RCA-Templat bereitzustellen, eine Region mit Komplementarität zu einer Sequenz innerhalb der Sonde umfasst, so dass diese einen Teil einer Haarnadelstruktur bildet, die eine Spalterkennungsstelle umfasst.

4. Sonde nach einem der Ansprüche 1 bis 3, wobei
(i) das Ligationstemplat und die Primerdomäne als separate Domänen bereitgestellt werden; oder
(ii) die Primerdomäne und das Ligationstemplat von dem gleichen Teil der Sonde bereitgestellt werden.

5. Sonde nach Anspruch 4(i), wobei jede RCA-Nukleinsäurekomponente von einer separaten Domäne bereitgestellt wird, und wobei die Domänen, die in der Lage sind, ein Ligantionstemplat und einen Primer bereitzustellen, umfassen:
(a) eine Region mit Komplementarität zu einer Sequenz innerhalb der Sonde, so dass diese einen Teil einer Haarnadelstruktur bildet, die eine Spalterkennungsstelle umfasst; oder
(b) eine Region einer doppelsträngigen Nukleinsäure, die eine Spalterkennungsstelle umfasst.

6. Die Sonde nach einem der Ansprüche 1 bis 5, wobei die Sonde ein einzelsträngiges Nukleinsäuremolekü umfassend wenigstens zwei Haarnadelstrukturen umfasst.

7. Sonde nach einem der Ansprüche 1 bis 6, wobei ein Spalten und/oder Entfalten der Sonde ein einzelsträngiges 3'-Ende freisetzt, welches von einem Enzym abgebaut werden kann, welches exonukleolytische Aktivität aufweist, bevorzugt wobei das Enzym einen Teil eines Strangs der Sonde abbaut, so dass eine RCA-Nukleinsäurekomponente freigesetzt wird, die von der Sonde bereitgestellt wird.

8. Sonde nach einem der Ansprüche 1 bis 5 oder 7, wobei die Sonde ein teilweise doppelsträngiges Nukleinsäuremolekül umfasst, und wobei wenigstens ein Teil wenigstens eines der Nukleinsäurestränge einzelsträngig ist, bevorzugt wobei
a) wenigstens einer Stränge des teilweise doppelsträngigen Konstrukt ein Prä-Circle-Oligonukleotid ist; oder
b) ein Strang des teilweise doppelsträngigen Moleküls wenigstens eine Haarnadelstruktur umfasst und ein zweiter Strang als ein Oligonukleotid bereitgestellt wird, welches an einen Teil des ersten Strangs hybridisiert ist, welcher keine Haarnadelstruktur bildet.

9. Sonde nach einem der Ansprüche 1 bis 8, wobei
(i) die Spalterkennungsstelle eine Endonukleoaseerkennungsstelle umfasst; oder
(ii) die Sonde eine Domäne umfassend einen oder mehrere Uracilreste umfasst.

10. Sonde nach Anspruch 9, wobei
(i) die Endonukleaseerkennungsstelle eine Nickase- oder Typ-IIS-Endonukleaseerkennungsstelle ist; oder
(ii) die Domäne umfassend die ein oder mehreren Uracilreste mit einem Uracil-DNA-Glycosylaseenzym in Kombination mit einem Endonukleoaseenzym gespalten werden kann, welches in der Lage ist, apurinische/apyrimidinische-(AP)-Stellen von dsDNA zu erkennen.

11. Sonde nach einem der Ansprüche 1 bis 10, wobei die Sonde eine oder mehrere Blockinggruppen umfasst, bevorzugt wobei die ein oder mehreren Blockinggruppen eine Targetbindungsdomäne der Sonde sind; und/oder wobei die ein oder mehreren Blockinggruppen einen Domäne der Sonde sind, die sich von einer Targetbindungsdomäne unterscheiden.

12. Sonde nach Anspruch 11, wobei die Blockinggruppe ausgewählt ist aus der Gruppe bestehend aus einem 2'O-Me-RNA-Rest, einer Locked-Nukleinsäure (engl. *locked nucleid acid,* LNA), einer Peptidnukleinsäure (engl. *peptide nucleic acid,* PNA), einer Phosphothioat-modifizierten Nukleinsäure, einem Polyethylenlinker-Rückgratabschnitt zwischen Nukleinsäuren und einem Acridinrest.

13. Sonde nach einem der Ansprüche 1 bis 12, wobei die Sonde nicht in der Lage ist, ein RCA-Produkt zu erzeugen, es sei denn, die Sonde ist an das Target- oder Intermediatmolekül gebunden.

14. Verwendung einer Sonde nach einem der Ansprüche 1 bis 13 im Nachweisen eines Analyten in einer Probe, wobei die Probe mit einem Analyten oder einem daran gebundenen Intermediatmolekül wechselwirkt, um ein RCA-Produkt zu erzeugen.

15. Verfahren zum Nachweisen eines Analyten in einer Probe umfassend:
(a) In-Kontakt-bringen der Probe mit einer Sonde nach einem der Ansprüche 1 bis 13, wobei die Sonde mit dem Analyten oder einem daran gebundenen Intermediatmolekül, wechselwirkt;
(b) Freisetzen der Nukleinsäurekomponenten, um durch Abspalten und/oder Entfalten der Sonde direkt eine RCA-Reaktion zu ermöglichen, wobei wenigstens einer der freigesetzten Komponenten der abgespaltenen und/oder entfalteten Sonde als der Primer für die RCA-Reaktion wirkt;
(c) Erweitern des Primers unter Verwendung des RCA-Templats, um ein RCA-Produkt zu erzeugen; und
(d) Nachweisen des RCA-Produkts.

## Revendications

1. Sonde à utiliser dans la détection d'une substance à analyser cible dans un échantillon, dans laquelle la sonde fournit ou est susceptible de fournir des composants d'acides nucléiques suffisants pour amorcer une réaction d'amplification par cercle roulant (RCA), ladite sonde étant un constituant comprenant de l'acide nucléique :
(i) au moins deux domaines comprenant chacun un domaine de liaison à la cible susceptible de se lier à la substance à analyser cible ou à une molécule intermédiaire liée, directement ou indirectement, à la substance à analyser cible ;
(ii) un ou plusieurs domaines susceptibles ensemble de fournir une matrice RCA ; et
(iii) au moins un domaine susceptible de fournir une amorce pour ladite réaction RCA qui s'hybride à une région de ladite matrice RCA et de manière facultative une matrice de ligature qui modélise la ligature de la matrice RCA, ledit domaine comprenant :
(a) une région de complémentarité à une séquence à l'intérieur de la sonde, de sorte qu'elle forme une partie d'une structure en épingle à cheveux qui comprend un site de reconnaissance de clivage ; ou
(b) une région d'acide nucléique à double brin qui comprend un site de reconnaissance de clivage,
dans laquelle au moins une partie de la sonde doit être clivée et/ou dépliée pour libérer ladite amorce pour permettre ladite réaction d'amplification par cercle roulant, et dans laquelle
(1) chaque domaine susceptible de fournir un composant d'acide nucléique RCA est directement ou indirectement attaché à la substance à analyser cible via un domaine de liaison à la cible ;
(2) le domaine susceptible de fournir la matrice RCA est adjacent au domaine susceptible de fournir la matrice de ligature ; et
(3) le clivage de la sonde libère lesdits composants d'acides nucléiques RCA pour permettre une réaction RCA lorsque les domaines de liaison à la cible sont liés à la substance à analyser cible ou à une molécule intermédiaire.

2. Sonde selon la revendication 1, dans laquelle :
a) au moins un desdits domaines de liaison à la cible comprennent une région de complémentarité à une molécule d'acide nucléique cible ou une molécule d'acide nucléique intermédiaire liée, directement ou indirectement, à la substance à analyser cible, de préférence dans laquelle la molécule d'acide nucléique cible est la substance à analyser cible ou un marqueur pour la substance à analyser cible, et/ou dans laquelle les domaines de liaison à la cible sont des domaines d'acides nucléiques qui s'hybrident à la molécule d'acide nucléique cible ou intermédiaire de sorte que les extrémités 5' et 3' de la sonde peuvent directement ou indirectement subir une ligature ; ou
b) dans laquelle la sonde comprend une molécule d'acide nucléique couplée à un ou plusieurs domaines liaison à une substance à analyser pour former les domaines de liaison à la cible, de préférence dans laquelle le domaine de liaison à la substance à analyser est sélectionné à partir d'une protéine, comme un anticorps monoclonal ou polyclonal, une lectine, un récepteur de surface de cellule soluble, une protéine obtenue de manière combinatoire à partir d'une présentation sur phages ou d'une présentation sur ribosomes, un peptide, un hydrate de carbone, un aptamère d'acide nucléique, ou une combinaison de ceux-ci, de préférence dans laquelle le domaine de liaison à la substance à analyser est un anticorps ou un dérivé ou un fragment de ce dernier.

3. Sonde selon la revendication 1 ou 2, dans laquelle au moins un desdits un ou plusieurs domaines susceptibles ensemble de fournir une matrice RCA comprend une région de complémentarité à une séquence à l'intérieur de la sonde, de sorte qu'elle forme une partie d'une structure en épingle à cheveux qui comprend un site de reconnaissance de clivage.

4. Sonde selon l'une quelconque des revendications 1 à 3, dans laquelle
(i) la matrice de ligature et le domaine d'amorce sont fournis en tant que domaines distincts ; ou
(ii) le domaine d'amorce et la matrice de ligature sont fournis par la même partie de la sonde.

5. Sonde selon la revendication 4(i), dans laquelle chaque composant d'acide nucléique RCA est fourni par un domaine distinct et dans laquelle les domaines susceptibles de fournir une matrice de ligature et une amorce comprennent :
(a) une région de complémentarité à une séquence à l'intérieur de la sonde, de sorte qu'elle forme une partie d'une structure en épingle à cheveux qui comprend un site de reconnaissance de clivage ; ou
(b) une région d'acide nucléique à double brin qui comprend un site de reconnaissance de clivage.

6. Sonde selon l'une quelconque des revendications 1 à 5, dans laquelle la sonde comprend une molécule d'acide nucléique à simple brin comprenant au moins deux structures en épingle à cheveux.

7. Sonde selon l'une quelconque des revendications précédentes 1 à 6, dans laquelle le clivage et/ou le dépliement de la sonde libèrent une extrémité 3' à simple brin qui peut être dégradée par une enzyme ayant une activité exonucléolytique, de préférence dans laquelle ladite enzyme dégrade une partie d'un brin particulier de la sonde libérant de ce fait un composant d'acide nucléique RCA fourni par la sonde.

8. Sonde selon l'une quelconque des revendications précédentes 1 à 5 ou 7, dans laquelle la sonde comprend une molécule d'acide nucléique partiellement à double brin et dans laquelle au moins une partie d'au moins un des brins d'acide nucléique est à simple brin, de préférence dans laquelle
a) au moins un des brins du constituant partiellement à double brin est un oligonucléotide de pré-cercle ; ou
b) un brin particulier de la molécule partiellement à double brin comprend au moins une structure en épingle à cheveux et un second brin est fourni comme un oligonucléotide qui est hybridé à une partie du premier brin qui ne forme pas de structure en épingle à cheveux.

9. Sonde selon l'une quelconque des revendications 1 à 8, dans laquelle
(i) le site de reconnaissance de clivage comprend une séquence de reconnaissance d'endonucléase ; ou
(ii) ladite sonde comprend un domaine comprenant un ou plusieurs résidus d'uracile.

10. Sonde selon la revendication 9, dans laquelle
(i) la séquence de reconnaissance d'endonucléase est une séquence de reconnaissance de nickase ou d'endonucléase de type IIS ; ou
(ii) le domaine comprenant lesdits un ou plusieurs résidus d'uracile peut être clivé avec une enzyme uracile-ADN glycosylase en combinaison avec une enzyme endonucléase susceptible de reconnaître des sites apuriniques / apyrimidiniques (AP) d'ADN à double brin.

11. Sonde selon l'une quelconque des revendications 1 à 10, dans laquelle la sonde comprend un ou plusieurs groupes de blocage, de préférence dans lequel lesdits un ou plusieurs groupes de blocage sont dans un domaine de liaison à la cible de la sonde ; et/ou dans lequel lesdits un ou plusieurs groupes de blocage sont dans un domaine de la sonde autre qu'un domaine de liaison à la cible.

12. Sonde selon la revendication 11, dans laquelle le groupe de blocage est sélectionné à partir du groupe constitué par un résidu de 2'O-Me-ARN, un Acide Nucléique Verrouillé (LNA), des Acides Nucléiques Peptidiques (PNA), un acide nucléique modifié par phosphothioate, un bout de squelette de lieur de polyéthylène entre des acides nucléiques et un résidu d'acridine.

13. Sonde selon l'une quelconque des revendications précédentes 1 à 12, dans laquelle la sonde est incapable de produire un produit RCA à moins que la sonde ne soit liée à ladite molécule cible ou intermédiaire.

14. Utilisation d'une sonde selon l'une quelconque des revendications 1 à 13, dans la détection d'une substance à analyser dans un échantillon, dans laquelle ladite sonde interagit avec une substance à analyser ou une molécule intermédiaire liée à celle-ci pour produire un produit RCA.

15. Procédé pour détecter une substance à analyser dans un échantillon comprenant :
(a) la mise en contact dudit échantillon avec une sonde selon l'une quelconque des revendications 1 à 13, dans lequel ladite sonde interagit avec ladite substance à analyser ou une molécule intermédiaire liée à celle-ci ;
(b) la libération des composants d'acide nucléique pour directement permettre une réaction RCA par clivage et/ou dépliement de la sonde, dans lequel au moins un des composants libérés de la sonde clivée et/ou dépliée fonctionne comme l'amorce pour la réaction RCA ;
(c) l'extension de l'amorce en utilisant la matrice RCA pour produire un produit RCA ; et
(d) la détection dudit produit RCA.
